(19) Europäisches Patentamt
European Patent Office
Office européen des brevets

(11) Veröffentlichungsnummer: **0 230 942 B1**

(12) # EUROPÄISCHE PATENTSCHRIFT

(45) Veröffentlichungstag der Patentschrift: **29.04.92**

(51) Int. Cl.[5]: **C07D 495/14**, A61K 31/55, C07D 495/04, //(C07D495/14, 333:00,249:00,243:00), (C07D495/14,333:00,243:00, 235:00),(C07D495/04,333:00, 243:00)

(21) Anmeldenummer: **87100593.0**

(22) Anmeldetag: **17.01.87**

Die Akte enthält technische Angaben, die nach dem Eingang der Anmeldung eingereicht wurden und die nicht in dieser Patentschrift enthalten sind.

(54) **Thieno-1,4-diazepine.**

(30) Priorität: **21.01.86 DE 3601557**
**22.07.86 DE 3624646**

(43) Veröffentlichungstag der Anmeldung:
**05.08.87 Patentblatt 87/32**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 000 479**
**EP-A- 0 194 416**
**EP-A- 0 254 245**
**US-A- 4 307 237**
**EP-A- 0 176 927**
**EP-A- 0 240 899**
**CH-A- 612 972**

**CHEMICAL ABSTRACTS, vol. 81, no. 23, December 9, 1974, page 537, abstract 152 238u Columbus, Ohio, USA; & JP - A - 74 69 667**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE ES FR GR IT LI LU NL SE AT**

(73) Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Stransky, Werner, Dr.**
**Im Hippel 24**
**W-6535 Gau-Algesheim(DE)**
Erfinder: **Weber, Karl-Heinz, Dr.**
**Kaiser-Karl-Strasse 11**
**W-6535 Gau-Algesheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 230 942 B1

Erfinder: **Walther, Gerhard, Dr.**
**Pfarrer-Heberer-Strasse 37**
**W-6530 Bingen(DE)**
Erfinder: **Harreus, Albrecht, Dr.**
**Sandstrasse 1**
**W-6507 Ingelheim am Rhein(DE)**
Erfinder: **Casals Stenzel, Jorge, Dr.**
**Sertoriusring 295**
**W-6500 Mainz 21(DE)**
Erfinder: **Muacevic, Gojko, Dr.**
**In der Dörrwiese 13**
**W-6507 Ingelheim am Rheim(DE)**
Erfinder: **Bechtel, Wolf-Dietrich**
**Mühlstrasse 3**
**W-6531 Appenheim(DE)**

**Beschreibung**

Die Erfindung betrifft neue Thieno-1,4-diazepine, ihre Herstellung nach bekannten Methoden und ihre Verwendung als Arzneistoffe und als Zwischenprodukte. Die neuen Thieno-1,4-diazepine entsprechen der allgemeinen Formel

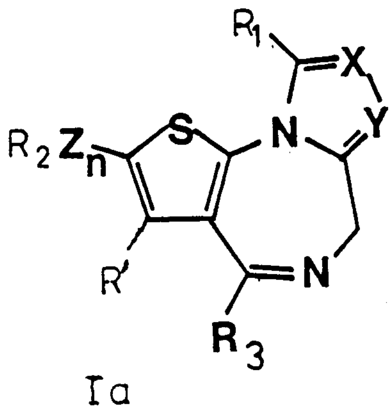

Ia

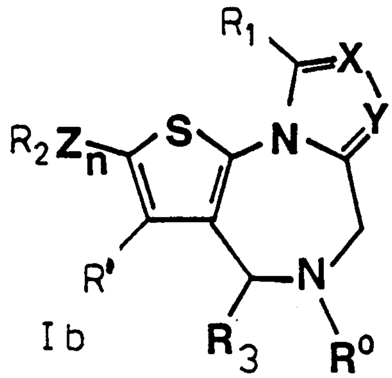

Ib

worin

$R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropylgruppe, eine verzweigte oder unverzweigte Alkoxygruppe mit 1 bis 4 Kohlenstoffatomen, bevorzugt Methoxy, Halogen, bevorzugt Chlor oder Brom;

für n > O

$R_2$ Halogen, Hydroxy,

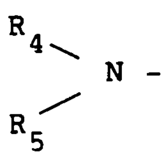

wobei $R_4$ und $R_5$, die gleich oder verschieden sein können, Wasserstoff, eine verzweigte oder unverzweigte Alkyl-, Alkenyl oder Alkinylgruppe mit 1 - 10 Kohlenstoffatomen, die gegebenenfalls durch Halogen, Hydroxy oder durch einen C-verknüpften Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen, bevorzugt Chlor, oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

eine gegebenenfalls substituierte Arylcarbonylgruppe, bevorzugt Phenylcarbonyl, eine gegebenenfalls substituierte Arysulfonylgruppe, bevorzugt Phenylsulfonyl oder Tolylsulfonyl, eine Alkylsulfonylgruppe mit 1 bis 4 Kohlenstoffatomen, oder

$R_4$ und $R_5$ bilden zusammen mit dem Stickstoffatom einen gesättigten oder ungesättigten gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff,

Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen, bevorzugt Methyl, substituiert sein kann;

$R_2$ eine Arylsulfonyloxygruppe, bevorzugt Tolylsulfonyloxy oder Phenylsulfonyloxy, gegebenenfalls ein- oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylsulfonyloxygruppe mit 1 bis 4 Kohlenstoffatomen;

$R_2$ eine Arylcarbonyloxygruppe, bevorzugt Phenylcarbonyloxy, gegebenenfalls ein oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylcarbonyloxygruppe mit 1 bis 12, bevorzugt 1 bis 8, Kohlenstoffatomen, wobei die Alkylkette durch Stickstoff, Sauerstoff oder Schwefel unterbrochen sein kann;

$R_2$

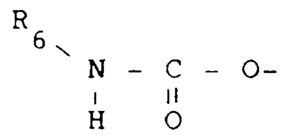

wobei $R_6$ eine verzweigte oder unverzweigte Alkyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe,

$R_2$

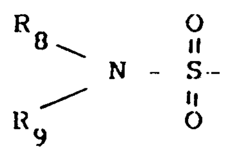

wobei $R_8$ und $R_9$, die gleich oder verschieden sein können, eine verzweigte oder unverzweigte Alkylgruppe mit 1 bis 4 Kohlenstoffatomen,

oder $R_8$ und $R_9$ zusammen mit dem Stickstoffatom einen gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, bevorzugt Methyl, substituiert ist;

$R_2$ eine verzweigte oder unverzweigte Alkoxygruppe mit 1 bis 4 Kohlenstoffatomen; eine Aryloxygruppe, bevorzugt Phenyloxy oder substituiertes Phenyloxy;

$R_2$ einen Imidorest; Dioxolan, substituiertes Dioxolan;

für n größer gleich O

$R_2$ -CH=0; Cyano;

$R_2$ eine Aryloxycarbonylgruppe, bevorzugt Phenyloxycarbonyl;

$R_2$ einen Rest der allgemeinen Formel

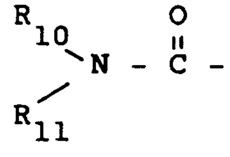

worin $R_{10}$ und $R_{11}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 10 Kohlenstoffatomen, die gegebenenfalls durch Halogen, Hydroxy, Nitro, Amino, substituiertes Amino oder im Fall von $R_{10}$ = Wasserstoff oder Alkyl und Y = C-$R_1$ oder Y Stickstoff und X C-Alkyl, $R_{11}$ durch eine Esterfunktion oder ein Säureamid der allgemeinen Formel

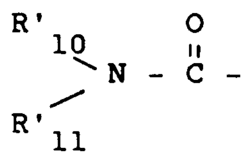

worin $R'_{10}$ und $R'_{11}$ dieselbe Bedeutung wie $R_{10}$ und $R_{11}$, jedoch mit Ausnahme eines Säureamids haben, substituiert sein kann,

$R_{10}$ oder $R_{11}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6- oder 7-gliedriger heterocyclischer Ring,

$R_{10}$ und $R_{11}$ zusammen mit dem Stickstoffatom einen gesättigten oder ungesättigten gegebenenfalls ein-oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,

$R_2$ einen Rest der allgemeinen Formel

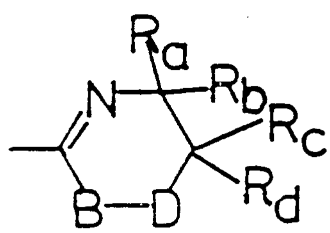

worin

B Sauerstoff, Schwefel, NH oder $NC_1$-$C_6$-Alkyl, D den Rest (C Re Rf)n, wobei n 0 bis 3 sein kann, Ra Wasserstoff, gegebenenfalls durch eine Hydroxy-oder Aminogruppe substituiertes Alkyl mit 1 bis 6 Kohlenstoffatomen, $C_1$ bis $C_4$ Alkoxycarbonyl, Dialkylaminocarbonyl,

Rb, Rc, Rd, Re, Rf Wasserstoff, gegebenenfalls durch eine Hydroxy- oder Aminogruppe substituiertes Alkyl mit 1 bis 6 Kohlenstoffatomen, oder Phenyl;

$R_3$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl;

R° Wasserstoff, eine Alkyl- oder eine Acylgruppe mit 1 bis 4 Kohlenstoffatomen in der Alkylkette, bevorzugt Acetyl;

R' Wasserstoff, Phenyl, substituiertes Phenyl oder eine verzweigte oder unverzweigte Alkylgruppe mit 1 bis 4 Kohlenstoffatomen, bevorzugt Methyl;

X,Y unabhängig voneinander C-$R_1$ oder N, aber nicht gleichzeitig beide C-$R_1$,
oder Y die Gruppe C-COOR*, mit R* = Alkyl oder Wasserstoff und X = Stickstoff;

Z eine verzweigte oder unverzweigte Alkyl- oder Alkenylgruppe mit n-Kohlenstoffatomen, wobei Z gegebenenfalls zusätzlich durch Aryl, bevorzugt Phenyl, substituiertes Phenyl oder 2-fach durch $R_2$ substituiert sein kann, wobei $R_2$ gleich oder verschieden sein kann;

und

n eine der Zahlen 0, 1, 2, 3, 4, 5, 6, 7, 8, 9, oder 10 bedeuten können,
in Form ihrer Racemate, ihrer Enantiomere, ihrer Diasteromere und ihrer Gemische; jeweils als freie Basen oder ihre physiologisch unbedenklichen Säureadditionssalze.

Bevorzugt sind Verbindungen der allgemeinen Formel Ia und Ib, worin

$R_1$ Methyl, Ethyl, Methoxy, Ethoxy oder Halogen, bevorzugt Chlor oder Brom;

$R_2$ Chlor, Brom, Jod, Hydroxy,

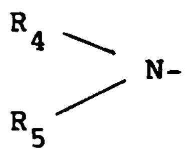

worin $R_4$ und $R_5$, die gleich oder verschieden sein können, Wasserstoff, eine verzweigte oder unverzweigte Alkylgruppe mit 1 bis 6, besonders bevorzugt 1 bis 4, Kohlenstoffatomen, wobei die Kohlenstoffkette durch Stickstoff unterbrochen sein kann, eine verzweigte oder unverzweigte Alkylcarbonylgruppe mit 1 bis 4 Kohlenstoffatomen, gegebenenfalls substituiert durch eine Dimethylaminogruppe, eine Phenylcarbonylgruppe,

im Fall von $R_5$ = Wasserstoff eine gegebenenfalls durch Acylamino, besonders bevorzugt Acetylamino, Amino, Alkylamino- oder Dialkylamino substituierte Phenylsulfonylgruppe, oder

$R_4$ und $R_5$ bilden zusammen mit dem Stickstoffatom eine Piperidin-, Pyrrolidin-, N'-Methylpiperazin-, einen gegebenenfalls dimethylsubstituierten Morpholinring, einen Pyrrol-, Pyrazol-, Imidazol- oder Triazolring;

$R_2$ -CH = O, COOH;

ein gegebenenfalls durch Methyl ein- oder mehrfach substituiertes $\Delta^2$-Imidazolin, -Oxazolin, -Thiazolin; eine Tolylsulfonyloxygruppe, eine Methylsulfonyloxygruppe;

eine Phenylcarbonyloxygruppe;

eine verzweigte oder unverzweigte Alkylcarbonyloxygruppe mit 1 bis 5 Kohlenstoffatomen; eine Methoxy- oder Ethoxycarbonylgruppe;

$R_2$

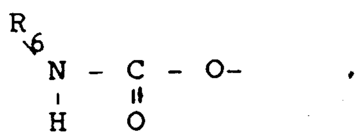

wobei $R_6$ eine verzweigte oder unverzweigte Alkylgruppe mit 1 bis 4 Kohlenstoffatomen,

$R_2$

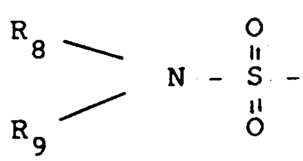

wobei $R_8$ und $R_9$, die gleich oder verschieden sein können, eine Methyl-, Ethyl-, Propyl- oder Isopropylgruppe oder $R_8$ und $R_9$ zusammen mit dem Stickstoffatom einen N'-Methylpiperazin- oder Morpholinring;

$R_2$

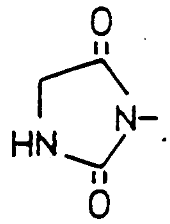 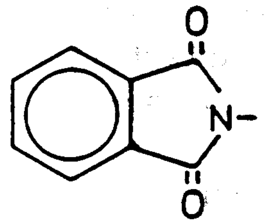 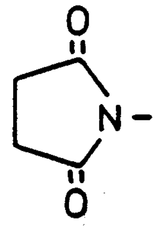

$R_3$ Phenyl, wobei der Phenylring, bevorzugt in 2-Stellung durch H Halogen, bevorzugt Chlor, substituiert sein kann;

R° Wasserstoff, Methyl, Acetyl;

R′ Wasserstoff;

X,Y unabhängig voneinander C-$R_1$ oder N, aber nicht gleichzeitig beide C-$R_1$, ($R_1$ bevorzugt Wasserstoff),
oder Y die Gruppe C-COOR*, mit R* = Alkyl oder Wasserstoff und X = Stickstoff;

Z wie zuvor definiert, bevorzugt $-(CH_2)_n-$,gegebenenfalls substituiert durch Phenyl oder 2-fach durch $R_2$, wobei $R_2$ auch verschieden sein kann oder $-CH_2-CHR_2-CH_2-R_2$ - $CH_2-CHR_2R_2$, $-CH_2CHR_2-CH_2-C_6H_5$;
und

n eine der Zahlen 1, 2, 3, 4, 5, 6, 7, 8, 9 oder 10 bedeuten können sowie gegebenenfalls ihre physiologisch unbedenklichen Säureadditionssalze.

Besonders bevorzugt sind Verbindungen der allgemeinen Formel Ia, worin $R_1$ = Methyl oder Methoxy, $R_3$ = o-Chlorphenyl, R′ Wasserstoff, X, Y beide Stickstoff, oder X CH und Y Stickstoff, Z $(CH_2)_n$,n = 2, 3 oder 7 und $R_2$ und R° wie zuvor definiert.

Soweit nichts anderes angegeben, bedeutet Halogen eines der Atome Fluor, Chlor, Brom oder Jod, unter Arylgruppen werden gegebenenfalls ein- oder mehrfach substituierte aromatische Reste mit bis zu 10 Kohlenstoffatomen im Ringsystem verstanden, wie z.B. Phenyl, Pyridyl, Thienyl, Furyl oder Naphthyl, wobei der Phenylring bevorzugt ist. Als Alkyl-, Alkenyl-, Alkinyl- und Alkoxygruppen werden, soweit nicht anderes angegeben, gegebenenfalls substituierte, verzweigte oder unverzweigte Reste mit 1 bis 8 Kohlenstofatomen in der Kohlenstoffkette verstanden. Als Substituenten können ein oder mehrere Atome aus der Gruppe Halogen, Methyl, Methoxy- Hydroxy- oder Trifluormethyl vorliegen.

Bevorzugte Imidoreste sind die folgenden Strukturen

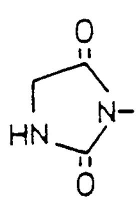 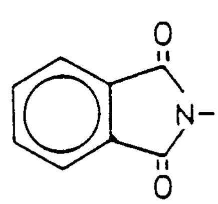 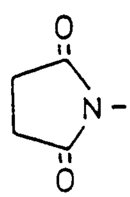

Als niederes Alkyl bzw. niederes Acyl werden, soweit nichts anderes angegeben, verzweigte oder unverzweigte Reste mit 1 bis 4 Kohlenstoffatomen in der Alkylkette verstanden.

In den allgemeinen Formeln gibt "n" in "$Z_n$" die Anzahl der Kohlenstoffatome in der Alkyl- bzw. Alkenylkette an. Ist $Z_n$ zweifach durch $R_2$ substituiert, so können die Substituenten gleich oder verschieden sein. Ist $Z_n$ eine verzweigte Alkyl- oder Alkenylgruppe, so ist die Verzweigung bevorzugt in der $\alpha$-oder $\beta$-Position zur endständigen funktionellen Gruppe..

Als Alkenyl werden Alkylketten mit mindestens einer Doppelbindung, als Alkinyl Alkylketten mit mindestens einer Dreifachbindung bezeichnet.

Als Alkylgruppen werden bevorzugt, soweit nichts anderes angegeben ist: Methyl, Ethyl, Propyl, iso-Propyl; n-Butyl, iso-Butyl und t-Butyl, wobei die Methylgruppe besonders wichtig ist. Bevorzugte Alkoxygruppe ist Methoxy.

Aus der europäischen Patentanmeldung 176 927 sind bereits Diazepine mit einer PAF-antagonistischen Wirkung bekannt. Sofern die dort vorgestellten Verbindungen als Stand der Technik gemäß Artikel 54 (2) anzusehen sind, weisen die erfindungsgemäßen Verbindungen bei einer guten PAF-antagonistischen Wirkung gleichzeitig eine verringerte sedierende Wirkung auf. In der Patentanmeldung sind jedoch folgende

Verbindungen namentlich genannt und als Stand der Technik im Sinne von Artikel 54 (3) anzusehen.
2-(2-Chlorethyl)-4-(2-chlorphenyl)-9-methyl-6H-thieno-[3,2-f][1,2,4]triazolo[4,3-a][1,4]diazepin,
2-(Methylsulfonylmethyl)-4-(2-chlorphenyl)-9-methyl-6H-thieno[3,2-d][1,2,4]triazolo[4,3-a][1,4]diazepin,
2-Cyano-4-(2-chlorphenyl)-9-methyl-thieno[3,2-f][1,2,4]-triazolo[4,3-a][1,4]diazepin,
2-(1-Hydroxyethyl)-4-(2-chlorphenyl)-9-methyl-6H-thieno-[3,2-f][1,2,4]triazolo[4,3-a][1,4]diazepin.

Als nicht vorveröffentlichter Stand der Technik ist das europäische Patent 194 416 anzusehen. Dort werden Thieno-triazolo-1,4-diazepino-2-carbonsäureamide der allgemeinen Formel I

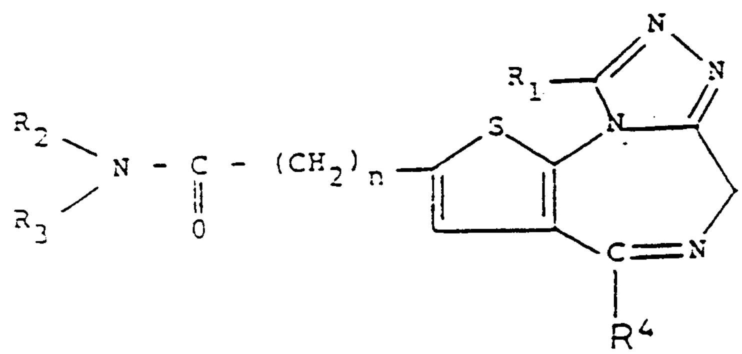

(I)

offenbart.

In dieser Formel bedeuten :

$R_1$ Wasserstoff, eine geradkettige oder verzweigte Alkylgruppe mit 1-4 Kohlenstoffatomen, gegebenenfalls durch Halogen, bevorzugt Cl oder Br, oder Hydroxy substituiert, Cyclopropyl, Alkoxy mit 1-3 Kohlenstoffatomen, bevorzugt Methoxy, Halogen, bevorzugt Chlor oder Brom ;

$R_2$, und $R_3$, die gleich oder verschieden sein können, Wasserstoff, einen geradkettigen oder verzweigten Alkyl- oder Hydroxyalkylrest mit 1-4 Kohlenstoffatomen oder beide Reste $R_2$ und $R_3$ zusammen mit dem Stickstoffatom einen 5-, 6- oder 7-Ring, der gegebenenfalls als weiteres Heteroatom ein Stickstoff-. Sauerstoff- oder Schwefelatom enthalten und wobei, das zweite Stickstoffatom gegebenenfalls durch eine Alkylgruppe mit 1-4 Kohlenstoffatomen, bevorzugt die Methylgruppe, substituiert sein kann ;

$R_4$ Phenyl, wobei der Phenylring, bevorzugt in 2-Stellung, durch Methyl, Halogen, besonders bevorzugt Chlor oder Brom, Nitro oder Trifluormethyl substituiert sein kann oder $\alpha$-Pyridyl ; und

n eine der Zahlen 0, 1, 2, 3, 4, 5, 6, 7 oder 8.

Zur Vermeidung einer Doppelpatentierung wurden die genannten Strukturen disclaimt.

Die neuen Verbindungen der allgemeinen Formel Ia können nach bekannten Verfahren aus den entsprechenden Thienodiazepinthionen der allgemeinen Formel II oder aber durch Variation funktioneller Gruppen der Seitenkette des bereits fertiggestellten Hetrazepingerüstes erhalten werden.

Die neuen Verbindungen der allgemeinen Formel Ib erhält man durch Reduktion von Verbindungen der allgemeinen Formel Ia. Die Umsetzung erfolgt mit bekannten Reduktionsmitteln in organischen Lösungsmitteln, so z.B. mit Zink in einer Mischung aus Eisessig und einem inerten organischen Lösungsmitteln, wie z.B. halogenierten Kohlenwasserstoffen, wie z.B. Dichlormethan, bei Temperaturen zwischen Raumtemperatur und dem Siedepunkt des Reaktionsgemisches oder z.B. mittels Lithiumaluminiumhydrid (soweit $R_2$ nicht reduziert wird).

Verbindungen der allgemeinen Formel Ib, in denen $R^o$ eineAlkyl- oder Acylgruppe bedeutet, lassen sich aus den zuvor genannten Verbindungen durch Alkylierung oder Acylierung nach bekannten Verfahren herstellen.

Verbindungen der allgemeinen Formel Ia

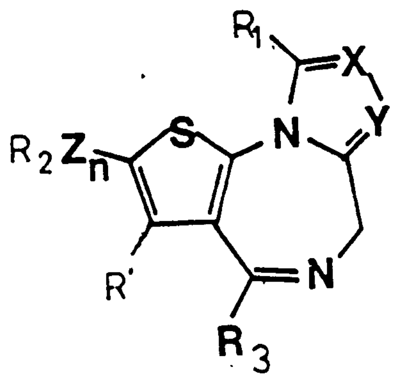

worin $R_2$ -COOR* eine Estergruppierung wie zuvor definiert, bevorzugt R* = niederes Alkyl besonders bevorzugt Methyl und Ethyl bedeutet oder $R_2$ = OH, bevorzugt als Essigsäureester geschützt, sind pharmakologisch wirksam und zugleich wichtige Zwischenverbindungen zur Herstellung weiterer $R_2$-funktionalisierter Hetrazepine der allgemeinen Formel Ia bzw. Ib.

Verbindungen der allgemeinen Formel Ia mit $R_2$ -COOR* werden im weiteren auch als Formel I bezeichnet.

Verbindungen der allgemeinen Formel Ia mit einem ankondensierten Triazolring können in üblicher Weise aus den entsprechenden Thieno-1,4-diazepin-thionen der allgemeinen Formel

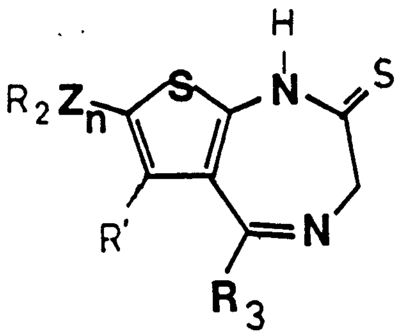

II

($R_2$ = -COOR*, (bevorzugt R* = niederes Alkyl) oder $R_2$ $Z_n$ die Bedeutung eines Alkyldicarbonsäuremethyl- oder -ethylesters mit 1 bis 10 Kohlenstoffatomen in der Alkylkette oder $R_2$ = $OCOCH_3$ oder $SO_2$ $NR_8R_9$) hergestellt werden.

Hierzu kann eine Verbindung der Formel II entweder

a) mit einem Säurehydrazid der allgemeinen Formel

$R_1$-CONHNH$_2$ III

umgesetzt,

oder aber

b) mit Hydrazin in eine Verbindung der allgemeinen Formel

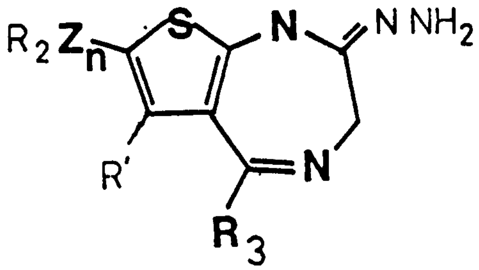

IV

überführt
und anschließend mit einem Säurehalogenid, bevorzugt einem Säurechlorid, der allgemeinen Formel

$R_1$-COHal V

oder mit einem Orthoester der allgemeinen Formel

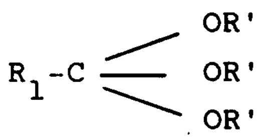

VI

worin R' eine niedere Alkylgruppe, bevorzugt Methyl oder Ethyl bedeutet,
umgesetzt werden.

Die Umsetzung des Thions II mit einem Säurehydrazid III nach dem Verfahren a) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen der Raumtemperatur und dem Siedepunkt des Reaktionsgemisches. Die Isolierung der Endprodukte geschieht nach bekannten Methoden, wie z.B. durch Kristallisation.

Die Umsetzung des Thions II mit Hydrazin nach dem Verfahren b) erfolgt in inerten organischen Lösungsmitteln, wie beispielsweise Tetrahydrofuran, Dioxan, halogenierten Kohlenwasserstoffen, wie z.B. Methylenchlorid, geeigneten Kohlenwasserstoffen, bei Temperaturen zwischen Raumtemperaturen und dem Siedepunkt des Reaktionsgemisches.

Die dabei entstehenden Hydrazin-1,4-diazepine können nach herkömmlichen Verfahren isoliert oder aber auch direkt weiterverarbeitet werden.

Die weitere Umsetzung mit einem Säurehalogenid V oder einem Orthoester VI erfolgt in einem inerten organischen Lösungsmittel, wie z.B. halogenierten Kohlenwasserstoffen, cyclischen oder aliphatischen Ethern,
kann aber auch direkt in Substanz erfolgen. Die Isolierung des Endprodukts Ia erfolgt nach bekannten Methoden, beispielsweise durch Kristallisation.

Der Aufbau der Hetrazepine der allgemeinen Formel Ia, in der X eine CH-Gruppe und Y Stickstoff bedeutet, erfolgt in an sich bekannter Weise aus dem Thion der allgemeinen Formel II, durch Umsetzung mit einem Aminoalkin der allgemeinen Formel VII, worin $R_{11}$ Wasserstoff oder eine Alkylgruppe, bevorzugt

Wasserstoff, bedeutet, wobei durch den Einsatz von Hydrochloriden für den Hetrazepinringschluß hydrolyseempfindliche $R_2$-Gruppen zugänglich werden.

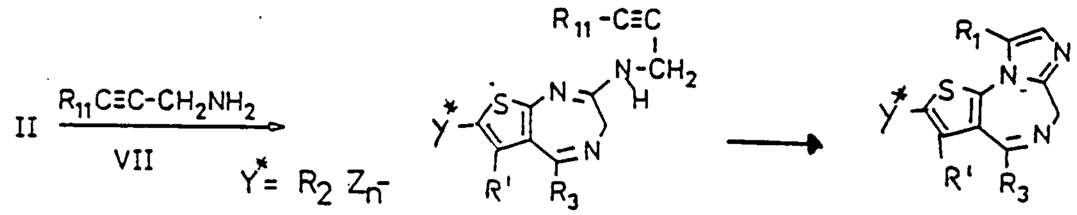


Ia

Nach diesem Verfahren können Verbindungen der allgemeinen Formel Ia hergestellt werden, worin $R_1$ eine Alkyl, bevorzugt die Methylgruppe, bedeutet.
Ein weiteres Verfahren besteht in der Umsetzung des Thions der allgemeinen Formel II mit einem
α-Aminoaldehyd-alkylacetal oder
α-Aminoketon-alkylketal der allgemeinen Formel VIII nach folgendem Syntheseschema,

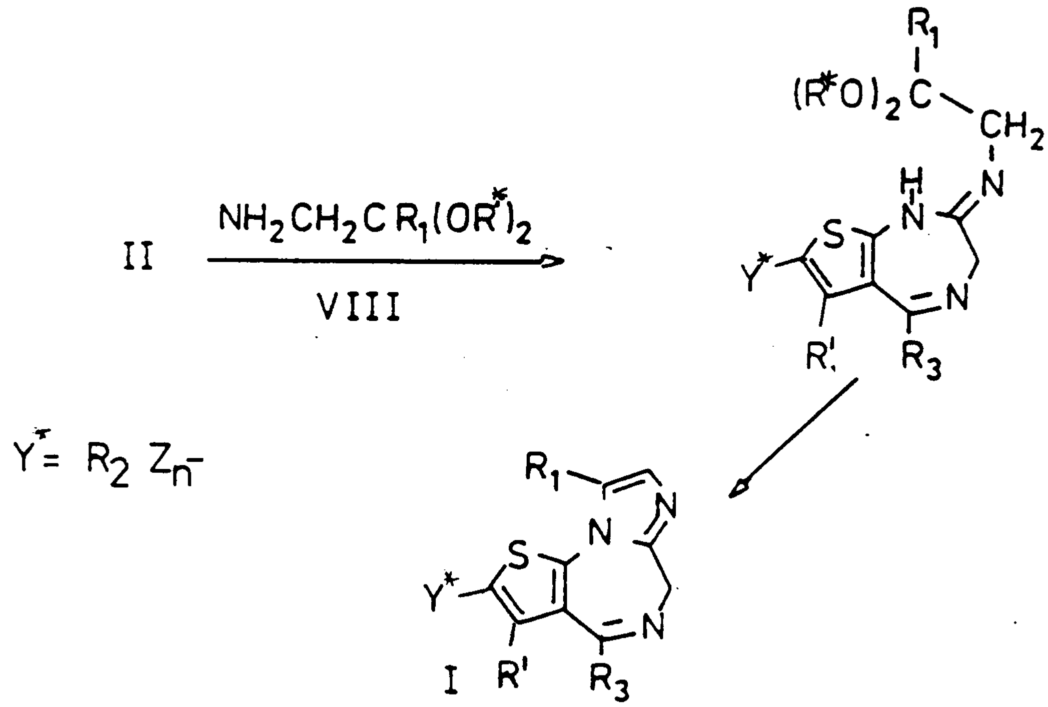


wobei $R_1$ Wasserstoff oder eine Alkylgruppe mit 1 bis 4 Kohlenstoffatomen oder Cyclopropylgruppe und R* eine niedere Alkylgruppe bedeutet.

Analogieverfahren zur Synthese eines Acetals oder Ketals der allgemeinen Formel VIII sowie ein Analogieverfahren zum Ringschluß sind in der Schweizerischen Patentschrift Nr. 580 099 beschrieben,

Verbindungen der allgemeinen Formel Ia, in denen X Stickstoff und Y CH bedeutet, können durch Decarboxylierung von Verbindungen der allgemeinen Formel

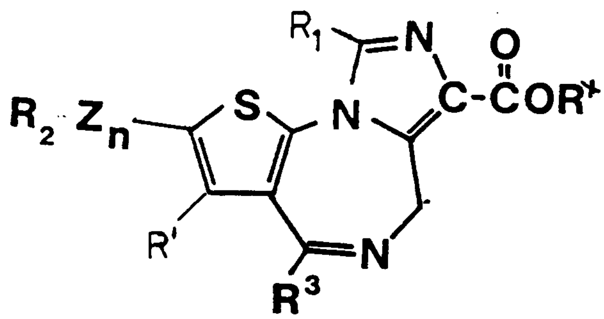

Ic

$R^x$ = niederes Alkyl

erhalten werden. Verbindungen der allgemeinen Formel Ic erhält man beispielsweise aus den Diazepinthionen der allgemeinen Formel II durch Umsetzung mit Isocyanessigsäureestern. Analogieverfahren zur Herstellung geeigneter Verbindungen der allgemeinen Formel Ic sind beispielsweise in der niederländischen Patentanmeldung 78 03 585 beschrieben.

Verbindungen der allgemeinen Formel Ia, die einen [1,5-a] verknüpften Imidazolring enthalten, können beispielsweise auch in Analogieverfahren zu den in der DE-OS 25 40 522 beschriebenen hergestellt werden.

Verbindungen der allgemeinen Formel Ia, in denen $R_1$ Chlor oder Brom bedeuten, werden aus Verbindungen mit $R_1$ = Wasserstoff durch Umsetzung mit Chlor oder Brom in Pyridin hergestellt.

Die entsprechenden Alkoxyverbindungen erhält man beispielsweise aus einer der zuvor erwähnten Chlor- bzw. Bromverbindung durch Umsetzung mit dem entsprechenden Alkoholat.

Bevorzugt werden die Reste $R_1$ für Halogen und Alkoxy, jedoch erst nach dem Aufbau des vollständig funktionalisierten Hetrazepins der allgemeinen Formel Ia nach der beschriebenen Methode eingefügt.

Die oben beschriebenen Verfahren zur Herstellung von Verbindungen der allgemeinen Formel I bzw. Ia erfolgen aus dem Thion der allgemeinen Formel II, bei denen der funktionelle Rest $R_2$ unter den angewandten Reaktionsbedingungen nicht angegriffen wird oder aber durch entsprechende Schutzgruppen geschützt werden kann. Dies trifft insbesondere für $R_2$ eine Esterfunktion, eine Alkylcarbonyloxygruppe Dioxolanyl, substituiertes Dioxolanyl wie auch für $R_2$ = $R_8R_9NSO_2$- zu.

So sind Diazepinthione der allgemeinen Formel II wie folgt herstellbar.

In Analogie zu dem von Gewald et al. Chem. Ber. 98, 3571 (1965), ibid 99, 94 (1966) beschriebenen Verfahren, erhält man ausgehend von den entsprechend funktionalisierten Aldehyden oder Ketonen der allgemeinen Formel a durch Reaktion mit dem entspechenden Acetophenon b die funktionalisierten Thiene c. Nach bekannten Verfahren werden diese durch Bromacetylierung und anschließende Umsetzung mit Ammoniak in die ringgeschlossenen 1,4-Diazepinone überführt, die anschließend mit Phosphorpentasulfid oder Lawesson Reagenz [R] in das Thion der allgemeinen Formel II überführt werden.

Syntheseschema I

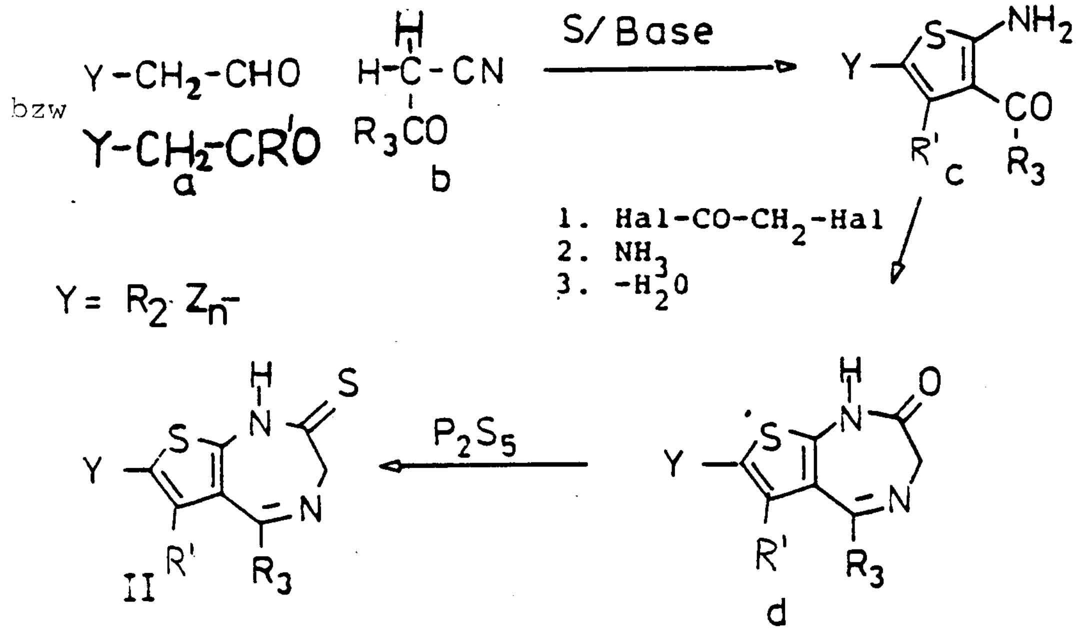


Bevorzugt hat $R_2$ die Bedeutung eines Carbonsäureesters, wie z.B.eines Carbonsäuremethyl- oder ethylesters, einer Alkylcarbonyloxygruppe oder einer Aminosulfonylgruppe.

Die zur Herstellung erforderlichen ω-funktionalisierten Aldehyde a können beispielsweise durch reduktive Ozonspaltung von cyclischen Enolethern (L. Claisen, Ber. dtsch. chem. Ges. 40, 3907 und V. Schmid u. P. Grafen, Liebigs Ann. Chem. 656, 97 (1962), geeigneten Fettsäurederivaten, wie z.B. Essigsäure-Oleylester oder geeigneten ungesättigten Heterocyclen erhalten werden. Die Ozonisierung wird bevorzugt in Methylenchlorid oder Essigester bei -78°C bis +20°C, bevorzugt zwischen -40°C und -20°C durchgeführt.

Ein weiteres Herstellungsverfahren ist in dem folgenden Reaktionsschema angegeben

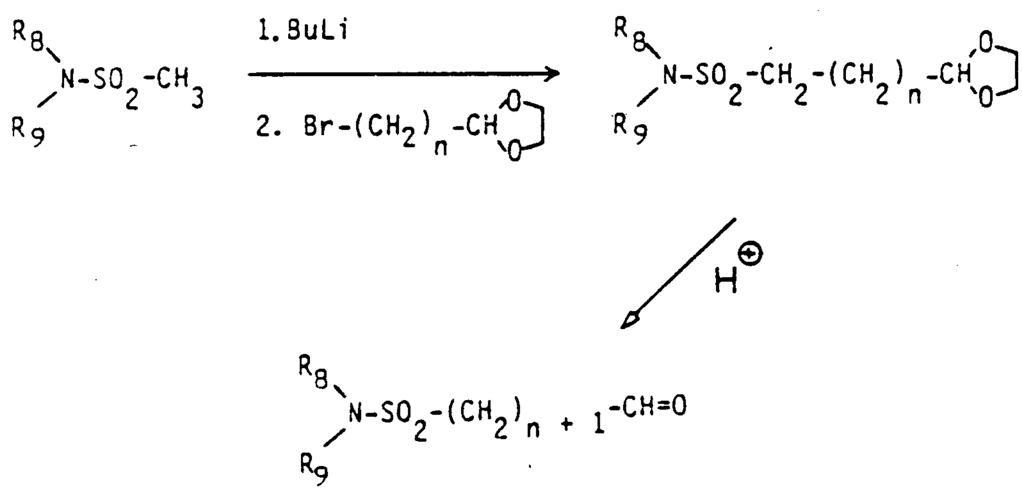


Die Umsetzung der Aldehyde a führt in an sich bekannter Weise, wie im Syntheseschema 1 angegeben, gemäß K. Gewald et.al., Chem. Ber. 98, 3571 (1965) und Chem. Ber.99, 94 (1966) zu den Aminothiophenderivaten c.

Die bei der weiteren Umsetzung von c mit einem Halogenacetylhalogenid entstehenden 2-Halogenacetylaminothiophenderivate können entweder isoliert, oder aber auch als Rohprodukt über das 2-Aminoacetylaminothiophenderivat in das Diazepinon d überführt werden, welches anschließend mit Phosphorpentasulfid oder Lawesson-Reagenz$^R$ zu dem Thion der allgemeinen Formel II reagiert.

Ist $Z_n$ eine verzweigte Alkyl- oder Alkenylgruppe, gegebenenfalls durch Aryl, bevorzugt Phenyl, oder $Z_n$ zweifach durch $R_2$ substituiert, kann der Aufbau der Verzweigung auf der Stufe des $\omega$-funktionalisierten Aldehyds oder nach Fertigstellung des vollständig aufgebauten Hetrazepins nach bekannten Methoden erfolgen.

Im Fall, daß $Z_n$ zweifach funktionalisiert ist, können die funktionellen Gruppen an demselben Kohlenstoffatom oder an verschiedenen Kohlenstoffatomen gebunden sein.

Die Carbonsäureester ($R_2$ = COOR* R* = niederes Alkyl) der allgemeinen Formel Ia sind wertvolle Ausgangsverbindungen I zur Einführung weiterer funktioneller Gruppen.

Ausgehend von den Estern können die entsprechenden Carbonsäuren der allgemeinen Formel Ia durch Verseifen, z.B. in alkoholischer wäßriger Kalilauge, z.B. mit KOH in Ethanol, bei Temperaturen zwischen der Raumtemperatur und dem Siedepunkt des Reaktionsgemisches erhalten werden.

Carbonsäureamide der allgemeinen Formel Ia können nach bekannten Verfahren hergestellt werden, wie z.B. aus den entsprechenden Carbonsäuren oder deren Carbonsäureäquivalenten durch Umsetzung mit einem primären oder sekundären Amin oder Ammoniak der allgemeinen Formel

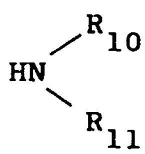

Bevorzugt ist die Überführung in ein Carbonsäurechlorid oder Säureanhydrid oder die Umsetzung der Säure in Gegenwart von Carbonyldiimidazol, Sulfonyldiimidazol, Cyclohexylcarbodiimid.

Die Umsetzung der freien Säure mit dem Amin erfolgt in Gegenwart eines Carbodiimids, beispielsweise von Cyclohexylcarbodiimid, Carbonyldiimidazols oder Sulfonyldiimidazols in einem inerten Lösungsmittel wie Dimethylformamid, Tetrahydrofuran, Dioxan oder halogenierten Kohlenwasserstoff bei Temperaturen zwischen O °C und dem Siedepunkt des Reaktionsgemisches.

Bei der Reaktion des Amins mit einem Säurehalogenid oder Säureanhydrid wird das Amin in einem inerten Lösungsmittel, beispielsweise Dimethylformamid, Tetrahydrofuran, Dioxan oder einem geeigneten Kohlenwasserstoff wie Toluol bei Temperaturen zwischen Raumtemperatur und dem Siedepunkt des Reaktionsgemisches mit dem Säurehalogenid oder dem Säureanhydrid umgesetzt, wobei gegebenenfalls ein säurebindendes Mittel wie Natriumcarbonat, Natriumbicarbonat oder eine tertiäre organische Base, beispielsweise Pyridin oder Triethylamin, zugegeben wird.

Falls es sich bei dem Amin um eine Flüssigkeit handelt, kann die Umsetzung auch in einem Überschuß des Amins ohne zusätzliches Lösungsmittel erfolgen.

Das Säurehalogenid bzw. Säureanhydrid erhält man aus der freien Säure in üblicher Weise, z.B. durch Reaktion der Säure mit einem Thionylhalogenid bzw. durch Umsetzung eines Alkalisalzes der Säure mit Acetylchlorid oder Chlorameisensäurechlorid.

Anstelle der Reaktion mit einem Amin kann auch mit einem Aminosäurederivat umgesetzt werden.

Ester der allgemeinen Formel Ia, insbesondere die Methyl-oder Ethylester, können durch selektive Reduktion der Esterfunktion in den entsprechenden Alkohol überführt werden. Die Reaktion wird unter inverser Zugabe des Reduktionsmittels, wie z.B. Lithiumalanat oder Natriumborhydrid (inverse Aktivierung), unter allgemein üblichen Reaktionsbedingungen durchgeführt, wie z.B. in inerten organischen Lösungsmitteln, z.B. Ethern, Tetrahydrofuran bei Temperaturen zwischen Raumtemperatur und dem Siedepunkt des Reaktionsgemisches.

Carbamate der allgemeinen Formel Ia mit $R_2$ = $R_6$NHCOO - erhält man aus der Reaktion der entsprechenden Alkohole oder Amine mit dem gewünschten Isocyanat in organischen Lösungsmitteln, wie z.B. Tetrahydrofuran, Methylenchlorid, bei Temperaturen zwischen Raumtemperatur und dem Siedepunkt, bevorzugt bei erhöhten Temperaturen, des Reaktionsgemisches unter Zusatz von Base, bevorzugt DABCO (1,4-Diazabicyclo(2,2,2)octan).

Verbindungen, in denen $R_2$ Alkyl- oder Arylcarbonyloxy ist, werden aus den entsprechenden Alkoholen der allgemeinen Formel Ia durch Umsetzung mit einem Säureäquivalent abgeleitet von einer Carbonsäure der allgemeinen Formel

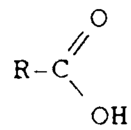

worin R ein Arylrest oder bevorzugt ein verzweigter oder unverzweigter Alkylrest mit 1 bis 8 Kohlenstoffatomen, wobei die Kohlenstoffkette durch Stickstoff, Sauerstoff oder Schwefel unterbrochen sein kann, hergestellt. Hierbei können die gleichen Reaktionsbedingungen wie bei der Herstellung der Säureamide angewandt werden.

Aus den Carbonsäuren der allgemeinen Formel Ia mit $R_2$ = COOH lassen sich nach bekannten Methoden die Carboxylazide herstellen, diese können dann in einem inerten organischen Lösungsmittel, wie z.B. Dioxan, durch die Curtiusumlagerung in die Isocyanate überführt werden. Diese Isocyanate lassen sich nach allgemein bekannten Verfahren in die primären Amine und wie oben beschrieben in die Urethane umwandeln.

Ausgehend von Verbindungen der allgemeinen Formel Ia, in denen $R_2$ = OH ist, erhält man durch Reaktion mit Alkyl-bzw. Arylsulfonsäurehalogenide Verbindungen der allgemeinen Formel Ia, worin $R_2$ eine Alkyl- oder Arylsulfonyloxygruppe darstellt. Die Umsetzung erfolgt in inerten organischen Lösungsmitteln, wie z.B. Methylenchlorid, mit Sulfonsäurehalogeniden unter Zusatz von Säurebindern, wie z.B. Triethylamin.

Die so erhaltenen Mesylate sind gute Abgangsgruppen und können nucleophil ausgetauscht werden. Entsprechend funktionalisierte Verbindungen der allgemeinen Formel Ia, z.B. $R_2$ = $CH_3SO_3$-, können mit primären oder sekundären Aminen der Formel

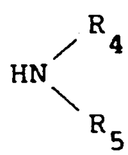

oder einen Imidorest, z.B. Phthalimid umgesetzt werden. Man erhält Verbindungen der allgemeinen Formel Ia, worin $R_2$ die Gruppe $NR_4R_5$ oder einen Imidorest aufweist.

Die Umsetzung erfolgt in inerten organischen Lösungsmitteln, wie z.B. Tetrahydrofuran, Dioxan, zwischen Raumtemperatur und dem Siedepunkt des Reaktionsgemisches, bevorzugt bei erhöhten Temperaturen.

Ausgehend von den oben genannten Mesylaten erhält man Verbindungen der allgemeinen Formel Ia, worin $R_2$ einen Aryloxy- bzw. Alkyloxyrest bedeutet, durch Umsetzung mit den entsprechenden Alkoholaten, entweder in einem Überschuß Alkohol als Lösungsmittel oder aber in inerten Solventien, wie z.B. Dioxan, zwischen Raumtemperatur und dem Siedepunkt des Lösungsmittelgemisches, bevorzugt zwischen 60 bis 80 °C.

Verbindungen der allgemeinen Formel Ia mit $R_2$ = $NH_2$ erhält man in Analogie nach bekannten Verfahren durch Spaltung des entsprechenden Phthalimids.

Die so erhaltenen primären oder sekundären Amine können nach bekannten Verfahren mit Carbonsäureäquivalenten abgeleitet von Carbonsäuren der allgemeinen Formel

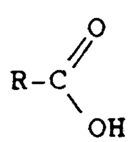

worin R die Bedeutung von $R_{10}$ hat, zu Verbindungen der allgemeinen Formel Ia, worin $R_4$ oder $R_5$ eine Alkyl-oder Arylcarbonylgruppe bedeutet, umgesetzt werden. Die Oxidation der Alkohole ergibt die um ein Kettenglied verkürzten Aldehyde.

Verbindungen der allgemeinen Formel Ia, in denen $R_2$ ein Heterocyclus, wie z.B. ein Oxazolin, Thiazolin oder ein Imidazolin bedeutet, erhält man beispielsweise aus den entsprechenden Carbonsäuren der allgemeinen Formel Ia durch Reaktion mit einem bisfunktionalisierten Amin, wie z.B. einem Aminoalkohol, einem Aminomercaptan oder einem Diamin, in Gegenwart von Triphenylphosphin, Tetrachlorkohlenstoff und einer tertiären organischen Base in Acetonitril. In Analogie hierzu lassen sich auch die entsprechenden 6- und 7-gliedrigen Heterocyclen herstellen. Die Reaktion erfolgt in einem Temperaturbereich zwischen 0°C und dem Siedepunkt der Reaktionsmischung, bevorzugt zwischen 0°C und Raumtemperatur. Verbindungen der allgemeinen Formel Ia, worin $R_2$ ein Oxazolinrest bedeutet, erhält man aus den entsprechend hydroxyfunktionalisierten Carbonsäureamiden durch Ringschlußreaktion mit Thionylchlorid in einem inerten organischen Lösungsmittel, wie z.B. Methylenchlorid.

Diese lassen sich gewünschtenfalls durch Schwefelung, z.B. mit Phosphorpentasulfid oder Lawesson Reagenz $^R$ in das entsprechende Thiazolin überführen.

Verbindungen der allgemeinen Formel Ia, in denen $R_2$ eine Cyanogruppe ist, erhält man aus den entsprechenden primären Cabonsäureamiden durch Reaktion mit Phosphoroxychlorid in einem inerten organischen Lösungsmittel, z.B. Dichlorethan, unter Rückflußbedingungen.

Verbindungen der allgemeinen Formel Ia, worin $R_2$ ein gegebenenfalls durch verzweigte oder unverzweigte Alkylgruppen substituiertes Imidazolin ist, können ausgehend von Verbindungen Ia mit $R_2$ = CN über das Imidoethylesterhydrochlorid durch Reaktion mit einem Diamin erhalten werden (Pinner-Reaktion). Die Bildung des Imidoethylesterhydrochlorids erfolgt durch Behandlung des Nitrils mit einem Überschuß an ethanolischer Salzsäure. Das entstandene kristalline Rohprodukt wird in Ethanol mit dem Diamin (z.B. Ethylendiamin) zuerst unter Eiskühlung, dann unter Rückflußbedingungen umgesetzt. Man erhält so Verbindungen der Formel Ia, in der $R_2$ ein Imidazolin-2-rest ist. Dessen Aminofunktion kann im Fall von N-H nach bekannten Methoden alkyliert werden.

Verbindungen der allgemeinen Formel Ia, worin $R_2$ ein Halogenatom, z.B. Jod, erhält man aus einer Verbindung der allgemeinen Formel Ia, worin $R_2$ Toluolsulfonsäurerest ist durch Reaktion mit einem entsprechenden Halogenierungsmittel, z.B. NaJ, in wasserfreien Lösungsmitteln, wie z.B. Aceton.

Soweit die erfindungsgemäßen Verbindungen ein asymmetrisch substituiertes Kohlenstoffatom aufwesien, können sie nach bekannten Methoden in ihre optisch aktiven Antipoden aufgetrennt werden.

In Analogie zu bekannten Verfahren oder nach den vorstehend beschriebenen Verfahren können beispielsweise die folgenden Verbindungen hergestellt werden:

2-[4-(2-Chlorphenyl)-9-methyl-6H-thieno[3,2-f][1,2,4]triazolo[4,3-a][1,4]-ethan-1-sulfonsäure-N-methylpiperazid
Essigsäure{3-[4-(2-chlorphenyl)-9-methyl-6H-thieno[3,2-f][1,2,4]triazolo[4,3-a][1,4]diazepin-2-yl)-propylester}
2-[7-(N'-Methylpiperazinylcarbonyloxy)heptyl]-4-(2-chlorphenyl)-9-methyl-6H-thieno[3,2-f][1,2,4]triazolo[4,3-a][1,4]diazepin
2-[3-(N'-Methylpiperazinylcarbonyloxy)propyl]-4-(2-chlorphenyl)-9-methyl-6H-thieno[3,2-f][1,2,4]triazolo[4,3-a][1,4]diazepin
2-[3-(N-Morpholinylcarbonyloxy)propyl]-4-(2-chlorphenyl)-9-methyl-6H-thieno[3,2-f][1,2,4]triazolo[4,3-a][1,4]-diazepin
2-[7-(N-Morpholinylcarbonyloxy)heptyl]-4-(2-chlorphenyl)-9-methyl-6H-thieno[3,2-f][1,2,4]triazolo[4,3-a][1,4]-diazepin
2-(2-Hydroxyethyl)-4-(2-chlorphenyl)-9-methyl-6H-thieno[3,2-f][1,2,4]triazolo[4,3-a][1,4]diazepin
2-(4-Hydroxybut)-4-(2-chlorphenyl)-9-methyl-6H-thieno[3,2-f][1,2,4]triazolo[4,3-a][1,4]diazepin
2-(5-Hydroxyethyl)-4-(2-chlorphenyl)-9-methyl-6H-thieno[3,2-f][1,2,4]triazolo[4,3-a][1,4]diazepin
2-(10-Hydroxyethyl)-4-(2-chlorphenyl)-9-methyl-6H-thieno[3,2-f][1,2,4]triazolo[4,3-a][1,4]diazepin
Essigsäure-{7-[4-(2-Chlorphenyl)-9-methyl-6H-thieno[3,2-f][1,2,4]triazolo[4,3-a][1,4]diazepin-2-yl]-heptylester}
2-(3-Acetoxypropyl)-4-(2-chlorphenyl)-9-brom-6H-thieno[3,2-f][1,2,4]triazolo[4,3-a][1,4]diazepin
2-(2-(Methylcarbonyloxy)ethyl]-4-(2-chlorphenyl)-9-methyl-6H-thieno[3,2-f][1,2,4]triazolo[4,3-a][1,4]diazepin
2-(4-(Isopropylcarbonyloxy)butyl]-4-(2-chlorphenyl)-9-methyl-6H-thieno[3,2-f][1,2,4]triazolo[4,3-a][1,4]diazepin
2-(5-(Methylcarbonyloxy)pentyl]-4-(2-chlorphenyl)-9-methyl-6H-thieno[3,2-f][1,2,4]triazolo[4,3-a][1,4]diazepin
Methansulfonsäure-{3-[4-(2-chlorphenyl)-9-methyl-6H-thieno[3,2-f][1,2,4]triazolo[4,3-a][1,4]diazepin-2-yl]-propylester}
2-[2-(Methylsulfonyloxy)ethyl]-4-(2-chlorphenyl)-9-methyl-6H-thieno[3,2-f][1,2,4]triazolo[4,3-a][1,4]diazepin
2-[4-(Methylsulfonyloxy)butyl]-4-(2-chlorphenyl)-9-methyl-6H-thieno[3,2-f][1,2,4]triazolo[4,3-a][1,4]diazepin

2-[5-(Methylsulfonyloxy)pentyl]-4-(2-chlorphenyl)-9-methyl-6H-thieno[3,2-f][1,2,4]triazolo[4,3-a][1,4]diazepin
2-[10-(Methylsulfonyloxy)decyl]-4-(2-chlorphenyl)-9-methyl-6H-thieno[3,2-f][1,2,4]triazolo[4,3-a][1,4]diazepin
2-[3-(N-Morpholinyl)propyl]-4-(2-chlorphenyl)-9-methyl-6H-thieno[3,2-f][1,2,4]triazolo[4,3-a][1.4]diazepin
2-[2-(N-Morpholinyl)ethyl]-4-(2-chlorphenyl)-9-methyl-6H-thieno[3,2-f][1,2,4]triazolo[4,3-a][1.4]diazepin
2-[4-(N-Morpholinyl)butyl]-4-(2-chlorphenyl)-9-methyl-6H-thieno[3,2-f][1,2,4]triazolo[4,3-a][1.4]diazepin
2-[5-(N-Morpholinyl)pentyl]-4-(2-chlorphenyl)-9-methyl-6H-thieno[3,2-f][1,2,4]triazolo[4,3-a][1.4]diazepin
2-[10-(2,6-Dimethylmorpholin-4-yl)decyl]-4-(2-chlorphenyl)-9-methyl-6H-thieno[3,2-f][1,2,4]triazolo[4,3-a][1.4]-diazepin
2-[3-(N-Morpholinyl)propyl]-4-(4-chlorphenyl)-9-methyl-6H-thieno[3,2-f][1,2,4]triazolo[4,3-a][1,4]diazepin
2-[3-(N-Morpholinyl)propyl]-4-(2,6-dichlorphenyl)-9-methyl-6H-thieno[3,2-f][1,2,4]triazolo[4,3-a][1,4]diazepin
2-[3-(N-Morpholinyl)propyl]-4-(pyridin-2-yl)-9-methyl-6H-thieno[3,2-f][1,2,4]triazolo[4,3-a][1,4]diazepin
2-(7-Acetylaminoheptyl)-4-(2-chlorphenyl)-9-methyl-6H-thieno[3,2-f][1,2,4]triazolo[4,3-a][1,4]diazepin
2-(7-N-Phthalimidoheptyl)-4-(2-chlorphenyl)-9-methyl-6H-thieno[3,2-f][1,2,4]triazolo[4,3-a][1,4]diazepin
2-(3-Jodpropyl)-4-(2-chlorphenyl)-9-methyl-6H-thieno[3,2-f][1,2,4]triazolo[4,3-a][1,4]diazepin
2-(7-Aminoheptyl)-4-(2-chlorphenyl)-9-methyl-6H-thieno[3,2-f][1,2,4]triazolo[4,3-a][1,4]diazepin
2-(6-Formylhexyl)-4-(2-chlorphenyl)-9-methyl-6H-thieno[3,2-f][1,2,4]triazolo[4,3-a][1,4]diazepin
2-[2-(Imidazol-1-yl)ethyl]-4-(2-chlorphenyl)-9-methyl-6H-thieno[3,2-f][1,2,4]triazolo[4,3-a][1,4]diazepin
2-[3-(Imidazol-1-yl)propyl]-4-(2-chlorphenyl)-9-methyl-6H-thieno[3,2-f][1,2,4]triazolo[4,3-a][1,4]diazepin
2-[4-(Imidazol-1-yl)butyl]-4-(2-chlorphenyl)-9-methyl-6H-thieno[3,2-f][1,2,4]triazolo[4,3-a][1,4]diazepin
2-[5-(Imidazol-1-yl)pentyl]-4-(2-chlorphenyl)-9-methyl-6H-thieno[3,2-f][1,2,4]triazolo[4,3-a][1,4]diazepin
2-[7-(Imidazol-1-yl)heptyl]-4-(2-chlorphenyl)-9-methyl-6H-thieno[3,2-f][1,2,4]triazolo[4,3-a][1,4]diazepin
2-[2-([1,2,4]Triazol-1-yl)ethyl]-4-(2-chlorphenyl)-9-methyl-6H-thieno[3,2-f][1,2,4]triazolo[4,3-a][1,4]diazepin
2-[3-([1,2,4]Triazol-1-yl)propyl]-4-(2-chlorphenyl)-9-methyl-6H-thieno[3,2-f][1,2,4]triazolo[4,3-a][1,4]diazepin
2-[4-([1,2,4]Triazol-1-yl)butyl]-4-(2-chlorphenyl)-9-methyl-6H-thieno[3,2-f][1,2,4]triazolo[4,3-a][1,4]diazepin
2-[5-([1,2,4]Triazol-1-yl)pentyl]-4-(2-chlorphenyl)-9-methyl-6H-thieno[3,2-f][1,2,4]triazolo[4,3-a][1,4]diazepin
2-[2-(4,4-Dimethyloxazolin-2-yl)ethyl]-4-(2-chlorphenyl)-9-methyl-6H-thieno[3,2-f][1,2,4]triazolo[4,3-a][1,4]-diazepin
2-[3-(N-Morpholinyl)propyl]-4-(3,4,5-trimethoxyphenyl)-9-methyl-6H-thieno[3,2-f][1,2,4]triazolo[4,3-a][1,4]-diazepin

Die erfindungsgemäßen Verbindungen besitzen PAF-antagonistische Wirkung.
Bekanntlich handelt es sich bei PAF (Plättchen Aktivierender Faktor) um das Phospholipid Acetyl-glycerylether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen:
Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche Darmerkrankungen, EPH-Gestose (edima-protein uria Hypertension), Erkrankungen des extrakorporalen Kreislauf, ischämische Erkrankungen, entzündliche und immunoligische Erkrankungen, Immunmodulation bei Transplantationen von Fremdgeweben, Immunmodulation bei Leukämie. Metastasenausbreitung z.B. bei bronchialer Neoplasie, Erkrankungen des ZNS, wie z.B. Migräne, Agarosephobie (panic disorder), weiterhin

erweisen sich die erfindungsgemäßen Verbindungen als Cyto- und Organoprotektiv, z.B. zur Neuroprotektion, z.B. bei Leberzirrhose, DIC (disiminierte intravasale Gerinnung);
PAF assoziierte Interaktion mit Gewebshormonen (autocoid hormones), Lymphokine und anderen Mediatoren.

Die PAF-antagonistische Wirkung einzelner Benzodiazepine ist bekannt, vgl. E. Kornecki et al, Science 226, 1454 - 1456 (1984). Für Alprazolam wurde nach der unten beschriebenen Methode ein $IK_{50}$ - (Konzentration bei einer 50%igen Aggregationshemmung) von 14 µM, für Triazolam ein $IK_{50}$ von 9 µM gemessen. Diese, als Tranquilizer beziehungsweise Hypnotika ausgewiesenen und im Handel befindlichen Verbindungen sind jedoch wegen ihrer ausgeprägten sedierenden Wirkung trotz ihrer guten PAF-antagonistischen Wirkung zum Einsatz als PAF-Antagonisten in der Therapie in vielen Fällen ungeeignet.

Bei vielen der erfindungsgemäßen Verbindungen hingegen fehlt die sedierende Wirkung, während die PAF-antagonistische Wirkung im Vergleich zu der der bekannten Benzodiazepine wesentlich besser ist.

Im folgenden werden die pharmakologischen Untersuchungsmethoden mitgeteilt:

## Pharmakologische Untersuchungsmethoden

Die PAF-antagonistische Wirksamkeit einiger Verbindungen der Formel I wurde anhand der Hemmung der Blutplättchen-Aggregation in vitro und der Antagonisierung der durch PAF bewirkten Bronchokonstriktion am narkotisierten Meerschweinchen, Blutdrucksenkung an der narkotisierten Ratte und Hautquaddel an der Ratte untersucht. Darüber hinaus wurden diese Verbindungen hinsichtlich möglicher Nebenwirkungen auf das zentrale Nervensystem geprüft.

### 1. In vitro Untersuchungen: Hemmung der Blutplättchen-Aggregation

Zur Bestimmung der PAF-antagonistischen Wirkung von Substanzen wurde die durch PAF induzierte Aggregation von Humanthrombozyten in vitro verwendet. Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 × g (1200 U/min) 20 Minuten lang zentrifugiert. Die Messung der Thrombozytenaggregation erfolgt nach dem von Born und Cross ausgearbeiteten Verfahren (G. V. R. Born und M.J. Cross, J. Physiol. 168, 178 (1963)), wobei dem TRP unter ständigem Rühren PAF als Auslöser der Aggregation zugesetzt wird.

Die Prüfsubstanz wird jeweils 2 - 3 Minuten vor Auslösung der Aggregation in einem Volumen von 10 µl zugesetzt. Als Lösungsmittel dienen entweder Aqua.dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF ($5 \times 10^{-8}$ M) induziert.

Zur Beurteilung von Substanzeffekten wird das Maximum der ersten Aggregationswelle verwendet. Die durch PAF induzierte maximal Absorptionsrate (= maximale Aggregation × 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet.

Der unter dem Einfluß der Testsubstanz erreichte Aggregationswert wird als 100 % angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt und die $IK_{50}$ (Konzentration bei einer 50%igen Aggregationshemmung) ermittelt. Die IK-Werte von Verbindungen der allgemeinen Formel I bewegen sich im allgemeinen um Werte, die kleiner als 9 µM sind.

### 2. In vivo Untersuchungen

#### 2.1. Antagonisierung der durch PAF bewirkten Bronchokonstriktion an narkotisierten Meerschweinchen

Spontan atmende männliche, 300 - 450 g schwere Meerschweinchen werden 1 Stunde vor einer i.v. Infusion von PAF (30 ng/(kg × min) mit der Testsubstanz oder einem Kontrollvehikel oral vorbehandelt. Die Versuchstiere werden dann mit 2 mg/kg Urethan intraperitoneal anästhesiert, worauf die Vena jugularis, die Arteria carotis und die Trachea kanüliert werden. Die PAF-Infusion induziert bei den Kontrolltieren eine starke, anhaltende Bronchokonstriktion, die anhand des Atemzugvolumens, der Compliance und der

Resistance gemessen wird und ebenso eine Senkung des Blutdruckes. Nach ca. 7 - 10 Minuten tritt der Tod ein. Mit den beschriebenen PAF-Antagonisten können diese Effekte auf Atmung und Blutdruck sowie der Eintritt des Todes verhindert werden.

2.2 Antagonisierung der durch PAF bewirkten Blutdrucksenkung an der narkotisierten Ratte

Normotone, männliche, 200 - 250 g schwere Wistar-Ratten werden mit 2 mg/kg Urethan intraperitoneal anästhesiert. Die Arteria carotis und Vena jugularis werden kanüliert. Eine intravenöse PAF-Infusion (30 ng/-(kg × min)) induziert bei den Kontrolltieren eine starke, anhaltende Blutdrucksenkung. Diese kann dosisabhängig durch intravenöse Injektionen (kumulative Verabreichung) der beschriebenen Verbindungen aufgehoben werden. Auch eine orale oder intravenöse Verabreichung der Verbindung vor Beginn der PAF-Infusion kann die blutdrucksenkende Wirkung der oben genannten PAF-Infusion dosisabhängig verhindern.

2.3 Antagonisierung der durch PAF induzierten Hautquaddel an der Ratte (Modifiziert nach P.P. Koelzer und K.H. Wehr, Arzneim.-Forsch. 8, 181 (1958)

Eine intrakutane Injektion von PAF induziert eine Hautquaddel, die Ausdruck einer durch PAF bedingten Erhöung der Gefäßpermeabilität ist.

Männlichen Wistar-Ratten mit einem Körpergewicht von 250 ± 20 g wird die Bauchdecke (kurz) geschoren. Danach werden 1 ml/kg einer 1%igen Trypanblau-Lösung durch eine Schwanzvene den Tieren injiziert. Symmetrisch zur Mittellinie (linea alba) werden an drei Stellen in Abständen von ca. 1,5 cm intrakutane Injektionen von physiologischer Kochsalzlösung oder PAF-Lösung (12,5 bis 15,0 ng/Stelle in 0,1 ml) verabreicht. Während an der Injektionsstelle der Kochsalzlösung keine Reaktion auftrat, bewirkte PAF eine Hautreaktion (Quaddel), die durch eine Blaufärbung unterschiedlicher Intensität -abhängig von der PAF-Dosis - sichtbar wurde. Durch gleichzeitige intrakutane Verabreichung der beschriebenen Verbindungen oder durch eine intravenöse Vorbehandlung konnte diese durch PAF induzierte Hautreaktion verhindert werden.

3. Wirkungen auf das zentrale Nervensystem

Es ist allgemein bekannt, daß Substanzen dieses Strukturtyps zentralnervöse Effekte verursachen, die jedoch für eine Verbindung mit PAF-antagonistischer Wirkung nicht erwünscht sind. Daher wurden die beschriebenen Verbindungen hinsichtlich ihrer hypnogenen und antikonvulsiven Wirkung sowie ihres Einflusses auf die Lokomotion geprüft. Eine mögliche hypnotische Wirkung wurde an 400 bis 450 g schweren Meerschweinchen untersucht. Dosen biz zu 200 mg/kg p.o. dieser Substanzen waren nicht in der Lage, eine hypnotische oder sedative Wirkung an diesen Tieren zu erzeugen.

Zur Prüfung einer antikonvulsiven Wirkung kann der Pentetrazol-Antagonismus bei Mäusen (20 bis 25 g Körpergewicht) verwendet werden (M. I. Gluckmann, Current Therapeutic Research, 7:721, 1965). Dosen bis zu 100 mg/kg p.o. dieser Verbindungen (1 Stunde vor Pantetrazol) zeigten in diesem Test keinen Einfluß auf die durch Pentetrazol (125 mg/kg i.p., LD 100) hervorgerufene Mortalität.

Die Wirkung auf die Nachtmotilität (Lokomotion) von Mäusen (20 - 25 g Körpergewicht) kann in einem Lichtschrankenkäfig untersucht werden. Dabei wird die Anzahl der Lichtstrahlunterbrechungen gemessen. Dosen bis zu 300 mg/kg p.o. der oben genannten Verbindungen zeigten keine Aktivität.

In der Tabelle A sind die in vitro Untersuchungen bezüglich der Hemmung der Blutplättchen aufgeführt, wie sie unten beschrieben sind.

Tabelle A:

| Substanz | $IK_{50}$ x $10^{-6}$ Mol |
|---|---|
| Alprazolam | 14 |
| Triazolam | 9 |
| Beispiel Nr. | |
| 1 | 0,3 |
| 2 | <0,2 |
| 4 | <0,2 |
| 8 | <0,3 |
| 9 | 0,2 |
| 10 | 1,7 |
| 11 | <0,2 |
| 12 | 0,3 |
| 13 | 0,3 |
| 14i | 0,4 |
| 14l | 0,6 |
| 20 | 0,3 |
| 23 | 0,9 |
| 25 | 0,9 |
| 27 | 0,2 |
| 45 | 0,3 |
| 49 | 1,0 |
| 50 | 0,6 |
| 54 | 1,1 |
| 56 | 0,3 |
| 58 | <0,2 |
| 60 | 0,9 |
| 64 | <0,2 |
| 71 | 0,9 |
| 75 | 1,9 |
| 98 | 2,3 |
| 104 | 2,2 |

Die neuen Verbindungen der allgemeinen Formel Ia und Ib können warmblütigen Tieren topisch, oral, parenteral transdermal oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien usw.. Eine wirksame Dosis der erfindungsgemäßen Verbindungen liegt bei oraler Anwendung zwischen 1 und 50, vorzugsweise zwischen 3 und 20 mg/Dosis, bei intravenöser oder intramuskulärer Anwendung zwischen 0,01 und 50, vorzugsweise zwischen 0,1 und 10mg/Dosis. Für die Inhalation sollen Lösungen, die 0,01 bis 1,0, vorzugsweise 0,1 bis 0,5 % Wirkstoff enthalten, eingesetzt werden.

Die folgenden Beispiele dienen zur näheren Erläuterung der Erfindung:

Beispiel 1:

2-[4-(2-Chlorphenyl)-9-methyl-6H-thieno[3,2-f][1,2,4]triazolo[4,3-a][1,4]diazepin-2-yl]-ethan-1-sulfonsäure-N′-methylpiperazid.

1.1. 3-(1,3-Dioxolan-2-yl)-propan-1-sulfonsäuse-N′-methylpiperazid

Zu 23 g (0,13 mol) Methansulfonsäure-N′-methylpiperazid, gelöst in 200 ml wasserfreiem Tetrahydrofuran, werden bei 0-5 °C 87,5 ml einer 1,6 molaren BuLi-Lösung in Hexan (0,14 mol) zugetropft, die entstandene Suspension bei Raumtemperatur noch 90 Minuten gerührt und dann 23,4 g 2-(2-Bromethyl)-1,3-dioxolan 0,13 mol) in 200 ml wasserfreiem Tetrahydrofuran zugetropft, wobei eine klare Lösung entsteht. Nach 5 Stunden Rühren wird das Lösungsmittel abgezogen, der Rückstand mit Wasser/Methylenchlorid aufgenommen und die wäßrige Phase mehrmals mit Methylenchlorid ausgeschüttelt. Die Methylenchlorid-Lösung wird getrocknet und das Lösungsmittel abgezogen. Den öligen Rückstand filtriert man über eine Kieselgelsäule mit dem Fließmittel Methylenchlorid/Methanol (9 : 1). Nach erneutem Abziehen des Lösungsmittels verbleiben 20,7 g der gewünschten Verbindung als helles Öl.
(Ausbeute 57 % d.Th.).

1.2. 3-Formyl-propan-1-sulfonsäure-N′-methylpiperazid

20,7 g (0,074 mol) 3-(1,3-Dioxolan-2-yl)-propan-1-sulfonsäure-N′-methylpiperazid und 800 ml 2N Schwefelsäure werden 30 Minuten bei 80 °C gerührt, abgekühlt und mit konz. Ammoniak auf pH 6,5-7 gebracht. Die anschließend mit Kochsalz gesättigte Lösung wird mit Methylenchlorid gründlich extrahiert. Nach dem Trocknen und Abziehen des Lösungsmittels erhält man 13,2 g des Aldehyds als helles Öl.
(Ausbeute 76 % d.Th.).

1.3. 2-Amino-3-(2-chlorbenzolyl)-5-[2-(N′-methylpiperazinylsulfonyl)ethyl]-thiophen

10,1 g (0,056 mol) o-Chlorcyanoacetophenon und 18 g (0,056 mol) Schwefel werden in 10 ml Dimethylformamid vorgelegt und 7,8 ml (0,056 mol) Triethylamin zugetropft. Anschließend werden 13,2 g 3-Formyl-n-propan-l- sulfonsäure-N′-methylpiperazid in 50 ml Dimethylformamid zugetropft und die entstandene Lösung 30 Minuten bei 70 °C gerührt. Nach 12 Stunden gibt man das Reaktionsgemisch auf 100 ml Eiswasser und extrahiert mit Essigester. Nach dem Waschen, Trocknen und Abziehen des Lösungsmittels verbleiben 18 g öliger Rückstand, der über eine Kieselgelsäule mit Methylenchlorid/Methanol (9:1) als Fließmittel gereinigt wird. Man erhält 15,0 g der Thiophenverbindung als helles Öl.
(Ausbeute 63 % d. Th.).

$^1$H-NMR ($CDCl_3$) $\delta$ ppm 7,30-7,55 (4H, m, Aryl-H); 7,14 (2H, s, breit, $NH_2$); 6,18 (1H, s, Thiophen-H); 3,18-3,43 (4H, m, Piperazin $(CH_2)_2$-N); 3,07 (4H, s, $SO_2CH_2CH_2$); 2,33-2,61 (4H, m, Piperazin $(CH_2)_2$-N-$SO_2$);2,33 (3H, s, N-$CH_3$).

1.4. 2-Bromacetylamino-3-(2-chlorbenzoyl)-5-[2-N'-methylpiperazinylsulfonyl)ethyl]-thiophen

14 g der Verbindung des Beispiels 1.3 und 4,6 ml Triethylamin werden in 200 ml wasserfreiem Methylenchlorid vorgelegt und 2,85 ml Bromacetylbromid bei Raumtemperatur zugetropft. Nach 4 Stunden wird das Reaktionsgemisch mit Wasser versetzt, leicht alkalisch gestellt und mit Methylenchlorid extrahiert. Nach dem Waschen und Trocknen zieht man das Lösungsmittel bei einer Wasserbadtemperatur von 30 ° C ab. Die erhaltenen Rückstände (17,9 g) müssen sofort weiter umgesetzt werden.

1.5. 2-Aminoacetylamino-3-(2-chlorbenzoyl)-5-[2-N'-methylpiperazinylsulfonyl)ethyl]-thiophen.

17,9 g (0,032 mol) der Verbindung des Beispiels 1.4. werden in 200 ml Essigester gelöst und 4 Stunden Ammoniak eingeleitet. Das Reaktionsgemisch wird 12 Stunden bei Raumtemperatur gerührt, mehrmals mit Wasser gewaschen, die organische Phase getrocknet und das Lösungsmittel abgezogen. Man erhält 6,1 g eines öligen Rückstands..
(Ausbeute 39 % d.Th.).

1.6. 2-[4-(2-Chlorphenyl)-thieno[3,2-f][1,4]diazepin-7-on-2-yl]-ethan-1-sulfonsäure-N'-methylpiperazid

6,1 g (0,013 mol) der Verbindung des Beispiels 1.5. und 20 g Kieselgel werden mit 150 ml Toluol 3 Stunden am Wasserabscheider zum Rückfluß erhitzt. Das Kieselgel wird abgesaugt und mit heißem Methanol extrahiert. Die nach der Aufarbeitung erhaltenen 5,3 g Rohprodukt werden an Kieselgel chromatographiert (Fließmittel: Methylenchlorid/Methanol 9 : 1). Man erhält 2,6 g des Diazepinons (Ausbeute 44 % d.Th.) vom Fp. 135-138 ° C (Acetonitril).

1.7. 2-[4-(2-Chlorphenyl)-thieno[3,2-f][1,4]diazepin-7-thion-2-yl]-ethan-1-sulfonsäure-N'-methylpiperazid

2,6 g (0,0056 mol) des Diazepinons, 1,25 g Phosphorpentasulfid (0,0028 mol) und 1 g Natriumhydrogencarbonat werden 2 Stunden in 30 ml Diaglyme bei 80 ° C gerührt. Das Reaktionsgemisch wird in 100 ml Wasser gegeben und der ausgefallene Feststoff abgesaugt. Das erhaltene Diazepinthion (Ausbeute: 2,7 g, 100 % d.Th.) wird ohne Reinigung weiter umgesetzt.

1.8. 2-[4-(2-Chlorphenyl)-9-methyl-6H-thieno[3,2-f][1,2,4]triazolo[4,3-a][1,4]diazepin-2-yl]-ethan-1-sulfonsäure-N'-methylpiperazid

2,7 g (0,0056 mol) der Verbindung des Beispiels 1.7. und 0,3 ml (0,0062 mol) Hydrazinhydrat werden bei Raumtemperatur eine Stunde in 30 ml Tetrahydrofuran gerührt. Nach dem Abziehen des Lösungsmittels wird der Rückstand mit 10 ml Orthoessigsäuretriethylester eine Stunde bei 70-80 ° C gerührt. Den überschüssigen Orthoester zieht man anschließend ab, nimmt den Rückstand in 2N Salzsäure auf und extrahiert mit Ether und Methylenchlorid. Die wäßrige Phase wird alkalisch gestellt und das ausgefallene Thienotriazolodiazepin mit Methylenchlorid extrahiert. Nach dem Einengen und Trocknen erhält man 1,2 g Rohprodukt, das an Aluminiumoxid, neutral, Aktivitätsstufe III mit Methylenchlorid/Methanol 95:5 als Eluens chromatographiert wird. Man erhält 1,0 g (Ausbeute 35 %) des Thienotriazolodiaepins vom Fp. 148-150 ° C.

$^1$H-NMR ($CDCl_3$) δ ppm 7,30-7,61 (4H, m, Aryl-H); 6,48 (1H, s, Thiophen-H); 4,95 (2H, s, 7-Ring $CH_2$); 3,00-3,47 (8H, m, Piperazin-$(CH_2)_2$-N-$SO_2$, $SO_2$-$CH_2$-$CH_2$-); 2,72 (3H, s, Triazol-$CH_3$); 2,30-2,61 (4H, m, Piperazin-$(CH_2)_2$-N); 2,32 (3H, s, Piperazin-$CH_3$).

Beispiel 2:

Essigsäure-{3-[4-(2-chlorphenyl)-9-methyl-6H-thieno-[3,2-f][1,2,4]triazolo[4,3-a][1,4]diazepin-2-yl]-propylester}

2.1. 2-Amino-3-(2-chlorbenzoyl)-5-(3-acetoxypropyl)-thiophen

Analog Beispiel 1 wird ausgehend von 5-Hydroxypentanal das 2-Amino-3-(2-chlorbenzoyl)-5-(3-hydroxypropyl)- thiophen aufgebaut. 1 g (0,0034 mol) dieser Verbindung wird unter leichtem Erwärmen in 40 ml Essigester gelöst, nach 20-minütiger Chlorwasserstoffeinleitung wird noch 2 Stunden bei Raumtemperatur gerührt, das Lösungsmittel abgezogen, der Rückstand in Methylenchlorid aufgenommen und mit Wasser

gewaschen. Nach dem Trocknen und Abziehen des Lösungsmittels wird der ölige Rückstand an Kieselgel mit Methylenchlorid/Methanol 9:1 als Eluens chromatographiert. Man erhält 0,75 g (Ausbeute 65 % d.Th.) der oben genannten Verbindung.

$^1$H-NMR ($CDCl_3$) δ ppm 7,24-7,54 (4H, m, Aryl-H); 7,09 (2H, s, breit, $NH_2$); 6,13 (1H, s, Thiophen-H); 4,07 (2H, t, J=6 Hz, $OCH_2$); 2,61 (2H, t, J= 6 Hz, Thiophen-$CH_2$); 2,02 (3H, s, $CH_3C=O$); 1,85 (2H, m, $OCH_2$-$CH_2$-):

2.2. Essigsäure-{3-[4-(2-chlorphenyl)-9-methyl-6H-thieno[3,2-f][1,2,4]triazolo[4,3-a][1,4]diazepin-2-yl]-propylester}

Ausgehend von der Verbindung des Beispiels 2.1. erhält man analog Beispiel 1 in 92 % Ausbeute die 2-Bromacetylaminoverbindung, in 86 % Ausbeute die 2-Amino- acetylaminoverbindung, in 76 % Ausbeute das ringgeschlossene Diazepinon, in 70 % Ausbeute das entsprechende Diazepinthion und in 76 % Ausbeute die Titelverbindung vom Fp. 153-155 °C.

Beispiel 3:

2-(3-Hydroxypropyl)-4-(2-chlorphenyl)-9-methyl-6H-thieno-[3,2-f][1,2,4]triazolo[4,3-a][1,4]diazepin

a) Synthese aus der Verbindung des Beispiels 2:

3,0 g (0,0072 mol) der Verbindung des Beispiels 2 werden mit einem Äquivalent Kaliumhydroxid in 40 ml Ethanol 12 Stunden bei Raumtemperatur gerührt; das Lösungsmittel im Vakuum abgezogen und der Rückstand in Methylenchlorid aufgenommen. Nach dem Waschen, Trocknen und Abziehen des Lösungsmittels erhält man 2 g der Titelverbindung vom Fp. 155-160 °C (Ausbeute 74 % d.Th.).

b) Synthese aus 2-[4-(2-Chlorphenyl)-9-methyl-6H-thieno-[3,2-f][1,2,4]triazolo[4,3-a][1,4]diazephin-2-yl]-ethan-1-carbonsäuremethylester

2,5 g (0,0062 mol) des entsprechenden Methylesters werden in 25 ml wasserfreiem Tetrahydrofuran gelöst und bei Raumtemperatur in kleinen Portionen mit insgesamt 0,13 g (0,0034 mol) Lithiumalanatpulver versetzt. Nach 12 Stunden Rühren werden unter Kühlung 0,2 ml Wasser, dann 0,2 ml 6N Natronlauge und anschließend 0,4 ml Wasser zugegeben und gut durchgerührt. Der Feststoff wird abgesaugt und das Filtrat nach dem Abziehen des Lösungsmittels an Kieselgel mit Methylenchlorid/Methanol (4:1) als Eluens chromatographiert. Spuren der Ausgangsverbindung können über HPLC-Säulenchromatographie mit dem Eluens Methylenchlorid/Methanol (4:1) abgetrennt werden. Man erhält 1,6 g der Verbindung 3 (Ausbeute 69 % d.Th.).

$^1$H-NMR ($CDCl_3$) δ ppm 7,30-7,52 (4H, m, Aryl-H); 6,42 (1H, s, Thiophen H); 4,91 (2H, s, 7-Ring-$CH_2$); 3,69 (2H, t, J= 6 Hz, $OCH_2$); 2,88 (2H, t, J=6 Hz, Thiophen-$CH_2$); 2,69 (3H, s, Triazol-$CH_3$); 1,91 (2H, m, $OCH_2CH_2$-); 1,78 (1H, s, breit, OH).

Die Ausgangsverbindung, der 2-[4-(2-Chlorphenyl)-9-methyl-6H-thieno[3,2-f][1,2,4]triazolo[4,3-a][1,4]-diazepin-2-yl]-ethan-1-carbonsäuremethylester wird wie folgt erhalten:

2-Amino-3-o-chlorbenzoyl-5-(2-dicarbethoxyethyl)-thiophen

53,9 g (0,3 mol) o-Chlorcyanoacetophenon, 9,6 g Schwefel, 30,4 g (0,3 mol) Triethylamin und 120 ml Dimethylformamid werden unter Rühren, beginnend bei Raumtemperatur mit 64,8 g (0,3 mol) Dicarbethoxybutyraldehyd (D.T.Warner, J. Am. Chem. Soc. 70, 3470 (1948); Kp. 97 °C/0,1 mBar) versetzt, wobei die Temperatur auf 45-50 °C ansteigt. Man rührt 2 - 3 Stunden bei 60-70 °C, kühlt auf Raumtemperatur und fügt 400 ml Wasser hinzu. Das gebildete Thiophenderivat wird dreimal mit jeweils 200 ml Methyl-tert.-butylketon ausgeschüttelt. Nach dem Waschen mit Wasser und Trocknen der organischen Phase wird eingedampft und der kristalline Rückstand aus Isopropanol/Wasser 7:3 umkristallisiert. Ausbeute: 90 g (74 % d.Th.), Fp. 96-98 °C.

2-Amino-o-chlorbenzoyl-5-(2-carbomethoxyethyl)-thiophen

63 g (0,15 mol) obiger Verbindung werden mit 120 ml Ethanol und 32,5 g Ätzkali in 50 ml Wasser 2 Stunden unter Rückfluß gekocht. Man dampft im Vakuum ein, verdünnt mit 50 ml Wasser und säuert mit HCl an. Die schmierig ausgefallene Säure wird mehrmals mit Essigester ausgeschüttelt. Die Extrakte werden getrocknet und eingedampft, der Rückstand mit 300 ml Toluol und 30 ml Dimethylformamid 2 Stunden unter Rückfluß gekocht. Nach dem Eindampfen auf ca. 50 ml erhält man Kristalle der Monocarbonsäure.
Ausbeute: 20,5 g. Die gereinigte Säure schmilzt bei 171-173 ° C.
Die Rohsäure wird zusammen mit 400 ml absolutem Methanol und 0,4 ml konzentrierter Schwefelsäure 18 Stunden bei Raumtemperatur gerührt. Man gießt nach Abdampfen des Methanols auf Eis, schüttelt mit Methylenchlorid aus und erhält nach erneutem Eindampfen aus Isopropylether 15 g Ester vom Fp. 89-90 ° C.

2-Bromacetylamino-3-o-chlorbenzoyl-5-(2-carbomethoxyethyl)-thiophen

27,8 g (0,09 mol) obigen Esters werden in 700 ml Toluol suspendiert und mit 10 g Natriumbicarbonat in 57 ml Wasser versetzt. Unter Rühren fügt man allmählich bei 40-50 ° C 7,9 ml Bromacetylbromid hinzu und rührt 30 Minuten nach. Man wäscht mit Wasser, trocknet die Toluolphase, dampft im Vakuum ein und bringt mit Isopropylether zur Kristallisation.
Ausbeute: 35-37g, Fp. 104-106 ° C.

2-Aminoacetylamino-3-o-chlorbenzoyl-5-(2-carbomethoxyethyl)-thiophen

35,8 g (0,08 mol) obiger Bromacetylverbindung werden in 700 ml Essigester gelöst und unter Rühren 2 - 3 Stunden bei Raumtemperatur trockener Ammoniak eingeleitet. Man läßt über Nacht stehen, wäscht mit Eiswasser, trocknet, dampft ein und erhält 22 - 25 g ölige Aminoverbindung

7-(2-Carbomethoxyethyl)-5-o-chlorphenyl-thieno-1,4-diazepinon

21,3 g (0,056 mol) obiger Verbindung werden in 500 ml Toluol gelöst und mit 75 g Kieselgel am Wasserabscheider 2 Stunden unter Rückfluß gekocht. Man saugt das $SiO_2$ ab und extrahiert das Diazepin mit heißem Methanol. Nach Eindampfen des Methanols werden 12 - 15 g Diazepin vom Fp. 160-162 ° C erhalten.

7-(2-Carbomethoxyethyl)-5-o-chlorphenyl-thieno-1,4-diazepin-2-thion

10 g (0,03 mol) obigen Diazepinons werden in 100 ml Diglyme mit 6,8 g Phosphorpentasulfid und 5 g Natriumhydrogencarbonat 3 Stunden bei 70 - 80 ° C gerührt. Man gießt die Suspension auf Eis, rührt 30 - 45 Minuten und saugt die Kristalle ab. Nach dem Trocknen erhält man 10 g Thion vom Fp. 185-186 ° C.

2-[4-(2-Chlorphenyl)-9-methyl-6H-thieno[3,2-f][1,2,4]-triazolo[4,3-a][1,4]diazepin-2-yl]ethan-1-carbonsäuremethylester

6,1 g (0,016 mol) obiger Schwefelverbindung werden in 100 ml Tetrahydrofuran gelöst und nach Zugabe von 1 g Hydrazinhydrat 30 Minuten bei 45 - 50 ° C gerührt. Anschließend wird im Vakuum eingedampft. Es hinterbleiben 5 - 5,2 g Öl, die mit Isopropylether kristallisieren (Fp. 175-177 ° C).

Die Hydrazinoverbindung ergibt beim Erhitzen in 35 ml Ortho-Essigsäureester auf 80 ° C, Eindampfen aus Methylenchlorid- Ester 3 g des Triazolodiazepins vom Fp. 114-115 ° C.

Dieselbe Verbindung ist aus dem Thion mit Essigsäurehydrazid zugänglich.

Beispiel 4:

Essigsäure-{7-[4-(2-Chlorphenyl)-9-methyl-6H-thieno-[3,2-f][1,2,4]triazolo[4,3-a][1,4]diazepin-2-yl]-heptylester}

Gemäß H.J. Bestmann et al., Chem. Ber. 104, 65 (1971) unterwirft man Oleylacetat in Methylenchlorid bei -40 °C der Ozonolyse und spaltet das entstandene Ozonid reduktiv mit der äquivalenten Menge Triphenylphosphin, wobei das entstandene Triphenylphosphinoxid gründlich verrieben mit Ether abgeschieden wird. Spuren nicht umgesetzten Triphenylphosphins können in etherischer Lösung mit Methyljodid als Phosphoniumsalz gefällt werden. Die Trennung des Nonanals vom erwünschten 9-Acetoxynonanal erfolgt zweckmäßigerweise durch destillative Trennung in einer Silberspiegelkolonne. Ausgehend von 9-Acetoxynonanal erhält man analog Beispiel 1 jeweils in quantitativen Ausbeuten die Aminothiophenverbindung, die 2-Bromacetylaminoverbindung und die 2-Aminoacetylaminoverbindung, in 76 % Ausbeute den Diazepinonringschluß, in 65 % Ausbeute das Diazepinthion und in 33 % Ausbeute den Essigsäure-{7-[4-(2-chlorphenyl)-9-methyl-6H-thieno-[3,2-f][1,2,4]triazolo[4,3-a][1,4]diazepin-2-yl]-heptylester} als Öl.

$^1$H-NMR ($CDCl_3$ δ ppm 7,26-7,58 (4H, m, Aryl-H); 6,36 (1H, s, Thiophen-H); 4,92 (2H, s, 7-Ring-$CH_2$); 4,06 (2H, t, J = 6 Hz, $OCH_2$); 2,76 (2H, t, J = 6 Hz, Thiophen-$CH_2$);2,72 (3H, s, Triazol-$CH_3$); 2,05 (3H, s, $CH_3$-C = O); 1,15-1,86 (10H, m, $0CH_2$-$(CH_2)_5$-).

Beispiel 5:

2-Methylpropionsäure-{3-[4-(2-chlorphenyl)-9-methyl-6H-thieno[3,2-f][1,2,4]triazolo[4,3-a][1,4]diazepin-2-yl]-propylester}

0,5 g (0,0013 mol) 2-(3-Hydroxypropyl)-4-(2-chlorphenyl)-9-methyl-6H-thieno[3,2-f][1,2,4]triazolo[4,3-a]-[1,4]diazepin (Beispiel 3), 0,12 g (0,0015 mol) Pyridin und 0,2 g (0,0013 mol) Isobuttersäureanhydrid werden 4 Stunden bei 60 °C gerührt. Nach weiteren 12 Stunden Rühren bei Raumtemperatur versetzt man das Reaktionsgemisch mit 20 ml Wasser und extrahiert mit Ether. Nach dem Trocknen und Abziehen des Lösungsmittels wird der ölige Rückstand an Kieselgel mit Methylenchlorid/Methanol (9 : 1) als Eluens chromatographiert und ergibt in quantitiativer Ausbeute die erwünschte Titelverbindung vom Fp. 124-125 °C.

Beispiel 6:

Methansulfonsäure-{3-[4-(2-chlorphenyl)-9-methyl-6H-thieno[3,2-f][1,2,4]triazolo[4,3-a][1,4]diazepin-2-yl]-propylester}

Zu 3,9 g (0,0105 mol) 2-(3-Hydroxy-propyl)-4-(2-chlorphenyl)-9-methyl-6H-thieno[3,2-f][1,2,4]triazolo[4,3-a][1,4]diazepin (Beispiel 3) in 60 ml wasserfreiem Methylenchlorid werden 1,7 g (0,015 mol) Methansulfonsäurechlorid gegeben und bei 10 °C 1,5 g (0,015 mol) Triethylamin zugetropft. Nach 12 Stunden Rühren bei Raumtemperatur wird das Realktionsgemisch gründlich mit Wasser extrahiert, die organische Phase getrocknet, das Lösungsmittel abgezogen und der Rückstand an Kieselgel mit Methylenchlorid/Methanol (9:1) als Eluens chromatographiert. Man erhält 3,8 g (Ausbeute 81 % d.Th.) der Titelverbindung mit dem Fp. 130-135 °C.

Beispiel 7:

2-(3-Jodpropyl)-4-(2-chlorphenyl)-9-methyl-6H-thieno-[3,2-f][1,2,4]triazolo[4,3-a][1,4]diazepin

0,6 g (0,0011 mol) p-Toluolsulfonsäure-{3-[4-(2-chlorphenyl)-9-methyl-6H- tieno[3,2-f][1,2,4]triazolo-[4,3-a][1,4]diazepin-2-yl]-propylester}, hergestellt analog Beispiel 6, und eine Lösung von 0,2 g (0,0014 mol) wasserfreiem Natriumjodid in 15 ml wasserfreiem Aceton werden 2 Stunden bei Raumtemperatur gerührt. Nach dem Abziehen des Lösungsmittels nimmt man den Rückstand in Methylenchlorid/Wasser auf, wäscht die organische Phase mehrmals mit Wasser, trocknet, zieht das Lösungsmittel ab und erhält so 0,2 g (Ausbeute 36 % d. Th.) der amorphen Titelverbindung.

$^1$H-NMR ($CDCl_3$) δ ppm 7,30-7,67 (4H, m, Aryl-H); 6,44 (1H, s, Thiophen-H); 4,94 (2H, s, 7-Ring-$CH_2$); 3,20 (2H, t, J = 7 Hz, $CH_2$-J); 2,92 (2H, t, J = 7 Hz, Thiophen-$CH_2$); 2,71 (3H, s, Triazol-$CH_3$); 2,12 (2H, m, $JCH_2CH_2$-).

Beispiel 8:

2-(7-N-Phthalimidoheptyl)-4-(2-chlorphenyl)-9-methyl-6H-thieno[3,2-f][1,2,4]triazolo[4,3-a][1,4]diazepin

3,0 g (0,006 mol) Methansulfonsäure-{7-[4-(2-chlorphenyl)-9-methyl-6H-thieno[3,2-f][1,2,4]-triazolo[4,3-a][1,4]diazepin-2-yl]-heptylester} und 1,1 g (0,006 mol) Phthalimid-Kalium werden in wasserfreiem Dimethylformamid 5 Stunden bei 60-70 °C gerührt. Nach dem Abkühlen gießt man das Reaktionsgemisch in 150 ml Eiswasser, extrahiert mit Methylenchlorid und filtriert nach dem Trocknen uhnd Einengen über eine kleine, breite Kieselgelsäule unter Verwendung von Methylenchlorid/ Methanol (9:1) als Eluens. Die Phthalimid-Verbindung fällt in quantitativer Ausbeute als Öl an.

$^1$H-NMR ($CDCl_3$) δ ppm 7,62-7,94 (4H, m, Phthal-Aryl-H); 7,26-7,55 (4H, m Aryl-H); 6,36 (1H, s, Thiophen-H); 4,93 (2H, s, 7-Ring $CH_2$); 3,52 (2H, t, J=6 Hz, N-$CH_2$-); 2,75 (2H, t, J=-6 Hz), Thiophen-$CH_2$); 2,69 (3H, s, Triazol-$CH_3$); 1,14-1,93 (10H, m, N-$CH_2$-$(CH_2)_5$-).

Beispiel 9:

2-(7-amino-heptyl)-4-(2-chlorophenyl)-9-methyl-6H-thieno-[3,2-f][1,2,4]-triazolo[4,3-a][1,4]diazepin

31,7 g (0,057 mol) der Phthalimidoverbindung des Beispiels 8 werden in 300 ml Ethanol gelöst und mit 13,8 mol Hydrazinhydrat (0,29 mol) 20 Stunden bei Raumtemperatur gerührt. Der ausgefallene Feststoff wird abgesaugt und das Filtrat eingeengt und mit 2N Salzsäure aufgenommen. Durch Extraktion mit Methylenchlorid läßt sich die nicht umgesetzte Ausgangsverbindung entfernen. Die wäßrige Phase wird alkalisch gestellt und erneut mit Methylenchlorid extrahiert. Nach dem Trocknen und Abziehen des Lösungsmittels erhält man 14,2 g
(Ausbeute 58 % d. Th.) der Aminoverbindung als Öl.

$^1$H-NMR $CDCl_3$) δ ppm 7,28 - 7,63 (4H, m, Aryl-H); 6,38 (1H, s, Thiophen-H); 4,95 (2H, s, 7-Ring-$CH_2$); 2,55 - 3,10 (6H, m $NCH_2$, Thiophen-$CH_2$, $NH_2$); 2,72 (3H, s, Triazol-$CH_3$); 1,07 - 1,90 (10H, m, N-$CH_2$-$(CH_2)_5$-).

Beispiel 10:

2-(7-Acetylaminoheptyl)-4-(2-chlorphenyl)-9-methyl-6H-thieno[3,2-f][1,2,4]triazolo[4,3-a][1,4]diazepin

Zu 1,3 g (0,003 mol) der Aminoverbindung des Beispiels 9 und 0,4 ml Triethylamin (0,003 mol) in 35 ml wasserfreiem Methylenchlorid werden 0,2 ml (0,003 mol) Acetylchlorid in 5 ml wasserfreiem Methylenchlorid zugetropft und 12 Stunden bei Raumtemperatur gerührt. Nach dem Waschen mit Wasser, Trocknen und Entfernen des Lösungsmittels chromatographiert man den Rückstand an Kieselgel mit Methylenchlorid/Methanol (95 : 5) als Eluens und erhält 1,1 g der Acetylaminovberbindung (Ausbeute 77 % d.Th.) als Öl.

$^1$H-NMR ($CDCl_3$) δ ppm 7,30 - 7,58 (4H, m, Aryl-H); 6,37 (1H, s, Thiophen-H); 5,67 (1H, s, breit, NH); 4,94 (2H, s, 7-Ring-$CH_2$); 3,24 (2H, m, N-$CH_2$); 2,76 (2H, t, J=6 Hz, Thiophen $CH_2$); 2,72 (3H, s, Triazol-$CH_3$); 1,98 (3H, s, $CH_3C=O$); 1,21 - 1,83 (10H, m, N-$CH_2(CH_2)_5$-).

Beispiel 11

2-(3-(N-Morpholinyl)propyl)-4-(2-chlorphenyl)-9-methyl-6H-thieno[3,2-f][1,2,4]triazolo[4,3-a][1,4]diazepin

2,7 g (0,006 mol) der Verbindung des Beispiels 6 und 2,6 g (0,030 mol) Morpholin werden in 50 ml Dioxan 6 Stunden zum Rückfluß erhitzt, anschließend das Lösungsmittel abgezogen und der Rückstand in Methylenchlorid/Wasser aufgenommen. Die organische Phase wird gründlich mit Wasser ausgeschüttelt, getrocknet und das Lösungsmittel entfernt. Der Rückstand wird an Kieselgel mit Methylenchlorid/. Methanol (9:1) als Eluens chromatographiert. Man erhält 1,3 g der Morpholinverbindung (Ausbeute 50 % d.Th.) vom Fp. 162-164 °C.

Durch Lösen der Substanz in methanolischer Salzsäure und Ausfällen mit Ether erhält man das Hydrochlorid der Titelverbindung vom FP. 95 °C (Zersetzung).

Beispiel 12

2-(6-Formylhexyl)-4-(2-chlorphenyl)-9-methyl-6H-thieno[3,2-f][1,2,4]triazolo[4,3-a][1,4]diazepin

Zu 1,5 g (0,0075 mol) Pyridinchlorochromat in 20 ml Methylenchlorid tropft man 2,1 g (0,005 mol) 2-(7-Hydroxyheptyl)-4-(2-chlorphenyl)-9-methyl-6H-thieno[3,2-f][1,2,4]triazolo[4,3-a][1,4]diazepin (hergestellt z.B. aus der Verbindung des Beispiels 4 durch alkalische Verseifung) in 5 ml Methylenchlorid gelöst zu, rührt noch 90 Minuten bei Raumtemperatur und versetzt die Lösung mit 50 ml Ether. Das ausgefallene schwarze, harzige Öl wird mehrmals gründlich mit Ether ausgerührt, die Etherlösungen werden vereinigt, über Kieselgur abgesaugt, das Filtrat eingeengt und der Rückstand an Kieselgel mit Methylenchlorid/Methanol (9:1) als Eluens chromatographiert. Man erhält 0,6 g (Ausbeute 29 % d.Th.) des Aldehyds als farbloses Öl.

$^1$H-NMR ($CDCl_3$) $\delta$ ppm 9,77 (1 H, t, J = <2 Hz, CHO); 7,25 - 7,59 (4H, m, Aryl-H); 6,38 (1 H, s, Thiophen-H); 4,94 (2H, s, 7-Ring-$CH_2$); 2,76 (2H, t, J = 6 Hz, Thiophen-$CH_2$); 2,71 (3H, s, Triazol-$CH_3$); 2,41 (2H, m, -$CH_2C=O$); 1,17 - 1,90 (8H, $O=CCH_2(CH_2)_4$-)

Beispiel 13:

2-(3-Acetoxypropyl)-4-(2-chlorphenyl)-9-brom-6H-thienol[3,2-f][1,2,4]triazolo[4,3-a][1,4]diazepin

4 g (0,010 mol) 2-(3-Acetoxypropyl)-4-(2-chlorphenyl)-6H-thieno[3,2-f][1,2,4]triazolo[4,3-a][1,4]diazepin in 60 ml wasserfreiem Chloroform, 1,9 g (0,012 mol) Brom und 1,2 g (0,015 mol) Pyridin werden 20 Stunden bei Raumtemperatur gerührt. Anschließend wird das Reaktionsgemisch gründlich mit Wasser ausgeschüttelt, die organische Phase getrocknet, eingeengt und der Rückstand an Kieselgel mit Methylenchlorid/Methanol (9:1) als Eluens chromatographisch gereinigt. Man erhält 2,3 g (Ausbeute 48 % d.Th.) der 9-Brom-Verbindung als Öl.

$^1$H-NMR ($CDCl_3$) $\delta$ ppm 7,30 - 7,62 (4H, m, Aryl-H); 6,43 (1H, s, Thiophen-H); 4,90 (2H, s, 7-Ring-$CH_2$); 4,11 (2H, t, J = 7 Hz, -0-$CH_2$); 2,89 (2H, t, J = 7 Hz, Thiophen $CH_2$); 2,03 (3H, s, $CH_3C=O$); 2,00 (2H, m, $OCH_2CH_2$-).

Beispiel 14

2-(3-Hydroxypropyl)-4-(2-chlorphenyl)-9-methyl-6H-thieno[3,2-f]imidazo[1,2-a][1,4]diazepin

Zu 15 g (0,038 mol) 2-(3-Acetoxypropyl)-4-(2-chlorphenyl)-6H-7,8-dihydrothieno[3,2-f][1,4]diazepin-7-thion in 200 ml wasserfreiem Dioxan tropft man bei Raumtemperatur 6,3 g (0,114 mol) Propargylamin ein, rührt 20 Stunden und zieht das Lösungsmittel ab. Der Rückstand wird in Methylenchlorid/Wasser aufgenommen, die organische Phase gründlich mit Wasser gewaschen, getrocknet und eingeengt. Das erhaltene Rohprodukt wird mit Isopropylether verrieben, wobei es kristallisiert. Man erhält 13,5 g 2-(3- Acetoxypropyl)-4-(2-chlorphenyl-6H-7-propargylamino-thieno[3,2-f][1,4]-diazepin (Ausbeute 86 % d.Th.) vom Fp. 122-124 ° C.

11 g (0,027 mol) der so erhaltenen Zwischenverbindung und 55 ml konzentrierter Schwefelsäure werden 10 Minuten in einem vorgeheizten Ölbad auf 100 ° C erhitz, auf Eis gegossen und mit konzentriertem Ammoniak auf pH 10 eingestellt. Die Extraktion mit Methylenchlorid ergibt 1,2 g Rückstand, der in 100 ml Methanol aufgenommen und mit 0,2 g Kaliumhydroxid eine Stunde bei 60 ° C gerührt wird. Nach dem Abziehen des Methanols nimmt man den Rückstand in Methylenchlorid/Wasser ayf, wäscht die organische Phase gründlich mit Wasser, trocknet, zieht das Lösungsmittel ab und chromatographiert den Rückstand an Kieselgel mit Methylenchlorid/Methanol (9:1) als Eluens und erhält so 0,7 g der Imidazo-Verbindung (Ausbeute 7 % d.Th.) vom Fp. 131-134 ° C.

$^1$H-NMR ($CDCl_3$)$\delta$ ppm 7,22-7,58 (4H, m, Aryl-H); 6,89 (1H, q, J<1 Hz, Imidazol-H); 6,38 (1 H, s, Thiophen-H); 4,77 (2H, s, 7-Ring $CH_2$); 3,68 (2H, t, J = 7 Hz, $OCH_2$); 2,86 (2H, t, J = 8 Hz, Thiophen-$CH_2$); 2,42 (3H, d, J = <1 Hz, Imidazol,$CH_3$); 1,88 (2H, m -$OCH_2$-$CH_2$-); 2,00 (1H, s, breit, OH).

Beispiel 14a

2-(2-Methoxycarbonyethyl)-7-ethoxycarbonyl-6-(2-chlorphenyl)-6H-thieno[3,2-f]imidazo[1,5-a][1,4]diazepin

9,0 (0.025 Mol) 7-(2-Methoxycarbonyethyl)-5-chlorphenyl)-1,2-dihydro-3H-thieno[2,3-e][1,4]diazepin-2-on (W.D. Bechtel und K.H.Weber, J.Pharmac. Sci. 74, 1265 (1985) vom Fp. 152 - 154 ° C werden in 25 ml Dimethylformamid suspendiert und unter Stickstoff mit 3,5 g Kaliumtert.-Butylat versetzt. Man rührt 10-15 min, kühlt auf -30 ° und gibt innerhalb 10-15 min 4,7g Diethylchlorphosphat hinzu.

Zu dieser Lösung fügt man das aus 3,3g Kalium-tert.-Butylat und 3.3g Isocyanessigsäureethylester bei -40 ° in 22 ml Dimethylformamid bereitete Gemisch zu und hält die Temperatur 2 Stunden auf -10 ° C und läßt anschließend das Reaktionsgemisch auf Raumtemperatur erwärmen. Unter Kühlen werden 2,5 ml Eisessig und anschließend 350 ml Wasser zugegeben und dann das Reaktionsgemisch mit Essigester extrahiert. Nach Waschen, Trocknen und Eindampfen der Essigesterphasen wird der Rückstand über $SiO_2$ chromatographiert. Aus dem Eluat werden nach Etherzusatz 4,0g Kristalle vom Fp: 139 - 140 ° C erhalten.

$C_{22}H_{20}ClN_3O_4S$(457,9)

| | | | | |
|---|---|---|---|---|
| C 57.7 | H 4.40 | N 9.18 | C 7.74 | S 7.00 |
| C 58.0 | H 4.44 | N 9.04 | C 7.55 | S 6.87 |

Beispiel 14b

2-[2-(Morpholin-4-yl-carbonyl)-ethyl]-4-(2-chlorophenyl)-6H-thieno-[3,2-f]imidazo[1,5-a][1,4]diazepin.

3,5 g (0.0076 Mol) des Diesters hergestellt nach Beispiel 14a werden in 75 ml Tetrahydrofuran mit 0,75 g NaOH in 75 ml Methanol und 40 ml Wasser 1 Stunde unter Rückfluß erhitzt. Man säuert mit 2,6 ml Eisessig an und engt die Lösung ein. Die nach Zugabe von 50 ml Wasser ausfallenden Kristalle der Dicarbonsäure schmelzen bei 275 ° C unter Zersetzung.

Ausb. 3.0 g.

Diese Verbindung wurde zusammen mit 100 ml 1,2,4-Trichlorbenzol unter Stickstoff 1 Stunde erhitzt. Man filtriert und versetzt das Filtrat mit Petroläther, wobei die 2-(2-Carboxyethyl)-Verbindung ausfällt. Aus dieser Carbonsäure erhält man analog Beispiel 3 das Morpholid vom Fp. 158 ° C.

$C_{22}H_{21}ClN_4O_2S$ (440.9)

| | | | |
|---|---|---|---|
| C 59.12 | H 4,80 | N 12,71 | S 7.27 |
| C 60.12 | H 4.91 | N 12.54 | S 7.06 |

Beispiel 14c

2-(2-Diethylaminocarbonylethyl)-6-(2-chlorphenyl)-9-methyl-6H-thieno[3,2-f]imidazo[1,2-a][1,4]diazepin

Ausgehend von 7-(2-Methoxycarbonylethyl)-5-(2-chlorphenyl)-1,2-dihydro-3H-thieno [2,3-e][1,4]-diazepin-2-thion (W.D. Bechtel und K.H. Weber, J. Pharm. Sci 74, 1265 (1985) vom Fp 190 ° C enthält man analog dem in Beispiel 14 beschriebenen Syntheseweg das entsprechende Diethylamid vom Fp. 201-203 ° C.

Beispiel 14d

2-[2-(Morpholin-4-yl-carbonyl)-ethyl]-6-(2-chlorphenyl)-9-methyl-6H-thieno[3,2-f][imidazo[1,5-a][1,4]diazepin

Aus den oben beschriebenen Carbonsäure erhält man die Titelverbindung bei Verwendung von Morpholin an Stelle von Diethylamin. Fp: 248-250 ° C.

Beispiel 14e

2-[1-(Morpholin-4-yl-carbonyl)-prop-1-en-2-yl]-4-(2-chlorphenyl)-9-methyl-6H-thieno[3,2-f][1,2-4]triazolo[4,3-a]-[1,4]diazepin

a) 48.8 g (0.2 Mol) 2-Acetylamino-3-(2-chlorbenzoyl)-thiophen werden mit 500 ml Acetanhydrid und 6 ml 85%ige Phosporsäure 1 1/2 Std. unter Rückfluß erhitzt. Man dampft i. Vak. ein, versetzt den Rückstand mit 200 ml Wasser und neutralisiert mit 40%iger Natronlauge. Die Kristalle werden abgesaugt und mit Wasser, Methanol und Ether gewaschen.

Ausbeute: 49-51 g, Fp.: 228 - 230 ° C

Unter Stickstoff werden 50.3 g dieser Verbindung in eine Lösung von 10 g K0H gelöst in 300 ml Methanol gegeben und 1 bis 2 Stunden bei Raumtemperatur gerührt. Man neutralisiert mit 2n Salzsäure und extrahiert das Aminoderivat mit Methylenchlorid. Nach Chromatographie des Rückstands erhält man 43 g 5-Acetyl-2-amino-3-(2- chlorbenzoyl)thiophen vom Fp: 190 - 191 ° C.

b) 5.5 g (0.02 Mol) dieser Verbindung werden zum Schutz der Acetylgruppe in 50 ml Chloroform aufgenommen und nach Zugabe von 2 ml 1,3-Propandithiol bei Raumtemperatur unter Einleiten von trockenem HCl-Gas 5 Min. gerührt. Anschließend extrahiert man nacheinandere mit Wasser, verdünnter Natronlauge und nochmals mit Wasser, trocknet die organische Phase, verdampft das Lösungsmittel und chromatographiert den Rückstand über $SiO_2$ (Methylenchlorid/Ethanol). Mit Ether erhält man aus dem Rückstand der Hauptfraktion 4.3 g Kristalle vom Fp. 162-163 ° C. Diese Verbindung kann auf analogem Weg, wie zuvor (z.B.Beispiel 3 beschrieben) zum Diazepinon (Fp.260 ° C), Diazepinthion (Fp. 218 ° C) und dem Triazolodiazepin (Fp. 212 ° C) umgesetzt werden

c) Zur Entfernung der Schutzgruppe werden 9 g (0.02 Mol) des Triazolodiazepins mit 20 g Chloramin T in 200 ml Methanol 2 Stunden bei Raumtemperatur gerührt. Nach dem Chromatographieren über $SiO_2$ (Methylenchlorid/Ethanol 98 :2) erhält man 3,5-4 g, 2-Acetyl-4-(2-chlorphenyl)- 9-methyl-6H-thieno[3,2-f]-[1,2,4]triazolo[4,3-a][1,4]diazepin.

3,6 g (0.01 Mol) dieser Verbindung werden in 5 ml Benzol suspendiert und nach Zugabe von 2.25 g Phosphoroessigsäure-triethylester zu einer vorgelegten Lösung von 0.23 g Natrium in 5 ml Ethanol getropft. Man läß 20 Stunden bei 40 ° C sintern und arbeitet auf. Der erhaltene Ester wird mit ethanolischer Kalilauge verseift. Nach dem Ansäuern und Aufarbeiten erhält man 1.4 -1.6 g Kristalle vom Fp. 296 - 298 ° C.

Diese Carbonsäure kann analog zuvor beschriebener Beispiele in das Morpholid vom Fp. 194 - 196 ° C überführt werden.

Beispiel 14f

2-[2-(Morpholin-4-yl-carbonyl)-pent-4-en-1-yl]-4-(2-chlorphenyl)-9-methyl-6H-thieno[3,2-f][1,2-4]triazolo[4,3-a][1,4]diazepin

20 g (0.05 Mol) 2-Amino-3-(2-chlorbenzoyl)-5-(2,2-dicarboethoxyethyl)-thiophen (W.D.Bechtel u. K.H.Weber, J.Pharmac. Sci. 74, 1265 (1985) werden in 100 ml Tetrahydrofuran suspendiert. Diese Suspension tropft man zu 2,5 g Natriumhydrid, suspendiert in Tetrahydrofuran (100 ml) und rührt 1 Stunde bei 30 ° C. Nach Zugabe von 4.5 ml Allylbromid (0.05 Mol) wird 2 Stunden bei Raumtemperatur gerührt, das Reaktionsgemisch eingeengt, der Rückstand mit Methylenchlorid aufgenommen und mit Wasser gewaschen. Aus der organischen Phase erhält man 13 g Kristalle vom Fp. 151-152 ° C. 13 g (0.03 Mol) dieses Dicarbonsäureesters werden mit 30 ml Ethanol, 6 ml Wasser mit 3.8 g Ätzkali verseift. Man erhält 11.3 g Dicarbonsäure, die sich durch einstündiges Erhitzen in 20 ml DMF (120-130 ° C) decarboxyliert wird und 6 - 7 g Monocarbonsäure vom Fp. 202 - 203 ° C ergeben.

Der hieraus erhältliche Methylester ist ein Öl. Dieses wird, analog wie oben beschrieben, bromacetyliert, aminiert kund zum Diazepinon cyclisiert.

Fp. 162-165 ° C.

Das aus dem Diazepinon mit Phorphorpentasulfid zugängliche Diazepinthion schmilzt bei 179 ° C und ergibt die Triazolo-thieno-diazepincarbonsäure vom Fp. 207 - 208 ° C. Hieraus erhält man Morpholid über das Imidazolid gemäß der früher beschriebenen Methode als zähes Öl.

Beispiel 14g

2-[2-(Benzyl-2-(morpholin-4-yl-carbonyl)-ethyl]-4-(2-chlorphenyl)-9-methyl-6H-thieno[3,2-f][1,2,4]triazolo[4,3-a][1,4]diazepin

Ausgehend von 2-Amino-3-(2-chlorbenzoyl)-5-(2,2-dicarbethoxyethyl)-thiophen erhält analog Beispiel 14 f durch Umsetzung mit Benzylchlorid die Titelverbindung über folgende Stufen.
Diazepinon Fp. 158-161 ° C
Diazepinthion Fp. 170-172 ° C
Triazolothienodiazepincarbonsäure Fp. 158-162 ° C

Beispiel 14h

2-[2-(N,N-Diethylaminocarbonyl)-n-propyl]-4-(2-chlorphenyl)-9-methyl-6H-thieno[3,2-f][1,2,4]triazolo[4,3-a]-[1,4]diazepin

2-Amino-3-(2-chlorbenzoyl)-5-(2,2-dicarboethoxyethyl)-thiophen ergibt mit Methyljodid die entsprechende C-Methylverbindung und weiter die Titelverbindung nach vorbeschriebenem Weg über folgende Stufen:
Dicarbonsäurediethylester Fp. 181-182 ° C
Monocarbonsäure Fp. 161-162 ° C
Diazepinoncarbonsäuremethylester Fp. 179-180 ° C
Triazolothienodiazepincarbonsäuremethylester als Öl
Titelverbindung (Diethylamid) Fp. 102-103 ° C

Beispiel 14i

2-[2-(Morpholin-4-yl-carbonyl)-n-propyl]-4-(2-chlorphenyl)-9-methyl-6H-thieno[3,2-f][1,2,4]triazolo[4,3-a][1,4]-diazepin

In Analogie zu Beispiel 14h erhält man die Titelverbindung unter Verwendung von Morpholin anstelle von Diethylamin. Öl.

Beispiel 14j

2-(2-(N,N-Diethylaminocarbonylethyl)-4-(2-chlorphenyl)-9-methyl-4,5-dihydro-6H-thieno[3,2-f][1,2,4]triazolo-[4,3-a][1,4]diazepin

2,5 g (0,006 Mol) Diethylamid (hergestellt aus der Carbonsäure, W.D. Bechtel u. K.H. Weber, J. Pharm. Sci 74, 1265 (1985), mit Diethylamin und Dicyclohexylcarbodiimid) werden zusammen mit 2 g Zinkstaub, 50 ml Eisessig und 50 ml Methylenchlorid 15 Stunden bei Raumtemperatur gerührt. Anschließend filtriert man über Kieselgur, wäscht mit Methylenchlorid und macht das Filtrat mit Ammoniak alkalisch. Die Methylenchloridphase wird abgetrennt, mit Wasser gewaschen, getrocknet, eingedampft und der Rückstand über $SiO_2$ chromatographiert. Man erhält als Hauptfraktion 0,8 g Öl.

Beispiel 14k

2-[2-(4,4-Dimethyl-2-oxazolin-2-yl)-ethyl]-4-(2-chlorphenyl)-9-methyl-6H-thieno[3,2-f][1,2,4]triazolo-[4,3-a]-[1,4]diazepin

3 g (7,7 mMol) 2-[4-(2-chlorphenyl)-9-methyl-6H-thieno-[3,2-f][1,2,4]triazolo-[4,3-a][1,4]diazepin-2-yl]-ethan-1-carbonsäure, 0,69 g (7,7 mmol) 2-Amino-2-methyl-propan-1-ol, 2,35 g (23 mmol) Triethylamin und 4,8 g Tetrachlorkohlenstoff werden in 15 ml Pyridin/Acetonitril (1:1) bei Raumtemperatur gerührt. Zu dieser Mischung tropft man innerhalb von 2 Stunden eine Lösung von 6,2 g (23,6 mmol) Triphenylphosphin in 15 ml der obigen Pyridin/Acetonitril-Mischung.

Nach 15 Stunden wird die Suspension eingeengt, der Rückstand mit Ether/Essigester-Gemischen (1:1) extrahiert. Die Extrakte werden eingeengt und der verbliebene Rückstand an Kieselgel mit Dichlormethan/Methanol (95 : 5) chromatographiert. Die sauberen Fraktionen werden eingeengt und durch Umkristallisieren aus Ether gewinnt man 1,9 g (56 %) der Titelverbindung vom Fp. 168 ° C.

$C_{22}H_{22}ClN_5OS$ (439,97)

| | | | | | |
|---|---|---|---|---|---|
| ber.: | C 60,05 | H 5,04 | N 15,92 | Cl 8,06 | S 7,29 |
| gef.: | C 59,53 | H 5.08 | N 15,64 | Cl 8,11 | S 7,17 |

Beispiel 14 l

2-[3-(N-Morpholinyl-n-propyl]-4-(2-chlorphenyl)-9-methyl-4,5-dihydro-6H-thieno[3,2-f][1,2,4]triazolo[4,3-a]-[1,4]diazepin

1,2 g (0.0027 mol)2-[3-(N-Morpholinyl)-n-propyl]-4-(2-chlorphenyl)-9-methyl-6H-thieno[3,2-f][1,2,4]-triazolo[4,3-a][1,4]diazepinin 20 ml wasserfreiem THF werden in eine vorgelegte Suspension von 0,1 g (0.0027 mol) Lithiumaluminiumhydrid in 20 ml wasserfreiem THF bei Raumtemperatur zugetropft und 1 Stunde zum Rückfluß erhitzt. Man zersetzt das Reaktionsgemisch mit 0.1 ml Wasser, 0.1 ml 15%ige Natronlauge und 0.3 ml Wasser, rührt 30 Minuten und saugt den ausgefallenen Niederschlag ab. Das Filtrat wird eingeengt, in Methylenchlorid aufgenommen, gewaschen, getrocknet und das Solvens abgezogen. Der Rückstand wird als Kieselgel mit Methylenchlorid/Methanol 95 : 5 als Eluens chromatographiert. Man erhält 0.16 g (Ausbeute 13 %) der gewünschten Verbindung vom FP. 135-138° C

Beispiel 14m

2-[(2-Phenyl-1,3-dioxolan-4-yl)-methyl]-4-(2-chlorphenyl)-9-methyl-6H-thieno[3,2-f][1,2,4]triazolo[4,3-a][1,4]-diazepin

45,7 g (0.4 mol) 3,4-Dihydro-2H-pyran-2-methanol werden in 100 ml wasserfreiem Methylenchlorid bei -40° der Ozonolyse unterworfen. Anschließend werden 104,9 g (0.4 mol) Triphenylphosphin in 70 ml wasserfreiem Methylenchlorid zugetropft und das Reaktionsgemisch im Verlaufe von 3 Stunden langsam auf Raumtemperatur gebracht. Nach 12 Stunden wird das Solvens abgezogen und der Rückstand gründlich mit Ether digeriert und das ausgefallene Triphenylphosphinoxid abgesaugt. Nicht umgesetzte Triphenylplhosphinreste werden mit Methyljodid als Phosphoniumsalz in Ether ausgefällt und abgesaugt. Vom Filtrat wird das Solvens abgezogen und der Rückstand im Vakuum fraktioniert. Man erhält 32 g (55 % Ausbeute) des 4-Formyloxy-5-hydroxypentanals vom $Kp_{0,1}$ = 82-92° C.

23 g (0.157 mol) des Aldehyds, 28 g (0.137 mol) o-Chlorcyanoacetophenon, 5 g (0.157 mol) Schwefel und 16 g (0.157 mol) Triethylamin werden in 100 ml DMF in üblicher Weise zum Thiophenringschluß eingesetzt und das entstandene 2-Amino-3-(2-chlorbenzoyl)-5-(2-formyloxy-3-hydroxy-n-propyl)-thiophen als Rohprodukt mit 15 g (0,16 mol) Kaliumhydroxid in 500 ml Methanol 2 Stunden zum Rückfluß erhitzt. Nach dem Abziehen des Solvens wird der Rückstand in Essigester aufgenommen, mit Wasser gewaschen, getrocknet und nach dem Entfernen des Lösungsmittels an Kieselgel mit Essigester als Eluens chromatographiert. Man erhält 19,9 g (Gesamtausbeute 39 %) an 2-Amino-3-(2-chlorbenzoyl)-5-(2.3-dihydroxy-n-propyl)-thiophen vom Fp. 136-137° C.

5 g (0.016 mol) der Dihydroxythiophenverbindung, 1,7 g (0,016 mol) frisch destillierter Benzaldehyd und eine Spatelspitze p-Toluolsulfonsäure werden am Wasserabscheider in 250 ml Toluol zum Rückfluß erhitzt. Nach 2,5 Stunden wird die Toluolphase mit Pyrrolidin alkalisch gestellt, mit Wasser gewaschen, getrocknet und das Solvens abgezogen. Der Rückstand wird an Kieselgel mit Methylenchlorid/Methanol 9 : 1 als Eluens chromatographiert und ergibt 3,8 g (59 % Ausbeute) der 1,3-Dioxolan-Verbindung als rötliches Öl (Diastereomerengemisch).

Das so erhaltene 2-Amino-3-(2-chlorbenzoyl)-5-[(2-phenyl-1,3- dioxolan-4-yl)-methyl]-thiophen wird, wie in den vorhergehenden Beispielen beschrieben, zur Titelverbindung umgesetzt.

Beispiel 14 n

2-(2-Ethoxycarbonylethyl)-3,9-dimethyl-4-(2-chlorphenyl)-6H-thieno[3,2-f][1,2,4]triazolo[4,3-a][1,4]diazepin.

Ausgehend von 5-(2-chlorphenyl)-6-methyl-7-(2-ethoxycarbonyl-ethyl)-1,2-dihydro-3H-thieno[2,3-e][1,4]-diazepin-2-on erhält man in Analogie zu dem in Beispiel 3 beschriebenen Verfahren die Titelverbindung.

Die Ausgangsverbindung wird wie folgt hergestellt:

11,6 g (0,073 mol) 5-Oxo-hexansäureethylester werden mit äquimolaren Mengen o-Chlor-2-cyano-acetophenon und Schwefel unter Zusatz von 6.7 ml Diethylamin in 30 ml Ethanol 5 Stunden bei 60° C nach Gewald

umgesetzt. Der beim Einengen der Reaktionslösung erhaltene Rückstand wird an einer Kieselgelsäule mit Chloroform als Elutionsmittel chromatographiert. Das so erhaltene Aminoketon wird analog Beispiel 3 in die Titelverbindung überführt. Das Diazepinon hat einen Schmelzpunkt von 173-176°C (Toluol/Ether).

$C_{19}H_{19}ClN_2O_3S$ (390.9)

| | | | | | |
|---|---|---|---|---|---|
| Ber.: | C 58.38 | H 4,90 | Cl 9,07 | N 7,17 | S 8,20 |
| Gef.: | C 58.08 | H 4,83 | Cl 8,94 | N 7,11 | S 8,14 |

Beispiel 14 o

2-[2-(Morpholin-4-yl-carbonyl)-2-(ethoxycarbonyl)ethyl]-4-(2-chlorphenyl)-9-methyl-6H-thieno[3,2-f][1,2,4]-triazolo[4,3-a][l,4]diazepin

1 g (0,0021 mol) 2-[2,2-Di(ethoxycarbonyl)-ethyl]-4-(2-chlorphenyl)-9-methyl-6H-thieno[3,2-f][1,2,4]-triazolo[4,3-a][1,4]diazepinund 5 g Morpholin werden 10 Stunden bei 120°C gerührt. Man verdünnt mit 100 ml Wasser, schüttelt mehrmals mit Methylenchlorid aus, wäscht die organische Phase mit Wasser, trocknet und engt ein. Der so erhaltene Rückstand wird über eine Kieselgelsäule chromatographiert (Methylenchlorid/Methanol 97:3). Nach üblicher Aufarbeitung erhält man die Titelverbindung als amorphes Pulver.

In den Tabellen werden mitunter die folgenden Abkürzungen verwendet:

Et = Ethyl
Me = Methyl
iPr = iso-Propyl
t-Bu = tert.-Butyl
Ac = Acetyl

In Analogie zu den in den Beispielen beschriebenen Verfahren werden die folgenden Zwischenverbindungen hergestellt:

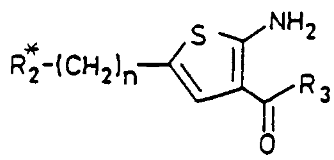

Tabelle 1

| Nr. | n | $R_3$ | $R_2^*$ | Fp. |
|---|---|---|---|---|
| 1 | 2 | 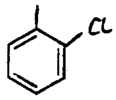 | $-SO_2-$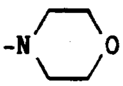 | Öl |
| 2 | 3 | 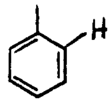 | -OH | 96-98°C |
| 3 | 3 | " | $-O-\overset{O}{\overset{\|}{C}}-CH_3$ | Öl |
| 4 | 3 | 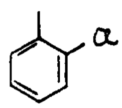 | -OH | 146-148°C |
| 5 | 7 | " | $-O-\overset{O}{\overset{\|}{C}}-CH_3$ | Öl |
| 5a | 3 | 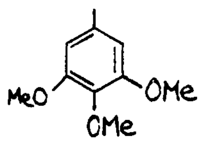 | -OH | Öl |
| 5b | 3 | " | $-OCOCH_3$ | Öl |
| 5c | 1 | 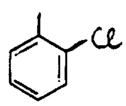 | $-CHOHCH_2OH$ | 136-137°C |
| 5d | 1 | " | 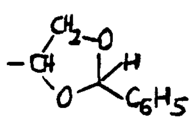 | Öl |
| 5e | 0 | " | 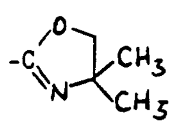 | 228-230°C |
| 5f | 1 | " | 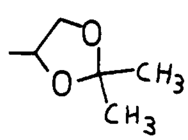 | Öl |

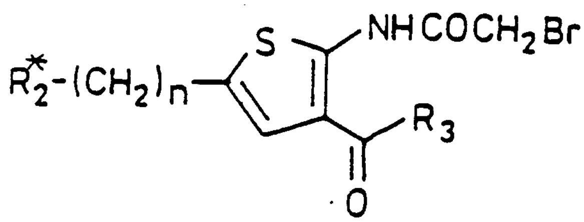

Tabelle 2

| Nr. | n | $R_3$ | $R_2^*$ | Fp. |
|---|---|---|---|---|
| 6 | 2 | 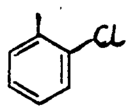 | $-SO_2-$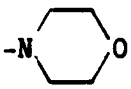 | Öl |
| 7 | 3 | 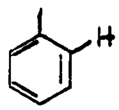 | $-O-\overset{\overset{O}{\|}}{C}-CH_3$ | Öl |
| 8 | 3 | 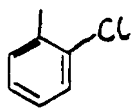 | $-O-\overset{\overset{O}{\|}}{C}-CH_3$ | Öl |
| 9 | 7 | " | $-O-\overset{\overset{O}{\|}}{C}-CH_3$ | Öl |
| 9a | 0 | " | 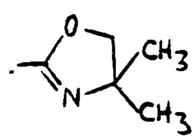 | Öl |
| 9b | 1 | " | $-CH(COOC_2H_5)_2$ | 83-85°C |
| 9c | 1 | " | 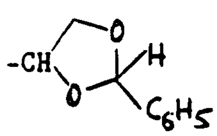 | Öl |
| 9d | 1 | " | 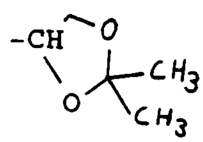 | Öl |

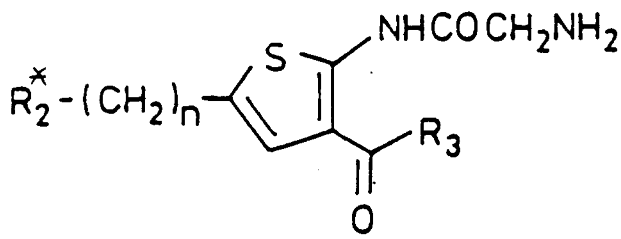

Tabelle 3

| Nr. | n | $R_3$ | $R_2^*$ | Fp. |
|---|---|---|---|---|
| 10 | 2 | 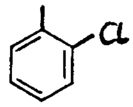 | $-SO_2-$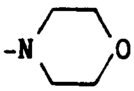 | 90-92°C |
| 11 | 3 | 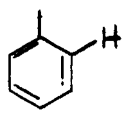 | $-O-C-CH_3$<br>‖<br>O | Öl |
| 12 | 3 | 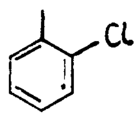 | $-O-C-CH_3$<br>‖<br>O | Öl |
| 13 | 7 | " | $-O-C-CH_3$<br>‖<br>O | Öl |
| 13a | 0 | " | 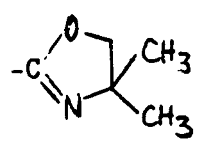 | Öl |
| 13b | 1 | " | $CH(COOC_2H_5)_2$ | Öl |

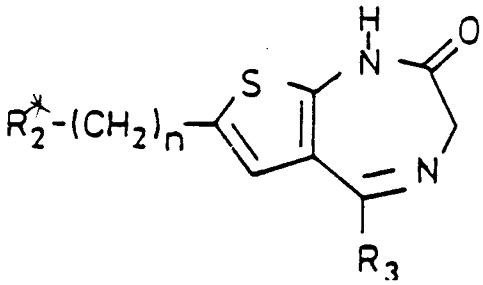

<u>Tabelle 4</u>

| Nr. | n | $R_3$ | $R_2^*$ | Fp. |
|---|---|---|---|---|
| 14 | 2 | 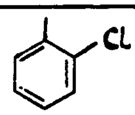 | $-SO_2-$ 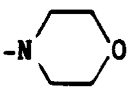 | >200°C |
| 15 | 3 | 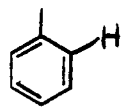 | $-O-\underset{\overset{\|}{O}}{C}-CH_3$ | 122-124°C |
| 16 | 3 | 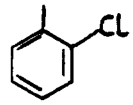 | $-O-\underset{\overset{\|}{O}}{C}-CH_3$ | 153-155°C |
| 17 | 7 | " | $-O-\underset{\overset{\|}{O}}{C}-CH_3$ | Öl |
| 17a | 0 | " | 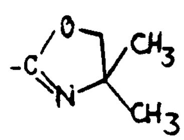 | 242-244°C |
| 17b | 1 | " | $CH(COOC_2H_5)_2$ | 144-145°C |
| 17c | 1 | " | 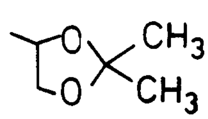 | >200°C |
| 17d | 1 | " | $\underset{OH}{-CH} - \underset{OH}{CH_2}$ | Öl |

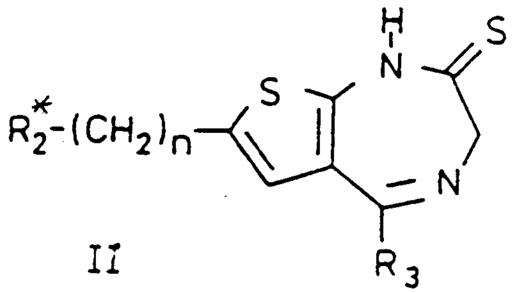

II

Tabelle 5

| Nr. | n | $R_3$ | $R_2^*$ | Fp. |
|---|---|---|---|---|
| 18 | 2 | 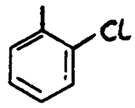 | $-SO_2-$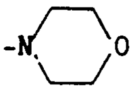 | 215-218°C |
| 19 | 3 | 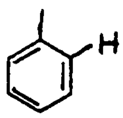 | $-O-\underset{\underset{O}{\|}}{C}-CH_3$ | 170-172°C |
| 20 | 3 | 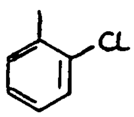 | $-O-\underset{\underset{O}{\|}}{C}-CH_3$ | 184-185°C |
| 21 | 7 | " | $-O-\underset{\underset{O}{\|}}{C}-CH_3$ | 132-135°C |
| 21a | 0 | " | 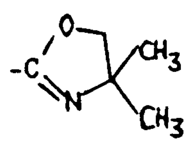 | 223-225°C |
| 21b | 1 | " | $-CH(COOC_2H_5)_2$ | 158-160°C |
| 21c | 1 | " | 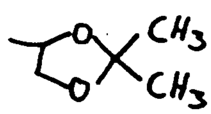 | 180°C |

In Analogie zu den zuvor beschriebenen Beispielen können beispielsweise die Verbindungen der allgemeinen Formel I hergestellt werden:

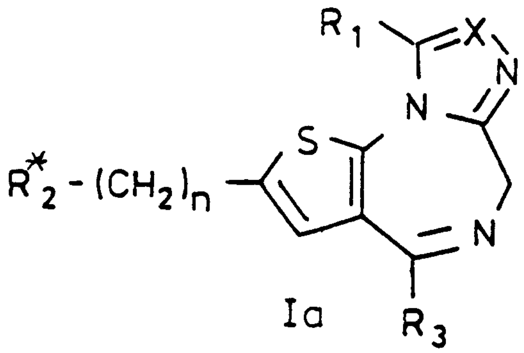

Ia

Tabelle 6

| Nr. | $R_1$ | $R_2^*$ | $R_3$ | X | n | Fp.°C |
|---|---|---|---|---|---|---|
| 15 | $CH_3$ | OH | 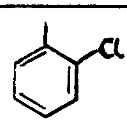 | N | 7 | Öl |
| 16 | $CH_3$ | -Cl | " | N | 3 | 142-144 |
| 17 | $CH_3$ | -Cl | " | N | 7 | 84-87 |
| 18 | $CH_3$ | J | " | N | 7 | |
| 19 | $CH_3$ | $-NH_2$ | " | N | 3 | Öl |

| Nr. | $R_1$ | $R_2^*$ | $R_3$ | X | n | Fp.°C |
|---|---|---|---|---|---|---|
| 20 | $CH_3$ | $-SO_2-$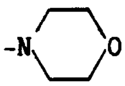 | " | N | 2 | 126-128 |
| 21 | $CH_3$ | $-O-SO_2-i-Pr$ | " | N | 3 | |
| 22 | $CH_3$ | $-O-SO_2-$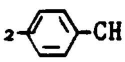$-CH_3$ | " | N | 3 | Öl |
| 23 | $CH_3$ | $-O-SO_2-CH_3$ | " | N | 7 | Öl |
| 24 | $CH_3$ | $-O-SO_2-$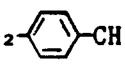$-CH_3$ | " | N | 7 | Öl |
| 25 | $CH_3$ | -O-CO-t-Bu | " | N | 3 | 163-165 |
| 26 | $CH_3$ | -O-CO-i-Pr | " | N | 7 | Öl |
| 27 | $CH_3$ | -O-CO-t-Bu- | " | N | 7 | Öl |
| 28 | $CH_3$ | $-O-CO-NHCH_3$ | " | N | 7 | Öl |
| 29 | $CH_3$ | 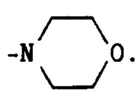 | " | N | 7 | 70-74 (HCl) |
| 30 | $CH_3$ | 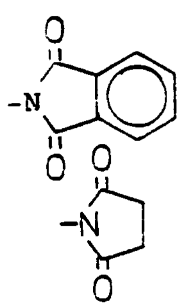 | " | N | 1 | 286-287 |
| 31 | $CH_3$ | | " | N | 3 | Öl |

| Nr. | $R_1$ | $R_2$* | $R_3$ | X | n | Fp.°C |
|---|---|---|---|---|---|---|
| 32 | $CH_3$ | 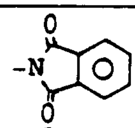 | 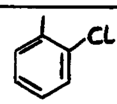 | N | 3 | Öl |
| 33 | $CH_3$ | 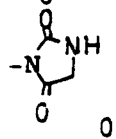 | " | N | 7 | Öl |
| 34 | $CH_3$ | 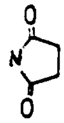 | " | N | 7 | Öl |
| 35 | $CH_3$ | -NH-CO-$CH_3$ | " | N | 1 | Zers. |
| 36 | $CH_3$ | -NH-CO-$CH_3$ | " | N | 3 | Öl |
| 37 | $CH_3$ | -NH-CO-i-Pr | " | N | 3 | 75-85 |
| 38 | $CH_3$ | -NH-CO-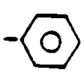 | " | N | 3 | 105-115 |
| 39 | $CH_3$ | -NH-CO-$CH_2$<br>\|<br>$N(CH_3)_2$ | " | N | 3 | Öl |
| 40 | $CH_3$ | -NH-$SO_2$-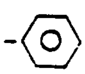-NH<br>\|<br>CO<br>\|<br>$CH_3$ | " | N | 3 | 148-155 |
| 41 | $CH_3$ | -NH-CO-i-Pr | " | N | 7 | Öl |
| 42 | $CH_3$ | -NH-CO-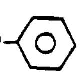 | " | N | 7 | 105/Zers. |
| 43 | $CH_3$ | -NH-CO-$CH_2$<br>\|<br>$N(CH_3)_2$ | " | N | 7 | Öl |

| Nr. | $R_1$ | $R_2^*$ | $R_3$ | X | n | Fp.°C |
|---|---|---|---|---|---|---|
| 44 | $CH_3$ | 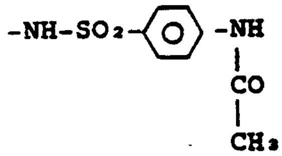 | 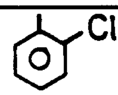 | N | 7 | 135/Z. |
| 45 | $CH_3$ | 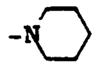 | " | N | 3 | Öl |
| 46 | $CH_3$ | 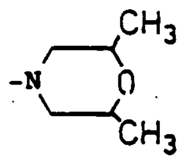 | " | N | 3 | 86-90 |
| 47 | $CH_3$ | " | " | N | 7 | Öl |
| 48 | $CH_3$ | 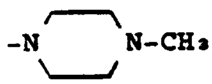 | " | N | 3 | Öl |
| 49 | $CH_3$ | 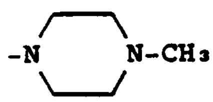 | " | N | 7 | Öl |
| 50 | H | $-O-CO-CH_3$ | " | N | 3 | 97-98 |
| 51 | $CH_3$ | $-O-CO-CH_3$ | 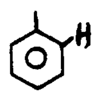 | N | 3 | 143-145 |
| 52 | $CH_3$ | $-OH$ | " | N | 3 | 160-163 |
| 53 | $CH_3$ | $-O-SO_2-CH_3$ | " | N | 3 | 76-79 |
| 54 | $CH_3$ | 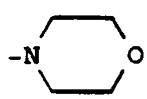 | " | N | 3 | Öl |
| 55 | $OCH_3$ | $-O-CO-CH_3$ | 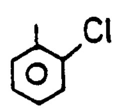 | N | 3 | |
| 56 | $CH_3$ | $-O-CO-CH_3$ | " | CH | 3 | Öl |

| Nr. | $R_1$ | $R_2$ * | $R_3$ | X | n | Fp.°C |
|---|---|---|---|---|---|---|
| 57 | $CH_3$ | $-OH$ | 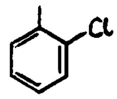 | CH | 7 | |
| 58 | $CH_3$ | $-O-CO-CH_3$ | ″ | CH | 7 | Öl |
| 59 | $CH_3$ | $-N(CH_3)_2$ | ″ | N | 7 | 92-95°C (HCl) |
| 60 | $CH_3$ | $-NH(CH_2)_2N(CH_3)_2$ | ″ | N | 7 | 55-60°C (HCl) |
| 61 | $CH_3$ | 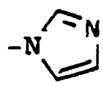 | ″ | N | 7 | 80-83°C (HCl) |
| 62 | $CH_3$ | 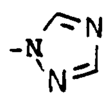 | ″ | N | 7 | 85-87°C (HCl) |
| 63 | $CH_3$ | 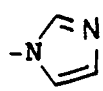 | ″ | N | 3 | 56-60°C |
| 64 | $CH_3$ | 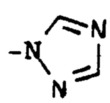 | ″ | N | 3 | 61-65°C |
| 65 | $CH_3$ | $-N(CH_3)_2$ | ″ | N | 3 | Öl |
| 66 | $CH_3$ | $-NH(CH_2)_2N(CH_3)_2$ | ″ | N | 3 | 60-65°C |

| Nr. | $R_1$ | $R_2$* | $R_3$ | X | n | Fp.°C |
|---|---|---|---|---|---|---|
| 67 | $CH_3$ | -NH-$SO_2$-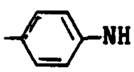-$NH_2$ | 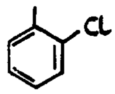 | N | 3 | |
| 68 | $CH_3$ | -NH-$SO_2$-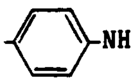-$NH_2$ | " | N | 7 | |
| 69 | $CH_3$ | -N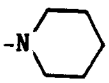 | " | N | 7 | Öl |
| 70 | $OCH_3$ | -OH | " | N | 3 | |
| 71 | $CH_3$ | $N(C_2H_5)_2$ | " | N | 3 | Öl |
| 72 | $CH_3$ | -N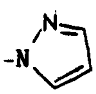 | " | N | 3 | Öl |
| 73 | $CH_3$ | -N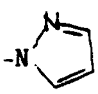 | " | N | 7 | Öl |
| 74 | $CH_3$ | -N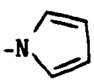 | " | N | 7 | Öl |
| 75 | $CH_3$ | -NH$(CH_2)_2$-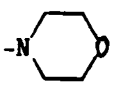 | " | N | 3 | 241 |
| 76 | $CH_3$ | -NH$CH_2$-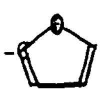 | " | N | 7 | 70-73 |
| 77 | $CH_3$ | -NH$(CH_2)_2$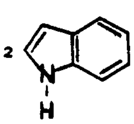 | " | N | 7 | amorph |
| 78 | $CH_3$ | 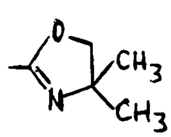 | " | N | 0 | 174-176 |

| Nr. | $R_1$ | $R_2^*$ | $R_3$ | X | n | Fp.°C |
|---|---|---|---|---|---|---|
| 79 | $CH_3$ | 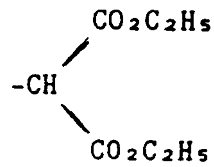 | 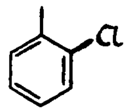 | N | 1 | 112-114 |
| 80 | $CH_3$ | 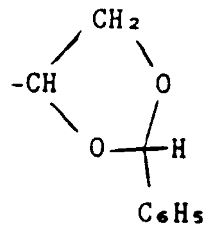 | " | N | 1 | |
| 81 | $CH_3$ | 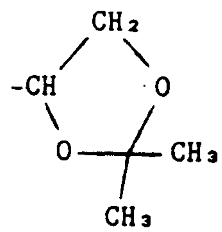 | " | N | 1 | Öl |
| 82 | $CH_3$ | 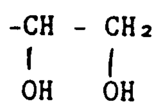 | " | N | 1 | Öl |
| 83 | $CH_3$ | 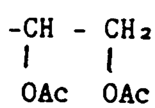 | " | N | 1 | |

| Nr. | $R_1$ | $R_2$* | $R_3$ | X | n | Fp.°C |
|---|---|---|---|---|---|---|
| 83a | $CH_3$ | $C_6H_5-CH_2-CH-$ 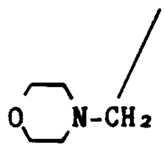 | 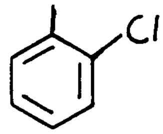 | N | 1 | 134-138° |
| 83b | $CH_3$ | $C_6H_5-CH_2-CH-$ / $CH_3-SO_2-O-CH_2$ | " | N | 1 | Öl |
| 83c | $CH_3$ | $C_6H_5-CH_2-CH-$ / $HO-CH_2$ | " | N | 1 | 148-150° |
| 83d | $CH_3$ | $-OC_6H_5$ | " | N | 3 | Öl |
| 83e | $CH_3$ | $-OCH_3$ | " | N | 3 | Öl |
| 83f | $CH_3$ | $-NH-(CH_2)_2$ 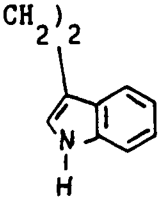 | " | N | 3 | Öl |
| 83g | $CH_3$ | -OAc | 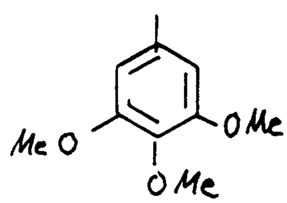 | N | 3 | Öl |

| Nr. | $R_1$ | $R_2$* | $R_3$ | X | n | Fp.°C |
|---|---|---|---|---|---|---|
| 84 | $CH_3$ | 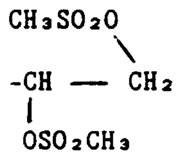 | 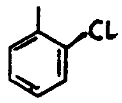 | N | 1 | |
| 85 | $CH_3$ | 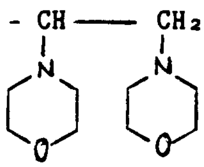 | " | N | 1 | |
| 86 | $CH_3$ | 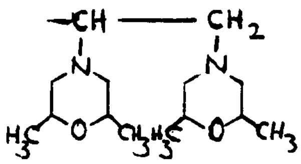 | " | N | 1 | |
| 87 | $CH_3$ | 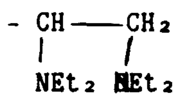 | " | N | 1 | |
| 88 | $CH_3$ | 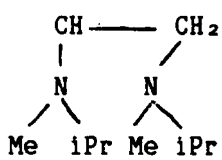 | " | N | 1 | |
| 89 | $CH_3$ | 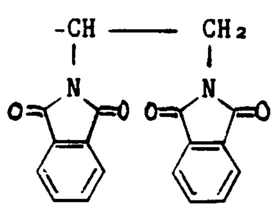 | " | N | 1 | |

| Nr. | $R_1$ | $R_2$* | $R_3$ | X | n | Fp.°C |
|---|---|---|---|---|---|---|
| 90 | $CH_3$ | 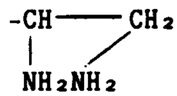 | 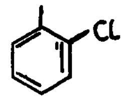 | N | 1 | |
| 91 | $CH_3$ | 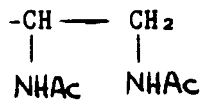 | " | N | 1 | |
| 92 | $CH_3$ | 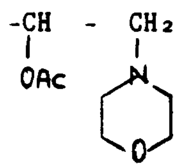 | " | N | 1 | |
| 93 | $CH_3$ | 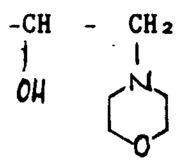 | " | N | 1 | |
| 94 | $CH_3$ | 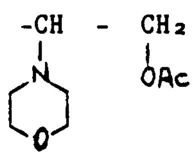 | " | N | 1 | |
| 95 | $CH_3$ | 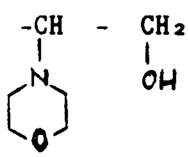 | " | N | 1 | |
| 96 | $CH_3$ | 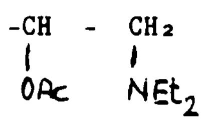 | " | N | 1 | |
| 97 | $CH_3$ | 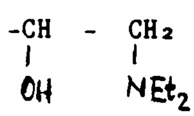 | " | N | 1 | |

Tabelle 8

Verbindungen der Formel Ib

| Nr | $R_1$ | $R_2$ | Zn | $R_3$ | R° | R' | X | Y | Fp |
|---|---|---|---|---|---|---|---|---|---|
| 98 | $CH_3$ | -OH | $-(CH_2)_3-$ | 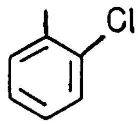 | H | H | N | N | Öl |
| 99 | $CH_3$ | -OAc | $-(CH_2)_3-$ | " | H | H | N | N | Öl |
| 100 | $CH_3$ | -OAc | $-(CH_2)_3-$ | " | -Ac | H | N | N | Öl |

Nachfolgend werden Beispiele für einige pharmazeutische Zusammensetzungen unter Verwendung von Verbindungen der allgemeinen Formel I als aktiver Bestandteil angegeben. Falls nicht ausdrücklich anders bezeichnet, handelt es sich bei den Teilen um Gewichtsteile.

1. Tabletten

| Die Tablette enthält folgende Bestandteile: | |
|---|---|
| Wirkstoff gemäß Formel Ia/b<br>Stearinsäure<br>Dextrose | 0,020 Teile<br>0,010 "<br>1,890 " |
| gesamt | 1,920 Teile |

Herstellung:

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel Ia/b<br>Neribas Salbe (Handelsware Scherax) | 50 mg<br>ad 10 g |

Herstellung:

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

Zusammensetzung:

| | | |
|---|---|---|
| Wirkstoff gemäß Formel Ia/b | | 50 mg |
| Neribas Salbe (Handelsware Scherax) | ad | 10 g |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

4. Ampullenlösung

Zusammensetzung:

| | | |
|---|---|---|
| Wirkstoff gemäß Formel Ia/b | | 1,0 mg |
| Natriumchlorid | | 45,0 mg |
| Aqua pro inj. | ad | 5,0 ml |

Herstellung:

Der Wirkstoff wird bei Eigen-pH, gegebenenfalls bei pH 5 - 7 in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 1 mg Wirkstoff.

5. Suppositorien

Jedes Zäpfchen enthält:

| | |
|---|---|
| Wirkstoff gemäß Formel Ia/b | 1,0 Teile |
| Kakaobutter (Fp. 36-37 °C) | 1200,0 Teile |
| Carnaubawachs | 5,0 Teile |

Herstellung

Kakaobutter und Carnaubawachs werden zusammengeschmolzen. Bei 45 °C gibt man den Wirkstoff hinzu und rührt, bis eine komplete Dispersion entstanden ist.
Die Mischung wird in Formen entsprechender Größe gegossen und die Zäpfchen zweckmäßig verpackt.

6. Inhalationslösungen

Zusammensetzung:

| | | |
|---|---|---|
| a) Wirkstoff gemäß Formel Ia/b | | 500 mg |
| Na-EDTA | | 50 mg |
| Benzalkoniumchlorid | | 25 mg |
| Natriumchlorid | | 880 mg |
| destilliertes Wasser | ad | 100 ml |

Herstellung:

96% der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) enthält 5 mg Wirkstoff.

b) Wirkstoff gemäß Formel Ia/b 500 mg
Natriumchlorid 820 mg
destilliertes Wasser ad 100 ml

Herstellung

96 % der Wasserstoffmenge werden vorgelegt, darin nacheinander der Wirkstoff und Natriumchlorid gelöst, mit dem restlichen Wasser aufgefüllt, und die Lösung in Eindosenbehälter (4 ml) abgefüllt. Die Lösung enthält 20 mg Wirkstoff.

In der Tabelle 9 sind die NMR-Sprekten ausgewählter Verbindungen der allgemeinen Formel Ia/Ib aufgeführt.

Tabelle 9

Beispiel I5

$^1$H-NMR ($CDCl_3$) δ ppm 7,23-7,62 (4H, m, Aryl-H); 6,36 (1H, s, Thiophen-H); 4,92 (2H, s, $CH_2$-7-Ring); 3,62 (2H, t, J = 6 Hz, $OCH_2$); 2,77 (2H, t,, J= 6 Hz, $CH_2$-Thiophen); 2,72 (3H, s, Triazol-$CH_3$), 2,07 (1H, s, breit, OH); 1,17-1,89 (10H, m, $OCH_2$-$(CH_2)_5$-).

Beispiel 19

$^1$H-NMR ($CDCl_3$) δ ppm 7,22-7,57 (4H, m, Aryl-H); 6,44 (1H, s, Thiophen-H); 4,95 (2H, s, 7-Ring-$CH_2$); 2,85 (2H, t, J= 6 Hz, Thiophen-$CH_2$); 2,80 (2H, t, J = 6 Hz, $NCH_2$); 2,71 (3H, s, Triazol-$CH_3$); 2,44 (2H, s, breit, $NH_2$); 1,84 (2H, m, $NCH_2$-$CH_2$-).

Beispiel 22

$^1$H-NMR ($CDCl_3$) δ ppm 7,22-7,87 (8H, m, Aryl-H); 6,40 (1H, s, Thiophen-H); 4,92 (2H, s, 7-Ring-$CH_2$); 4,06 (2H, t, J = 6 Hz, $OCH_2$); 2,87 (2H, t, J = 6 Hz, Thiophen-$CH_2$); 2,67 (3H, s, Triazol-$CH_3$); 2,44 (3H, s, Aryl-$CH_3$); 2,01 (2H, m, -$OCH_2CH_2$-).

Beispiel 23

$^1$H-NMR ($CDCl_3$) δ ppm 7,23-7,58 (4H, m, Aryl-H); 6,37 (1H, s, Thiophen-H); 4,93 (2H, s, 7-Ring-$CH_2$); 4,21 (2H, t, J = 7 Hz, $OCH_2$); 2,99 (3H, s, $CH_3SO_2$); 2,75 (2H, t, J = 7 Hz, Thiophen-$CH_2$); 2,70 (3H, s, Triazol-$CH_3$);1,14-1,98 (10H, m, $OCH_2$-$(CH_2)_5$).

Beispiel 24

$^1$H-NMR ($CDcl_3$) δ ppm 7,22-7,84 (8H, m, Aryl-H); 6,36 (1H, s, Thiophen-H); 4,93 (2H, s, 7-Ring-$CH_2$); 4,01 (2H, t, J = 6 Hz, $OCH_2$) 2,81 (2H, m, Thiophen-$CH_2$); 2,71 (3H, s, Triazol-$CH_3$); 2,49 (3H, s, Aryl-$CH_3$); 1,16-1,89 (10H, m, $OCH_2$-$(CH_2)_5$).

Beispiel 26

$^1$H-NMR ($CDCl_3$) δ ppm 7,30-7,59 (4H, m, Aryl-H); 6,37 (1H, s, Thiophen-H); 4,95 (2H, s, 7-Ring-$CH_2$); 4,06 (2H, t, J = 6 Hz, $OCH_2$-); 2,77 (2H, t, J = 6 Hz, Thiophen-$CH_2$); 2,72 (3H, s, Triazol-$CH_3$); 2,53 (1H, m, CH-C=O); 1,26;1,89 (10H, m, $OCH_2$-$(CH_2)_5$-); 1,28 (6H, d, J = 5 Hz, $(CH_3)_2$-CH-).

Beispiel 27

$^1$H-NMR ($CDCl_3$) δ ppm 7,31-7,58 (4H, m, Aryl-H); 6,37 (1H, s, Thiophen-H); 4,94 (2H, s, 7-Ring-$CH_2$); 4,04 (2H, t, J= 6 Hz, $OCH_2$); 2,71 (3H, s, Triazol-$CH_3$); 2,77 (2H, t, J = 7 Hz, Thiophen-$CH_2$); 1,23-1,93 (10H, m, $OCH_2(CH_2)_5$-); 1,20 (9H, s, $C(CH_3)_3$).

Beispiel 28

$^1$H-NMR ($CDCl_3$) δ ppm 7,31-7,59 (4H, m, Aryl-H); 6,37 (1H, s, Thiophen-H); 4,94 (2H, s, 7-Ring-$CH_2$); 4,69 (1H, s, breit, NH); 4,04 (2H, t, J = 7 Hz, $OCH_2$); 2,78 (3H, d, J = 6 Hz, $CH_3N$); 2,76 (2H, t, J = 7 Hz, Thiophen-$CH_2$); 2,71 (3H, s, Triazol-$CH_3$); 1,20-1,85 (10H, m, $OCH_2$-$(CH_2)_5$-).

Beispiel 31

$^1$H-NMR ($CDCl_3$) δ ppm 7,30-7,61 (4H, m, Aryl-H); 6,44 (1H, s, Thiophen-H); 4,94 (2H, s, 7-Ring-$CH_2$); 3,59 (2H, t, J = 7 Hz, N-$CH_2$); 2,81 (2H, t, J = 7 Hz, Thiophen-$CH_2$); 2,71 (7H, s, Succin-$CH_2CH_2$-,Triazol-$CH_3$); 1,93 (2H, m, N-$CH_2CH_2$).

Beispiel 32

$^1$H-NMR ($CDCl_3$) δ ppm 7,62-7,95 (4H, m, Phthalid-Aryl)-H); 7,28-7,56 (4H, m, Aryl-H); 6,43 (1H, s, Thiophen-H); 4,87 (2H, s, 7-Ring-$CH_2$), 3,76 (2H, t, J =6 Hz, N-$CH_2$); 2,86 (2H, t, J = 6 Hz, Thiophen-$CH_2$), 2,70 (3H, s, Triazol-$CH_3$); 2,06 (2H, m, N-$CH_2CH_2$-).

Beispiel 33

$^1$H-NMR ($CDCl_3$) δ ppm 7,28-7,58 (4H, m, Aryl-H); 6,35 (1H, s, breit, NH); 6,35 (1H, s, Thiophen-H); 4,93 (2H, s, 7-Ring-$CH_2$); 3,92 (2H, s, Imidazolidion-$CH_2$); 3,46 (2H, t, J = 7 Hz, N-$CH_2$); 2,75 (2H, t, J = 7 Hz, Thiophen-$CH_2$); 2,71 (3H, s, Triazol-$CH_3$); 1,13-1,86 (10H, m, N-$CH_2$-$(CH_2)_5$).

Beispiel 34

$^1$H-NMR ($CDCl_3$) δ ppm 7,30-7,51 (4H, m, Aryl-H); 6,44 (1H, s, Thiophen-H); 4,98 (2H, s, 7-Ring-$CH_2$); 3,51 (2H, t, J= 6 Hz, N-$CH_2$); 2,76 (2H, t, J = 6 Hz, Thiophen-$CH_2$); 2,74 (3H, s, Triazol-$CH_3$); 2,71 (4H, s, Succin-$CH_2$); 1,23-1,74 (10H, m, N-$CH_2$-$(CH_2)_5$-).

Beispiel 35

$^1$H-NMR ($CDCl_3$) δ ppm 7,21-7,52 (4H, m, Aryl-H); 7,27 (1H, t, J = 6 Hz, NH); 6,54 (1H, s, Thiophen-H); 4,84 (2H, s, 7-Ring-$CH_2$); 4,47 (2H, d, J - 6 Hz, N-$CH_2$); 2,66 (3H, s, Triazol-$CH_3$); 2,00 (3H, s, $CH_3$-C=O).

Beispiel 36

$^1$H-NMR ($CDCl_3$) δ ppm 7,33-7,57 (4H, m, Aryl-H); 6,54 (1H, t, J = 6 Hz, NH); 6,46 (1H, s, Thiophen-H); 4,96 (2H, s, 7-Ring-$CH_2$); 3,32 (2H, q, J = 6 Hz, NH-$CH_2$); 2,83 (2H, t, J = 6 Hz, Thiopen-$CH_2$); 2,71 (3H, s, Triazol-$CH_3$); 1,98 (3H, s, $CH_3$-CO); 1,88 (2H, m, N-$CH_2$-$CH_2$-).

Beispiel 39

$^1$H-NMR ($CDCl_3$) δ ppm 7,03-7,62 (5H, m, Aryl-H und NH); 6,40 (1H, s, Thiophen-H); 4,93 (2H, s, 7-Ring-$CH_2$); 3,33 (2H, q, J = 6,5 Hz, $CH_2$-NH); 2,94 (2H, s, $CH_2C$=O); 2,82 (2H, m, $CH_2$-Thiophen); 2,70 (3H, s, Triazol-$CH_3$); 2,27 (6H, s, N-$(CH_3)_2$); 1,89 (2H, m, N-$CH_2$-$CH_2$-).

Beispiel 41

$^1$H-NMR ($CDCl_3$) δ ppm 7,25-7,60 (4H, m, Aryl-H); 6,37 (1H, s, Thiophen-H); 5,64 (1H, s, breit, NH); 4,94 (2H, s, 7-Ring-$CH_2$); 3,23 (2H, m, N-$CH_2$); 2,76 (2H, t, J =6 Hz, Thiophen-$CH_2$); 2,71 (3H, s, Triazol-$CH_3$); 2,33 (1H, m, CH-C=O); 1,21-1,87 (10H, m, N-$CH_2$-$(CH_2)_5$); 1,16 (6H, d, J = 8 Hz, $(CH_3)_2$-C).

Beispiel 43

$^1$H-NMR ($CDCl_3$) δ ppm 7,45-7,60 (4H, m, Aryl-H); 7,17 (1H, s, breit, NH); 6,34 (1H, s, Thiophen-H); 4,93 (2H, s, 7-Ring $CH_2$); 3,27 (2H, q, J = 6,5 Hz, $CH_2NH$); 2,94 (2H, s, $CH_2C$=O); 2,71 (3H, s, Triazol-$CH_3$); 2,71 (2H, m, $CH_2$-Thiophen); 2,28 (6H, s, $N(CH_3)_2$); 1,21-1,88 (10H, m, $(CH_2)_5$).

Beispiel 45

$^{1}H$-NMR ($CDCl_3$) δ ppm 7,24-7,58 (4H, m, Aryl-H); 6,38 (1H, s, Thiophen-H); 4,93 (2H, s, 7-Ring-$CH_2$); 2,79 (2H, t, J = 8 Hz, N-$CH_2$); 2,69 (3H, s, Triazol-$CH_3$); 1,24-2,49 (14 H, m, Piperidin-$CH_2$, Thiophen-$CH_2$-$CH_2$).

Beispiel 47

$^{1}H$-NMR ($CDCl_3$) δ ppm 7,19-7,59 (4H, m, Aryl-H); 6,36 (1H, s, Thiophen-H); 4,93 (2H, s, 7-Ring-$CH_2$); 3,68 (2H, m, 2,6-Morpholin-CH-); 2,71 (3H, s, Triazol-$CH_3$); 1,16, 1,23 (6H, 2d, J = 6 Hz, 2,6-Morpholin-$CH_3$); 1,01-2,91 (18H, m, Morpholin-$NCH_2$/N-$(CH_2)_7$)

Beispiel 48

$^{1}H$-NMR ($CDCl_3$) δ ppm 7,24-7,56 (4H, m, Aryl-H); 6,38 (1H, s, Thiophen-H); 4,92 (2H, s, 7-Ring-$CH_2$); 2,80 (2H, t, J= 7 Hz, N-$CH_2$); 2,70 (3H, s, Triazol-$CH_3$); 2,42 (8H, s, Piperazin-$CH_2$); 2,37 (2H, t, J = 7 Hz, Thiophen-$CH_2$); 2,27 (3H, s, N-$CH_3$), 1,86 (2H, m, N-$CH_2$-$CH_2$).

Beispiel 49

$^{1}H$-NMR ($CDCl_3$)δ ppm 7,24-7.59 (4H, m, Aryl-H); 6,36 (1H, s, Thiophen-H); 4,93 (2H, s, 7-Ring-$CH_2$); 2,74 (2H, t, J-7 Hz), N-$CH_2$); 2,71 (3H, s, Triazol-$CH_3$); 2,44 (8H, s, Piperazin-$CH_2$); 2,28 (2H, t, J = 7 Hz, Thiophen-$CH_2$); 2,27 (3H, s, N-$CH_3$); 1,08-1,88 (10H, m, N-$CH_2$-$(CH_2)_5$).

Beispiel 54

$^{1}H$-NMR ($CDCl_3$) δ ppm 7,20-7,73 (5H, m, Aryl-H); 6,64 (1H, s, Thiophen-H); 4,87 (2H, s, 7-Ring-$CH_2$); 3,71 (4H, m, Morpholin-$OCH_2$); 2,89 (2H, t, J = 7 Hz, N-$CH_2$); 2,69 (3H, s, Triazol-$CH_3$); 2,21-2,57 (6H, m, Morpholin-N-$CH_2$/Thiophen-$CH_2$); 1,90 (2H, m, N-$CH_2$-$CH_2$-).

Beispiel 56

$^{1}H$-NMR ($CDCl_3$): δ 7,21 - 7,62 (m, 4H, Aryl-H); 6,92 (qu, J < 1 Hz, CH=); 6,41 (s, 1H, Thiophen-H); 4,81 (s, 2H, $CH_2$-7-Ring); 4,12 (t, J = 7 Hz, 2H, $OCH_2$); 2,86 (t, J = 7 Hz, 2H, $CH_2$-Thiophen); 2,45 (d, J < 1 Hz, 3H, $CH_3$-Imidazol); 2,05 (s, 3H, $CH_3$-C=O); 2,00 (m, 2H, $OCH_2CH_2$).

Beispiel 58

$^{1}H$-NMR ($CDCl_3$); δ 7,21 - 7,60 (m, 4H, Aryl-H); 6,91 (qu, J < 1 Hz, 1H, CH=); 6,35 (s, 1 H, Thiophen-H); 4,79 (s, 2H, $CH_2$-7-Ring); 4,06 (t, J = 7 Hz, 2H, $OCH_2$); 2,76 (t, J = 7 Hz, 2H, $CH_2$-Thiophen); 2,46 (d, J = < 1 Hz, 3H, $CH_3$-Imidazol); 2,05 (s, 3H, $CH_3C$=O); 1,15 - 1,91 (m, 1OH, $OCH_2$-$(CH_2)_5$-).

Beispiel 65

$^{1}H$-NMR ($CDCl_3$): δ 7,21 - 7,64 (m, 4H, Aryl-H); 6,43 (s, 1H, Thiophen-H); 4,97 (s, 2H, $CH_2$-7-Ring); 2,84 (t, J = 8 Hz, 2H, $CH_2$-Thiophen); 2,30 (t, J = 8 Hz, 2H, N-$CH_2$); 2,72 (s, 3H, $CH_3$-Triazol; 2,22 (s, 6H, N-$(CH_3)_2$), 1,81 (m, 2H, N-$CH_2CH_2$).

Beispiel 69

$^{1}H$-NMR ($CDCl_3$) δ ppm 7,22-7,58 (4H, m, Aryl-H)- 6,36 (1H, s, Thiophen-H); 4,93 (2H, s, 7-Ring-$CH_2$); 2,74 (2H, t, J = 7 Hz, N-$CH_2$); 2,71 (3H, s, Triazol-$CH_3$); 2,09-2,51 (6H, m, Piperidin-$CH_2$ Thiophen-$CH_2$); 1,12-1,83 (16H, m, Piperidin $CH_2$/N-$CH_2$-$(CH_2)_5$).

Beispiel 71

$^1$H-NMR ($CDCl_3$): δ 7,21 - 7,60 (m, 4H, Aryl-H), 6,39 (s, 1H, Thiophen H); 4,93 (s, 2H, $CH_2$-7-Ring); 2,79 (t, J = 8 HZ, 2H, $CH_2$-Thiophen); 2,69 (s, 3H, $CH_3$-Triazol); 2,52 (qu, J = 7 Hz, 4H, N-($CH_2CH_2$); 2,54 (t, J = 7 Hz, 2H, $CH_2$-Thiophen); 1,83 (m, 2H, N-$CH_2CH_2$); 0,98 (t, J = 8 Hz, 6H, N-$CH_2CH_3$).

Beispiel 72

$^1$H-NMR ($CDCl_3$): δ 7,22 - 7,63 (m, 6H,, Aryl-H, Pyrazol H 3/5); 6,38 (s, 1H, Thiophen-H); 6.22 (t, J =2 Hz, 1H, Pyrazol H 4); 4,94 (s, 2H, $CH_2$-7-Ring); 4,17 (t, J = 6 Hz, 2H, N-$CH_2$); 2,78 (t, J = 6 Hz, 2H, $CH_2$-Thiophen; 2,71 (s, 3H, $CH_3$-Triazol); 2,26 (m, 2H, $NCH_2CH_2$-).

Beispiel 73

$^1$H-NMR ($CDCl_3$); δ 7,22 - 7,66 (m, 6H, Aryl-H, Pyrazol H 3/5); 6,39 (s, 1H, Thiophen-H); 6,25 (t, J =2 Hz, 1H, Pyrazol-H4); 4,97 (s, 2H, $CH_2$-7-Ring); 4,15 (t, J = 7 Hz, 2H, N-$CH_2$); 2,78 (t, J = 7 Hz, 2H, $CH_2$-Thiophen); 2,72 (s, 3H, $CH_3$ Triazol); 1,08 - 2,05 (m, 1OH, N-$CH_2(CH_2)_5$-).

Beispiel 74/

$^1$H-NMR ($CDCl_3$): δ 7,29 - 7,62 (m, 4H, Aryl-H); 6,67 (t, J = 2 Hz, 2H, Pyrrol-H 2/5); 6,39 (s, 1H, Thiopheny-H); 6,16 (t, J = 2 Hz, 2H, Pyrrol-H/3/4); 4,98 (s, 2H, $CH_2$-7-Ring); 3,91 (t, J = 7 Hz, 2H, N-$CH_2$); 2,72 (t, J = 7 Hz, 2H, $CH_2$-Thiophen); 2,72 (s, 3H, $CH_3$-Triazol); 1,07 - 2,06 (m, 10 H, N-$CH_2(CH_2)_5$;

Beispiel 77

$^1$H-NMR ($CDCl_3$): δ 8,32 (s, 1H, NH-Indol); 7.00 -7,76 (m, 9H, Aryl-H, Indol-H); 6,35 (s, 1H, Thiophen-H); 4,93 (s, 2H, $CH_2$-7-Ring); 3,09 (s, 4H, $CH_2CH_2N$); 2,68 (s, 3H, $CH_3$ Triazol); 2,45 - 2,94 (m, 4H, Thiophen $CH_2CH_2N$-), 1,07 - 1,92 (m, 11H, N-$CH_2(CH_2)_5$-, NH).

Beispiel 81

1H-NMR ($CDCl_3$): δ 7.26 - 7.67 (4H, m, Aryl-H); 6.47 (lH, s, Thiophen-H); 4.96 (2H, s, $CH_2$-7-Ring); 4,35 (1H, m, X-part of ABX-System, OCH); 4.08, 3,63 (2H, m, AB-part of ABX-System, $OCH_2$); 3.02 (2H, d, J = 7 Hz, $CH_2$-Thiophen); 2.72 (3H, s, $CH_3$-Triazozol); 1.43, 1.37 (6H, 2 s, $C(CH_3)_2$).

Beispiel 82

1H-NMR ($CDCl_3$); δ 7.11 - 7.71 (4H, m. aryl-H; 6.46 (1H, s, Thiophen-H); 4.88 (2H, s, $CH_2$-7-Ring; 4.10 (2H, s. breit, 2 OH) 3.33 - 4.07 (3H, m, ABX-System, $OCH_2$-CH-O); 2.92 (2H, d, J = 5 Hz, $CH_2$-Thiophen); 2.65 (3H, s, $CH_3$-Triazol).

Beispiel 83b

$^1$H-NMR($CDCl_3$): δ 7,03 - 7,62 (9H, m, Aryl-H); 6,46 ($^1$H, s, (Thiophen-H); 5.00 4,87 (2H, AB-System, JAB = 15 Hz, $CH_2$-Ring):4,10 (2H, m, $OCH_2$); 2,93 (3H, s, $CH_3SO_2$); 2,70 (3H, s, $CH_3$ Triazol); 2,81 (4H, m, $CH_2$ Aryl, $CH_2$ Thiophen); 2,35 ($^1$H, m, -CH-).

Beispiel 83d

$^1$H-NMR ($CDCl_3$): δ 6,67 - 7,60 (9H, m, Aryl-H); 6,42 (1H, s, Thiophen-H); 4,91 (2H, s, $CH_2$-7-Ring); 3,98 (2H, t, J = 6 Hz, $OCH_2$); 2,98 (2H, t, J = 6 Hz, $CH_2$-Thiophen); 2,65 (3H, s, $Ch_3$-Triazol); 2,11 (2H, m, $=CH_2CH_2$-).

Beispiel 83e

$^1H$-NMR ($CDCl_3$): δ 7,27 - 7,58 (4H, m, Aryl-H); 6,41 (1H, s, Thiophen-H); 4,95 (2H, s, $CH_2$-7-Ring); 3,39 (2H, t, J = 6 Hz, $OCH_2$); 3,31 (3H, s, $OCH_3$); 2,87 (2H, t, J = 6 Hz, $CH_2$-Thiophen); 2,71 (3H, s, $CH_3$-Triazol); 1,90 (2H, m, $OCH_2CH_2$).

Biespiel 83f

$^1H$-NMR ($CDCl_3$): δ 8,31 (1 H, s, breit, NH); 6,95 - 7.70 (9H, m, Aryl-Indolyl-H); 6,32 (1H, s, Thiophen-H); 4,90 (2H, s, $CH_2$-7-Ring); 2,95 (4H, s, $NHCH_2CH_2$); 2,66 (3H, s, $CH_3$-Triazol); 2,52 - 2,92 (4H, m, $NHCH_2$, $CH_2$-Thiophen); 1,82 (2H, m, NH-$CH_2CH_2$).

Beispiel 83g

1H-NMR ($CDCl_3$): δ 6,25 (2H, s, 2-Aryl-H); 6,,78 (1H, s, Thiophen-H); 4,85 (2H, s, $CH_2$-7-Ring); 4,16 (2H, t, J =6 Hz, $OCH_2$); 3,86; 3,89 (9H , 2s, $OCH_3$); 2,95 (2H, t, J = 6 Hz, $CH_2$-Thiophen); 2,72 (3H, s, $CH_3$-Triazol);2,07 (3H, s, $CH_3$-C=O); 2,06 (2H, m, $OCH_2CH_2$-). In der Tabelle 10 sind die NMR-Spektren ausgewählter Zwischenverbindungen aufgeführt.

Beispiel 98

$^1H$-NMR ($CDCl_3$) δ ppm 7,15-7,59 (4H, m, Aryl-H); 6,14 (1H, s, Thiophen-H); 5,60 (s, 1H, CH-N), 4,14 (s, 2H, $CH_2$-7-Ring), 3,67 (t, J=7Hz, 2H, $OCH_2$), 2,80 (t, J=7Hz, 2H, $CH_2$-Thiophen), 2,70 (s, 3H, $CH_3$ Triazol), 1,58 - 2,24 (m, 4H, $OCH_2CH_2$, NH, OH).

Beispiel 99

$^1H$-NMR ($CDCl_3$) δ ppm 7,16 - 7,58 (m, 4H, Aryl-H), 6,14 (s, 1H, Thiophen-H, 5,61 (s, 1H, CH-N), 4,14 (s, 2H, $CH_2$-7-Ring), 4,08 (t, J=7Hz, $OCH_2$, 2,80 (t, J=7Hz, 2H, $CH_2$-Thiophen), 2,71 (s, 3H, $CH_3$-Triazol), 2,14 (s, breit, 1H, NH), 2,03 (s, 3H, $CH_3CO$), 1,96 (m, 2H, $OCH_2CH_2$).

Beispiel 100

$^1H$-NMR ($CDCl_3$) δ ppm 7,00 - 7,52 (m, 4H, Aryl-H), 7,07 (s, 1H, Thiophen-H), 6,56 (s, 1H, CH-N), 4,93/4,70 (AB-System, $J_{AB}$=15 Hz, 2H, $CH_2$-7-Ring), 4,12 (t, J=6Hz, 2H, $OCH_2$), 2,87 (t, J=6Hz, 2H, Thiophen-$CH_2$), 2,61 (s, 3H, $CH_3$-Triazol), 2,28 (s, 3H, $CH_3CO$ N), 2,07 (s, 3H, $CH_3CO$ O), 1,98 (m, 2H, $OCH_2CH_2$).

Tabelle 10

Verbindung Nr. 1

$^1H$-NMR ($CDCl_3$) δ ppm 7,29-7,54 (4H, m, Aryl-H); 7,10 (2H, s, breit, $NH_2$); 6,17 (IH, s, Thiophen-H); 3,75 (4H, m, Morpholin-$OCH_2$); 3,24 (4H, m, Morpholin-N-$CH_2$);3,06 (4H, s, $SO_2$-CH-$CH_2$).

Verbindung Nr. 3

$^1H$-NMR ($CDCl_3$) δ ppm 7,33-7,78 (5H, m, Aryl-H); 6,97 (2H, s, breit, $NH_2$); 6,56 (1H, s, Thiophen-H); 4,09 (2H, t, J = 6 Hz, $OCH_2$); 2,66 (2H, t, J = 7 Hz, Thiophen-$CH_2$); 2,04 (3H, s, $CH_3C$=O); 1,89 (2H, m, -0-$CH_2CH_2$-).

Verbindung Nr. 5

$^1H$-NMR ($CDCl_3$) δ ppm 7,24-7,49 (4H, m, Aryl-H); 7,07 (2H, s, breit, $NH_2$); 6,09 (1H, s, Thiophen-H); 4,05 (2H, t, J = 7 Hz, $OCH_2$); 2,49 (2H, t, J = 7 Hz, Thiophen-$CH_2$); 2,05 (3H, s, $CH_3C$=O); 1,08-1,83 (10H, m, $OCH_2$-$(CH_2)_5$).

Verbindung Nr. 5a

$^1$H-NMR ($CDCl_3$): δ 6,93 (s, 2H, Aryl-H); 6,64 (s, 1H, Thiophen-H); 3,91; 3,92 (2 s, 9H, 3 $OCH_3$); 3.71 (t, J = 7 Hz, 2H, $OCH_2$); 2.72 (t, J = 7 Hz, 2H, Thiophen-$CH_2$; 1,85 (m, 2H, $OCH_2CH_2$); NH und OH stark verbreitert!

Verbindung 5b

$^1$H-NMR ($CDCl_3$): δ 6,89 (s, 2H, Aryl-H); 6,59 (s, 1H, Thiophen-H); 4.09 (t, J = 7 Hz, 2H, $OCH_2$); 3,90; 3,89 (2s, 9H, 3-$OCH_3$); 2,66 (t, J = 7 Hz, 2H, Thiophen-$CH_2$); 2,03 (s, 3H, $CH_3C=O$); 1,88 (m, 2H, $OCH_2$-$CH_2$); $NH_2$ stark verbreitert!

Verbindung 5c

$^1$H-NMR ($CDCl_3$) δ ppm 7,19 - 7,52 (m, 4H, Aryl-H), 7,07 (s, breit, 2H, $NH_2$), 6,18 (s, 1H, Thiophen-H), 3,16 - 3,96 (m, 3H, $OCH_2$-CH), 2,67 (d, J=6Hz, $CH_2$-Thiophen), 2,51 (d, J=3Hz, 1H, CHOH), 2,19 (t, J=4Hz, 1H, $CH_2OH$).

Verbindung 5d

$^1$H-NMR ($CDCl_3$) δ ppm 7,09 - 7,67 (m, 9H, Aryl-H), 6,97 (s, breit, 2H, $NH_2$), 6,19 (t, J<1Hz, 1H, Thiophen-H), 5,93, 5,79 (2s, 1H, O-CH-O), 3,56 - 4,53 (m, 3H, $OCH_2CH$), 2,88 (dd, J=6Hz, <1Hz, 2H, Thiophen-$CH_2$).

Verbindung Nr. 6

$^1$H-NMR ($CDCl_3$) δ ppm 12,60 (1H, s, breit, NH); 7,24-7,61 (4H, m, Aryl-H); 6,53 (1H, s, Thiophen-H); 4,13 (2H, s, $CH_2Br$); 3,68 (4H, m, Morpholin-$OCH_2$); 3,23 (4H, m, Morpholin-N-$CH_2$); 3,17 (4H, s, $SO_2CH_2CH_2$-).

Verbindung Nr. 7

$^1$H-NMR ($CDCl_3$) δ ppm 12,70 (1H, s, breit, NH); 7,34-7,62 (5H, m, Aryl-H); 6,84 (1H, s, Thiophen-H); 4,11 (2H, s, $CH_2Br$); 4,11 (2H, t, J = 7 Hz, $OCH_2$); 2,82 (2H, t, J = 7 Hz, Thiophen-$CH_2$-); 2,04 (3H, s, $CH_3$-C=O); 1,98 (2H, m, -$OCH_2CH_2$-).

Verbindung Nr. 8

$^1$H-NMR ($CDCl_3$) δ ppm 12,66 (1H, s, breit, NH); 7,33-7,61 (4H, m, Aryl-H); 6,46 (1H, s, Thiophen-H); 4,14 (2H, s, $CH_2Br$); 4,08 (2H, t, J = 7 Hz, $OCH_2$); 2,78 (2H, t, J = 7 Hz, Thiophen-$CH_2$); 2,03 (3H, s, $CH_3C=O$); 1,93 (2H, m, ($OCH_2$-$CH_2$-).

Verbindung Nr. 9

$^1$H-NMR ($CDCl_3$) δ ppm 12,64 (1H, s, breit, NH); 7,31-7,44 (4H, m, Aryl-H); 6,41 (1H, s, Thiophen-H); 4,14 (2H, s, $CH_2Br$); 4,05 (2H, t, J = 6 Hz, $OCH_2$); 2,68 (2H, t, J = 6 Hz, Thiophen-$CH_2$); 2,04 (3H, s, $CH_3C=O$); 1,02-1,83 (10H, m, $OCH_2(CH_2)_5$-).

Verbindung Nr. 9 d

$^1$H-NMR ($CDCl_3$): δ 12,56 (1H, s, NH); 7,18 - 7,56 (4H, m, Aryl-H); 6,48 (1H, s, Thiophen H); 4,11 (2H, s, $CH_2Br$); 3,45-4,47 (3H, m, $OCH_2$-CHO); 2,90 (2H, d, J =6 Hz, $CH_2$-Thiophen); 1,40; 1,31 (6H, 2 s, 2$CH_3$).

Verbindung Nr. 11

$^1$H-NMR ($CDCl_3$) δ ppm 13,10 (1H, s, breit, NH-C=O); 7,30-7,86 (5H, m, Aryl-H); 6,80 (1H, s, Thiophen-H); 4,11 (2H, t, J = 7 Hz, $OCH_2$); 3,64 (2H, s, $CH_2C=O$); 2,81 (2H, t, J = 7 Hz, Thiophen-$CH_2$); 2,04 (3H, s, $CH_3C=O$); 2,00 (2H, m, $OCH_2CH_2$-); 1,73 (2H, s, breit, $NH_2$).

Verbindung Nr. 12

$^{1}$H-NMR ($CDCl_3$) δ ppm 12,24 (1H, s, breit, NH); 7,25-7,56 (4H, m, Aryl-H); 6,4l (1H, s, Thiophen-H); 4.07 (2H, t, J = 6 Hz, $OCH_2$); 3,67 (2H, s, $CH_2C=O$); 2,74 (2H, t, J = 6 Hz, Thiophen-$Ch_2$); 2,00 (3H, s, $CH_3C=O$); 1,92 (2H, m, $OCH_2CH_2$-); 1,78 (2H, s, breit, $NH_2$).

Verbindung Nr. 13

$^{1}$H-NMR ($CDCl_3$) δ ppm 13,60 (1H, s, breit, NH); 7,16-7,54 (4H, m, Aryl-H); 6,38 (1H, s, Thiophen-H), 4,04 (2H, t, J = 6 Hz, $OCH_2$); 3,66 (2H, s, $CH_2$-C=O); 2,65 (2H, t, J = 6 Hz, Thiophen-$CH_2$); 1,87 (2H, s, breit, $NH_2$); 2,04 (3H, s, $CH_3C=O$); 1,23-1,72 (10H, m, $OCH_2$-$(CH_2)_5$-).

Verbindung Nr. 14

$^{1}$H-NMR ($CDCl_3$) δ 9,29 (1H, s, breit, NH); 7,29-7,56 (4H, m, Aryl-H); 6,31 (1H, s, Thiophen-H); 4,48 (2H, s, 7-Ring-$CH_2$); 3,75 (4H, m Morpholin-$OCH_2$);, 3,23 (4H, m, Morpholin-N-$CH_2$); 3,14 (4H, s, -$SO_2$-$CH_2$-$CH_2$).

Verbindung Nr. 17

$^{1}$H-NMR ($CDCl_3$) δ ppm 10,03 (1H, s, breit, NH); 7,17-7,56 (4H, m, Aryl-H); 6,16 (1H, s, Thiophen-H); 4,47 (2H, s, 7-Ring-$CH_2$); 4,02 (2H, t, J= 6 Hz, $OCH_2$); 2,62 (2H, t, J = 6 Hz, Thiophen-$CH_2$); 2,02 (3H, s, $CH_3$-C=O); 1,03-1,82 (10H, m, $OCH_2$-$(CH_2)_5$).

Verbindung Nr. 17 c

$^{1}$H-NMR ($CDCl_3$): δ 9,33 ( 1H, s, breit, NH); 7,18 - 7,56 (4H, m, Aryl-H); 6,25 (1H, s, Thiophen-H); 4,46 (2H, s, $CH_2$-7-Ring); 3,45 - 4,37 (3H, m, $OCH_2$-CH-O); 2,88 (2H, d, J = 6 Hz), $CH_2$-Thiophen);1,40; 1,32 (6H, 2 s, $2CH_3$).

Verbindung Nr. 17 d

$^{1}$H-NMR ($CD_3OD/CDCl_3$ 1 : 1): δ 7,42 (4H, s, Aryl-H); 6,29 (1H, s, Thiophen-H); 4,35 (2H, s, $CH_2$-7-Ring); 3,53 - 3,87 (1H, m, CH-O); 3,45 (2H, d, J =5 Hz, $OCH_2$); 2,52 (2H, m, $CH_2$-Thiophen); NH, OH, is the solvent blind peak.

## Patentansprüche

**1.** Thieno-1,4-diazepine der allgemeinen Formel

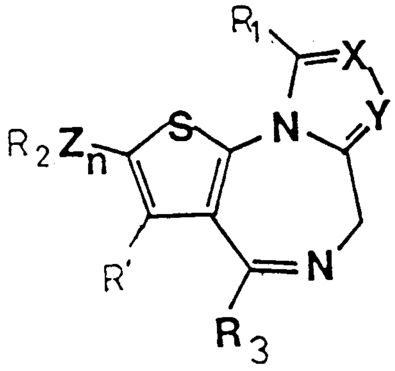
Ia

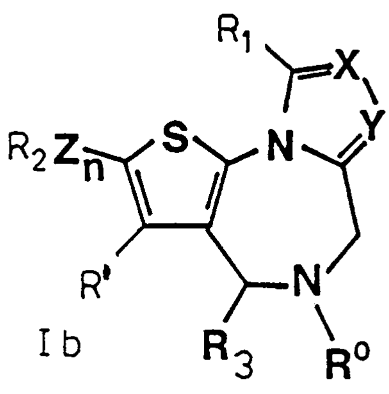
Ib

worin

$R_1$ Wassserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropylgruppe, eine verzweigte oder unverzweigte Alkoxygruppe mit 1 bis 4 Kohlenstoffatomen, Halogen,

bevorzugt Chlor oder Brom;

für n > O

$R_2$ Halogen, Hydroxy,

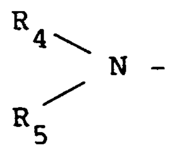

wobei $R_4$ und $R_5$, die gleich oder verschieden sein können, Wasserstoff, eine verzweigte oder unverzweigte Alkyl-, Alkenyl oder Alkinylgruppe mit 1 - 10 Kohlenstoffatomen, die gegebenenfalls durch Halogen, Hydroxy oder durch einen C-verknüpften 5-, 6- oder 7-gliedrigen Heterocyclus substituiert sein kann, wobei die Kohlenstoffkette durch Stickstoff, Sauerstoff oder Schwefel unterbrochen sein kann,

eine verzweigte oder unverzweigte Alkylcarbonylgruppe mit 1-6 Kohlenstoffatomen, gegebenenfalls substituiert durch Hydroxy oder Halogen oder substituiert durch eine gegebenenfalls ein- oder zweifach durch eine verzweigte oder unverzweigte Alkylgruppe mit 1 bis 6 Kohlenstoffatomen substituierte Aminogruppe, wobei die Alkylgruppe durch Halogen oder Hydroxy substituiert sein kann,

eine gegebenenfalls substituierte Arylcarbonylgruppe, eine gegebenenfalls substituierte Arylsulfonylgruppe, eine Alkylsulfonylgruppe mit 1 bis 4 Kohlenstoffatomen, oder

$R_4$ und $R_5$ bilden zusammen mit dem Stickstoffatom einen gesättigten oder ungesättigten gegebenenfalls ein-oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom gegebenenfalls durch eine verzweigte oder unverzweigte Alkylgruppe mit 1 bis 4 Kohlenstoffatomen substituiert sein kann;

$R_2$ eine Arylsulfonyloxygruppe, gegebenenfalls ein- oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylsulfonyloxygruppe mit 1 bis 4 Kohlenstoffatomen;

$R_2$ eine Arylcarbonyloxygruppe, gegebenenfalls ein oder mehrfach durch verzweigte oder unverzweigte Alkyl- und/oder Alkoxygruppen mit 1 bis 4 Kohlenstoffatomen substituiert;

$R_2$ eine verzweigte oder unverzweigte Alkylcarbonyloxygruppe mit 1 bis 12, Kohlenstoffatomen, wobei die Alkylkette durch Stickstoff, Sauerstoff oder Schwefel unterbrochen sein kann;

$R_2$

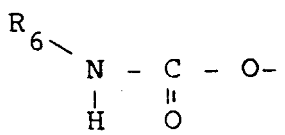

wobei $R_6$ eine verzweigte oder unverzweigte Alklyl-, Alkenyl- oder Alkinylgruppe mit 1 bis 4 Kohlenstoffatomen, gegebenenfalls durch Halogen substituiert, eine gegebenenfalls ein- oder mehrfach durch verzweigte oder unverzweigte Alkyl- und/oder Alkoxygruppen mit 1 bis 4 Kohlenstoffatomen substituierte Arylgruppe,

$R_2$

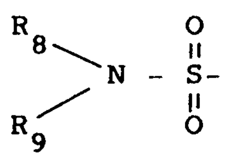

wobei $R_8$ und $R_9$, die gleich oder verschieden sein können, eine verzweigte oder unverzweigte Alkylkgruppe mit 1 bis 4 Kohlenstoffatomen,
oder $R_8$ und $R_9$ zusammen mit dem Stickstoffatom einen gegebenenfalls ein- oder mehrfach durch verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, substituiert ist;

$R_2$ eine verzweigte oder unverzweigteAlkoxygruppe mit 1 bis 4 Kohlenstoffatomen; eine Aryloxygruppe,;

$R_2$ einen Imidorest; Dioxolan, substituiertes Dioxolan;

für n größer gleich O

$R_2$ -CH = O; Cyano;

$R_2$ eine Aryloxycarbonylgruppe,

$R_2$ einen Rest der allgemeinen Formel

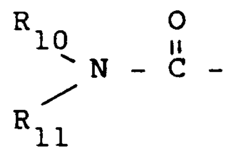

worin $R_{10}$ und $R_{11}$, die gleich oder verschieden sein können, Wasserstoff, Phenyl, substituiertes Phenyl, eine verzweigte oder unverzweigte Alkyl-, Alkenyl- oder Alkinylgruppe mit 1 bis 10 Kohlenstoffatomen, die gegebenenfalls durch Halogen, Hydroxy, Nitro, Amino, substituiertes Amino oder im Fall von $R_{10}$ = Wasserstoff oder Alkyl und Y = C-$R_1$ oder Y Stickstoff und X C-Alkyl, $R_{11}$ durch eine Esterfunktion oder ein Säureamid der allgemeinen Formel

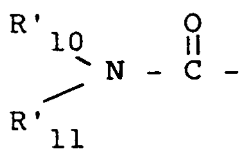

worin $R'_{10}$ und $R'_{11}$ dieselbe Bedeutung wie $R_{10}$ und $R_{11}$, jedoch mit Ausnahme eines Säureamids haben, substituiert sein kann,

$R_{10}$ oder $R_{11}$ ein gegebenenfalls ein- oder mehrfach durch verzweigtes oder unverzweigtes Alkyl mit 1 bis 4 Kohlenstoffatomen substituierter, gesättigter oder ungesättigter über ein Kohlenstoffatom gebundener 5-, 6-, oder 7-gliedriger heterocyclischer Ring,

$R_{10}$ und $R_{11}$ zusammen mit dem Stickstoffatom einen gesättigten oder ungesättigten gegebenenfalls ein-oder mehrfach durch verzweigte oder unverzweigte Alkylgruppen mit 1 bis 4 Kohlenstoffatomen substituierten 5-, 6- oder 7-ring, der als weitere Heteroatome Stickstoff, Sauerstoff oder Schwefel enthalten kann, wobei jedes weitere Stickstoffatom durch eine verzweigte oder unverzweigte Alkylgruppe mit 1 bis 4 Kohlenstoffatomen, bevorzugt Methyl, substituiert sein kann,

$R_2$ einen Rest der allgemeinen Formel

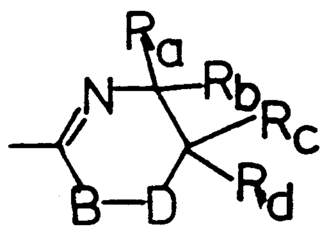

worin

B Sauerstoff, Schwefel NH oder $NC_1$-$C_6$-Alkyl D den Rest (C Re Rf)n, wobei n 0 bis 3 sein kann, Ra Wasserstoff, gegebenenfalls durch eine Hydroxy-oder Aminogruppe substituiertes Alkyl mit 1 bis 6 Kohlenstoffatomen, $C_1$ bis $C_4$ Alkoxycarbonyl, Dialkylaminocarbonyl,

Rb, Rc, Rd, Re, Rf Wasserstoff, gegenbenenfalls durch eine Hydroxy- oder Aminogruppe substituiertes Alkyl mit 1 bis 6 Kohlenstoffatomen, oder Phenyl;

$R_3$ Phenyl, wobei der Phenylring ein- oder mehrfach, bevorzugt in 2-Stellung, durch Methyl, Halogen, Nitro, Alkoxy, und/oder Trifluormethyl substituiert sein kann, Pyridyl;

R° Wasserstoff, eine Alkyl- oder eine Acylgruppe mit 1 bis 4 Kohlenstoffatomen,

R' Wasserstoff, Phenyl, substituiertes Phenyl oder eine verzweigte oder unverzweigte Alkylgruppe mit 1 bis 4 Kohlenstoffatomen,;

X,Y unabhängig voneinander C-$R_1$ oder N, aber nicht gleichzeitig beide C-$R_1$, oder Y die Gruppe C-COOR*, mit R* = Alkyl oder Wasserstoff und X = Stickstoff;

Z eine verzweigte oder unverzweigte Alkyl- oder Alkenylgruppe mit n-Kohlenstoffatomen, wobei Z gegenbenenfalls zusätzlich durch Aryl oder 2-fach durch $R_2$ substituiert sein kann, wobei $R_2$ gleich oder verschieden sein kann;

und

n eine der zahlen 0, 1, 2, 3, 4, 5, 6, 7, 8, 9 oder 10 bedeuten können, in Form ihrer Racemate, ihrer Enantiomeren, ihrer Diastereomere und ihrer Gemische; jeweils als freie Basen oder ihre physiologisch unbedenklichen Säureadditionssalze, wobei als Substituenten ein oder mehrere Atome aus der Gruppe Halogen, Methyl, Methoxy, Hydroxy und Trifluormethyl vorliegen können und Aryl ein aromatischer Rest mit bis zu 10 Kohlenstoffatomen bedeutet, der gegebenenfalls auch ein- oder mehrfach durch Halogen, Methyl, Methoxy, Hydroxy und Trifluormethyl substituiert sein kann,

mit der Maßgabe, daß im Fall einer Verbindung der allgemeinen Formel Ia, wenn X und Y beide Stickstoff, Z eine unverzweigte Alkylkette, n = 0 oder eine Zahl von 1 bis 8, $R_1$ Wasserstoff, eine verzweigte oder unverzweigte $C_1$-$C_4$-Alkylgruppe - gegebenenfalls substituiert durch Halogen oder Hydroxy-, eine Cyclopropylgruppe, eine $C_1$-$C_3$- Alkoxygruppe oder ein Halogenatom bedeuten und

$R_2$ ein Rest der allgemeinen Formel

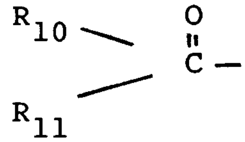

bedeutet, worin $R_{10}$ und $R_{11}$, die gleich oder verschieden sein können, Wasserstoff, eine verzweigte oder unverzweigte $C_1$-$C_4$-Alkyl-oder Hydroxyalkylgruppe oder $R_{10}$ und $R_{11}$ zusammen mit dem Stickstoffatom einen 5-, 6- oder 7-gliedrigen Ring bilden, der gegebenenfalls Stickstoff, Sauerstoff oder Schwefel als weitere Heteroatome enthalten kann, wobei das zweite Stickstoffatom gegebenenfalls durch eine $C_1$-$C_4$-Alkylgruppe substituiert sein kann,

$R_3$ **nicht** Phenyl oder eine Phenylgruppe, die ein oder mehrfach durch Methyl, Halogen, Nitro oder Trifluormethyl substituiert sein kann oder α-Pyridyl bedeuten kann,

wobei zusätzlich auch folgende Verbindungen ausgeschlossen sind:
2-(2-Chlorethyl)-4-(2-chlorphenyl)-9-methyl-6H-thieno-[3,2-f][1,2,4]triazolo[4,3-a][1,4]diazepin,
2-(Methylsulfonylmethyl)-4-(2-chlorphenyl)-9-methyl-6H-thieno[3,2-f][1,2,4]triazolo[4,3-a][1,4]diazepin,
2-Cyano-4-(2-chlorphenyl)-9-methyl-thieno[3,2-f][1,2,4]-triazolo[4,3-a][1,4]diazepin,
2-(1-Hydroxyethyl)-4-(2-chlorphenyl)-9-methyl-6H-thieno-[3,2-f][1,2,4]triazolo[4,3-a][1,4]diazepin.

**2.** Verbindungen der allgemeinen Formel Ia oder Ib nach Anspruch 1, worin

$R_1$ Methyl, Ethyl, Methoxy, Ethoxy, Chlor oder Brom;

$R_2$ Chlor, Brom, Jod, Hydroxy,

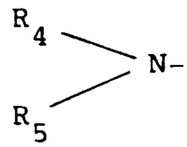

worin $R_4$ und $R_5$, die gleich oder verschieden sein können, Wasserstoff, eine verzweigte oder unverzweigte Alkylgruppe mit 1 bis 6, besonders bevorzugt 1 bis 4, Kohlenstoffatomen, wobei die Kohlenstoffkette durch Stickstoff unterbrochen sein kann, eine verzweigte oder unverzweigte Alkylcarbonylgruppe mit 1 bis 4 Kohlenstoffatomen, gegebenenfalls substituiert durch eine Dimethylaminogruppe, eine Phenylcarbonylgruppe,
im Fall von $R_5$ = Wasserstoff eine gegebenenfalls durch Acylamino, besonders bevorzugt Acetylamino, Amino, Alkylamino- oder Dialkylamino substituierte Phenylsulfonylgruppe, oder
$R_4$ und $R_5$ bilden zusammen mit dem Stickstoffatom eine Piperidin-, Pyrrolidin-, N'-Methylpiperazin-, einen gegebenenfalls dimethylsubstituierten Morpholinring, einen Pyrrol-, Pyrazol-, Imidazol- oder Triazolring;

$R_2$ -CH=O, COOH;
ein gegebenenfalls durch Methyl ein- oder mehrfach substituiertes $\Delta^2$-Imidazolin, -Oxazolin, -Thiazolin; eine Tolysulfonyloxygruppe; eine Methylsulfonyloxygruppe;
eine Phenylcarbonyloxygruppe;
eine verzweigte oder unverzweigte Alkylcarbonyloxygruppe mit 1 bis 5 Kohlenstoffatomen;
eine Methoxy- oder Ethoxycarbonylgruppe;

$R_2$

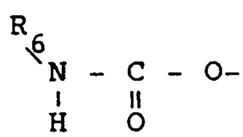 ,

wobei $R_6$ eine verzweigte oder unverzweigte Alkylgruppe mit 1 bis 4 Kohlenstoffatomen;

$R_2$

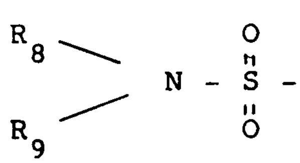

wobei $R_8$ und $R_9$, die gleich oder verschieden sein können, eine Methyl-, Ethyl, Propyl- oder Isopropylgruppe oder $R_8$ und $R_9$ zusammen mit dem Stickstoffatom einen N'-Methylpiperazin- oder Morpholinring;

$R_2$

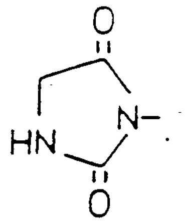

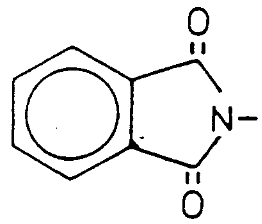

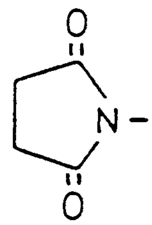

- $R_3$ Phenyl, wobei der Phenylring in 2-stellung durch Chlor substituiert sein kann;
- R° Wasserstoff, Methyl, Acetyl;
- R′ Wasserstoff;
- X,Y unabhängig voneinander C-$R_1$ oder N, aber nicht gleichzeitig beide C-$R_1$, ($R_1$ bevorzugt wasserstoff),
  oder Y die Gruppe C-COOR*, mit R* = Alkyl oder Wasserstoff und X = Stickstoff;
- Z wie zuvor definiert, bevorzugt $-(CH_2)_n-$,gegebenenfalls substituiert durch Phenyl oder 2-fach durch $R_2$, wobei $R_2$ auch verschieden sein kann oder $-CH_2-CHR_2-CH_2-R_2$, $CH_2-CHR_2R_2$ - $CH_2-CHR_2-CH_2-C_6H_5$;
  und
- n eine der Zahlen 1, 2, 3, 4, 5, 6, 7, 8, 9 oder 10 bedeuten können sowie gegebenenfalls ihre physiologisch unbedenklichen Säureadditionssalze.

**3.** Verbindungen der allgemeinen Formel Ia nach Anspruch 1 oder 2, worin $R_2$ wie zuvor definiert und $R_1$ Methyl oder Methoxy, $R_3$ o-Chlorphenyl, R′ Wasserstoff, X, Y beide Stickstoff oder X C-H und Y Stickstoff, $Z-(CH_2)_n-$, n die Zahlen 2, 3 oder 7 bedeuten.

**4.** Verfahren zur Herstellung von Verbindungen der allgemeinen Formel Ia nach Anspruch 1, 2 oder 3, worin $R_1$, $R_3$, R′, Z, X, Y und n wie zuvor definiert, $R_2$ - COOR*, mit R* = Alkyl, oder $R_2$ Hydroxy, Alkylcarbonyloxy, Alkyoxycarbonyl, Aryloxycarbonyl, Dioxolan, substituiertes Dioxolan oder $R_8R_9NSO_2$-bedeuten kann,
dadurch gekennzeichnet, daß man eine entsprechende Verbindung der allgemeinen Formel

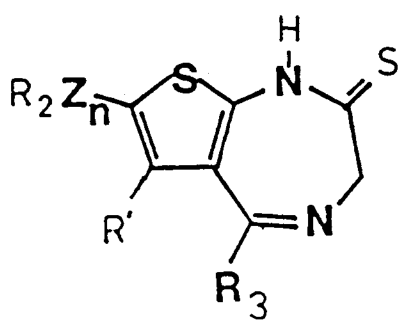

II

A) für den Fall, daß X und Y Stickstoff bedeutet
a) mit einem Säurehydrazid der allgemeinen Formel

$R_1-CONHNH_2$

unsetzt,

b) oder aber mit Hydrazin in eine Verbindung der allgemeinen Formel

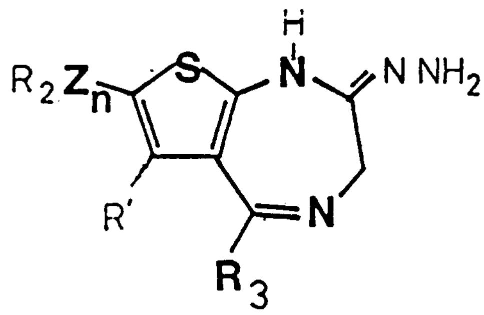

überführt und anschließend mit einen Säurehalogenid der allgemeinen Formel

$R_1$-CO-Hal

oder mit einem Orthoester der allgemeinen Formel

$R_1$ - C $(OR')_3$

worin R′ eine niedere Alkylgruppe bedeutet, umsetzt, oder

B) für den Fall daß X C-H und Y Stickstoff

a) mit einem Aminoalkin der allgemeinen Formel

$R_{11}$ - C≡C - $CH_2$-$NH_2$

worin $R_{11}$ Wasserstoff oder eine niedere Alkylgruppe bedeutet

oder aber

b) mit einem α-Aminoaldehyd-alkylacetal oder α-Aminoketon-alkylketal der allgemeinen Formel

$H_2NCH_2$-$CH_1(OR')_2$

worin $R_1$ eine niedere Alkylgruppe oder eine Cyclopropylgruppe bedeutet, umsetzt;

C) für den Fall, daß X Stickstoff und Y C-H man eine Verbindung der allgemeinen Formel

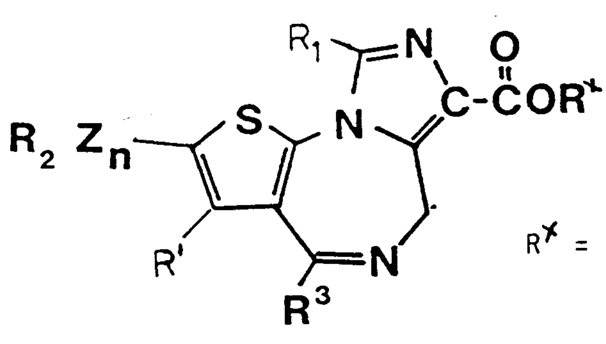

$R^x$ = niederes Alkyl

decarboxyliert.

und gegebenenfalls in ihre unbedenklichen Säureadditionssalze überführt.

**5.** Verfahren zur Herstellung von Verbindungen der Formel

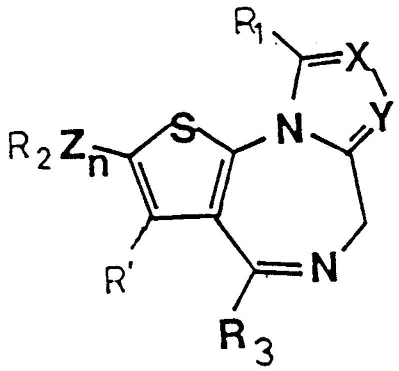

Ia

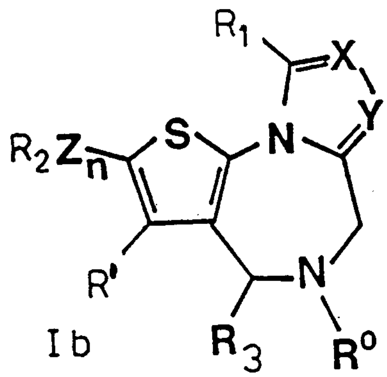

Ib

gemäß einem der Ansprüche 1, 2 oder 3
dadurch gekennzeichnet, daß man als Ausgangsverbindung eine Verbindung der allgemeinen Formel I

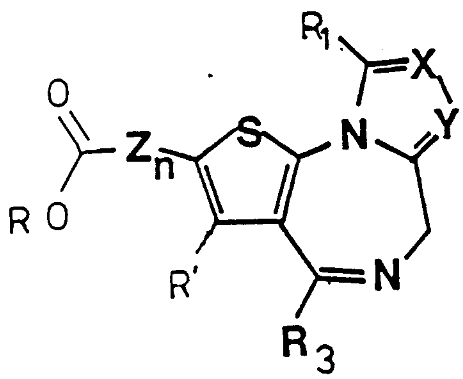

worin $R_1$ = Wasserstoff, Alkyl, Cycloalkyl;
Z, X, Y, R′, n und $R_3$ wie zuvor definiert und R eine niedere Alkylgruppe ist,
wie folgt unsetzt;

(1) Verbindungen der allgemeinen Formel Ia mit

$R_2$ = COOH

erhält man durch Verseifen von Verbindungen der Formel I;

(2) Verbindungen der allgemeinen Formel Ia mit

$R_2 = R_{10}R_{11}$ NCO-

erhält man durch Umsetzung von Ia mit

$R_2$ = COOH

mit einem Amin der Formel

$HNR_{10}R_{11}$

beispielsweise in Gegenwart von Sulfonyldiimidazol oder Carbonyldiimidazol;

(3) Verbindungen der allgemeinen Formel Ia mit

$R_2$ = OH

erhält man durch selektive Reduktion von I, z.B. mit Lithiumalanat oder Natriumborhydrid;
oder durch Hydrolyse einer Verbindung der allgemeinen Formel Ia, worin $R_2$ einen Alkylcarbonyloxyrest bedeutet;
(4) Verbindungen der allgemeinen Formel Ia mit

$R_2 = R_6$NH-COO- ,

erhält man durch Reaktion des Alkohols, z.B. hergestellt nach 3), mit einem Isocyanat der allgemeinen Formel

$R_6\text{-}N = C = O$;

(5) Verbindungen der allgemeinen Formel Ia mit
$R_2$ = Alkyl-bzw. Arylcarbonyloxy
erhält man durch Reaktion des Alkohols, z.B. hergestellt nach 3), mit einem Säureäqivalent einer Carbonsäure der Formel

R-C00H,

worin R ein Arylrest oder ein verzweigter oder ein unverzweigter Alkylrest mit 1 bis 8 Kohlenstoffatomen, wobei die Kohlenstoffkette durch Stickstoff, Sauerstoff oder Schwefel unterbrochen sein kann; bedeutet;
(6) Verbindungen der allgemeinen Formel Ia mit
$R_2$ = -Alkylsulfonyloxy oder Arylsulfonyloxy erhält man
durch Reaktion des Alkohols z.B. hergestellt nach 3), mit einem Alkyl- oder Arylsulfonylhalogenid unter Zusatz eines säurebindenden Mittels;
(7) Verbindungen der allgemeinen Formel Ia mit

$R_2 = NR_4R_5$

oder einem Imidorest
erhält man durch Reaktion des Mesylats, z.B. hergestellt nach 6), mit einem Amin der Formel

$HNR_4 R_5$

oder einem Imid;
(8) Verbindungen der allgemeinen Formel Ia mit

$R_2 = NH_2$

erhält man beispielsweise durch Spaltung des Phthalimids oder aus den entsprechenden Isocyanaten, $R_2$ = N = C = O durch Hydrolyse;
(9) Verbindungen der allgemeinen Formel Ia mit

$R_2 = NR_4R_5$

mit $R_4$ oder $R_5$ Alkyl- oder Arylcarbonyl
erhält man durch Reaktion eines primären oder sekundären Amins, z.B. hergestellt nach 7) bzw. 8), mit einem Carbonsäurequivalent abgeleitet von einer Carbonsäure der Formel

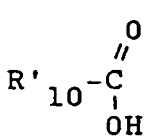

(10) Verbindungen der allgemeinen Formel Ia mit

$R_2$ = Formyl

erhält man durch Oxidation des Alkohols, z.B, hergestellt nach 3), unter Verkürzung der Kette von n auf n-1;

(11) Verbindungen der allgemeinen Formel Ia worin

$R_2$ ein Rest der allgemeinen Formel

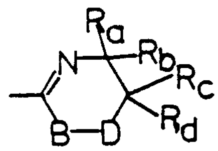

bedeutet,

erhält man durch Reaktion einer Verbindung der allgemeinen Formel Ia mit

$R_2$ = C00H

mit einem Amin der allgemeinen Formel

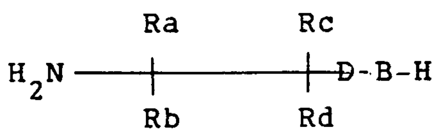

worin $R_a$, $R_b$, $R_c$, $R_d$, D und B die zuvor genannte Bedeutung aufweist,

in Gegenwart von Triphenylphospin, $CCl_4$ und einer Base;

(11a) Verbindungen der allgemeinen Formel Ia, worin $R_2$ ein gegebenenfalls substituierter Thiazolinrest ist, erhält man aus den entsprechenden Oxazolinen, z.b. hergestellt nach 11) durch Schwefelung, z.B. mit Phosphorpentasulfid oder Lawesson-Reagenz[R].

(12) Verbindungen der allgemeinen Formel Ia mit

worin $R_2$ = CN

erhält man durch Reaktion von Verbindungen der allgemeinen Formel Ia worin

$R_2 = CONH_2$

bedeutet, mit Phosphoroxychlorid;

(13) Verbindungen der allgemeinen Formel Ia

worin $R_2$ = ein gegebenenfalls durch verzweigte oder unverzweigte Alkylgruppen substituiertes 2-Imidazolin ist,

erhält man durch Reaktion von Verbindungen der allgemeinen Formel Ia mit

$R_2$ = CN

a) mit ehtanolischer HCl,

b) Umsetzen des entstandenen Imidoethylesters mit einen gegebenfalls durch verzweigte oder unverzweigte Alkylgruppen substituiertem Ethylendiamin,

c) gegebenfalls erfolgt die Alkylierung der freien N-H Funktion mit bekannten Alkylierungsmitteln;

(14) Verbindungen der allgemeinen Formel Ia mit $R_2$ einem Aryloxy- oder Alkyloxyrest erhält man beispielsweise durch Reaktion des Mesylats, z.B. hergestellt nach 6), mit den entsprechenden Alkoholaten;

(15) Verbindungen der allgemeinen Formel Ia, worin $R_2$ = Alkyl- oder Aryloxycarbonyl bedeutet, erhält man beispielsweise durch Umsetzung von einem Säurequivalent von I, $R_2$ COOH, mit den entsprechenden Alkoholen;

(16) Verbindungen der allgemeinen Formel Ia mit mit $R_2$ = Halogen, erhält man durch Reaktion des Tosylats, z.B. hergestellt nach 6), mit einem Halogenierungsmittel;

anschließend gewünschtenfalls die erhaltenen Verbindungen Ia mit $R_1$ = Wasserstoff in Gegenwart einer Base mit einem Halogenierungsmittel, wie z.B. mit Chlor oder Brom, zu einer Verbindung der allgemeinen Formel Ia mit $R_1$ Halogen, wie z.B. Chlor oder Brom umsetzt;

anschließend gewünschtenfalls die Halogenverbindung in eine Verbindung der allgemeinen Formel Ia mit $R_1$ = Alkoxy mit 1 bis 4 Kohlenstoffatomen durch Reaktion mit dem entsprechenden Alkoholat überführt;

anschließend gewünschtenfalls eine Verbindung der allgemeinen Formel Ia selektiv zu einer Verbindung der allgemeinen Formel Ib mit $R^o$ = Wasserstoff reduziert und anschließend gewünschtenfalls alkyliert oder acyliert;

und gegebenfalls die so erhaltenen Verbindungen Ia oder Ib in ihre unbendenklichen Säureadditionssalze überführt.

**6.** Verfahren zur Herstellung von Verbindungen der allgemeinen Formel Ia nach Anspruch 4, worin $R_1$ Halogen bzw. Alkoxy ist, dadurch gekennzeichnet, daß man eine Verbindung der allgemeinen Formel Ia mit $R_1$ Wasserstoff

a) mit einem Halogenierungsmittel, wie z.B. Chlor oder Brom halogeniert

und anschließend gewünschtenfalls

b) die Halogenverbindung mit dem entsprechendem Alkoholat umsetzt

und gegebenenfalls in ihr unbedenkliches Säureadditionssalz überfürt.

**7.** Verfahren zur Herstellung von Verbindungen der allgemeinen Formel Ib nach Anspruch 1, 2 oder 3 dadurch gekennzeichnet, daß man eine Verbindung der allgemeinen Formel Ia selektiv reduziert und gewünschtenfalls die erhaltene Verbindung Ib worin $R^o$ Wasserstoff bedeutet, alkyliert oder acyliert und gegebenenfalls in ihre pharmakologisch unbedenklichen Säureadditionssalze überführt.

**8.** Pharmazeutische Präparate, enthaltend als Wirkstoffe Verbindungen der allgemeinen Formel Ia oder Ib in Kombination mit üblichen Hilfs- und/oder Trägerstoffen.

**9.** Verfahren zur Herstellung von pharmazeutischen Präparaten nach Anspruch 8, dadurch gekennzeichnet, daß man Verbindungen der allgemeinen Formel Ia oder Ib mit üblichen galenischen Hilfs- und/oder Trägerstoffen zu üblichen pharmazeutischen Anwendungsformen verarbeitet.

**10.** Verbindungen der allgemeinen Formel Ia oder Ib gemäß Anspruch 1, 2 oder 3 zur Verwendung für die Herstellung von Arzneimitteln mit PAF-antagonistischer Wirkung.

**11.** Verfahren zur Herstellung von Arzneimitteln, enthaltend Verbindungen der allgemeinen Formel Ia oder Ib gemäß einem der Ansprüche 1, 2 oder 3, zur Behandlung von Krankenheit an denen PAF beteiligt ist.

**12.** Als neue Zwischenverbindungen der allgemeinen Formel

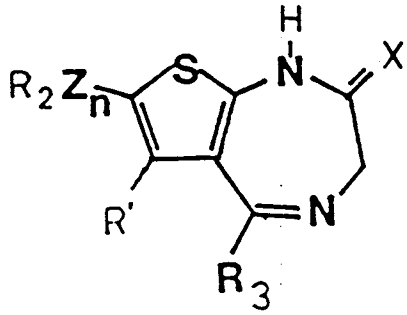

X= O, S

worin

R' Wasserstoff, Phenyl, substituiertes Phenyl oder eine verzweigte oder unverzweigte Alkylgruppe mit 1 bis 4 Kohlenstoffatomen,

$R_3$ Phenyl, wobei der Phenylring ein- oder mehrfach, bevorzugt in 2-Stellung, durch Methyl, bevorzugt Halogen, besonders bevorzugt Chlor oder Brom, Nitro, Alkoxy, bevorzugt Methoxy und/oder Trifluormethyl substituiert sein kann, Pyridyl;

Z eine verzweigte oder unverzweigte Alkyl- oder Alkylengruppe mit n-Kohlenstoffatomen, wobei Z gegebenenfalls zusätzlich durch Aryl oder 2-fach durch $R_2$ substituiert sein kann, wobei $R_2$ gleich oder verschieden sein kann;

n eine der Zahlen 0, 1, 2, 3, 4, 5, 6, 7, 8, 9 oder 10

$R_2$ Hydroxy, Alkylcarbonyloxy, Dioxolan, substituiertes Dioxolan oder

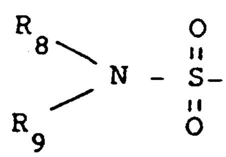

wobei $R_8$ und $R_9$, die gleich oder verschieden sein können, eine verzweigte oder unverzweigte Alkylgruppe mit 1 bis 4 Kohlenstoffatomen,

oder $R_8$ und $R_9$ zusammen mit dem Stickstoffatom einen gegebenenfalls ein- oder mehrfach durch eine verzweigte oder unverzweigte Alkylgruppen mit 1 bis 4 Kohlenstoffatomen substituierten 5-, 6- oder 7-Ring, der als weitere Heteroatome Stickstoff, Sauerstoff oder Schwefel enthalten kann, wobei jedes weitere Stickstoffatom durch eine Alkylgruppe mit 1 bis 4 Kohlenstoffatomen, bevorzugt Methyl, substituiert ist;

oder

wenn R'≠ Wasserstoff

$R_2$ auch Alkoxycarbonyl bedeuten können.

## Claims

1. Thieno-1,4-diazepines of general formula

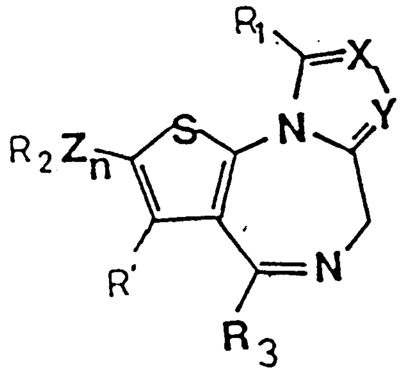

Ia

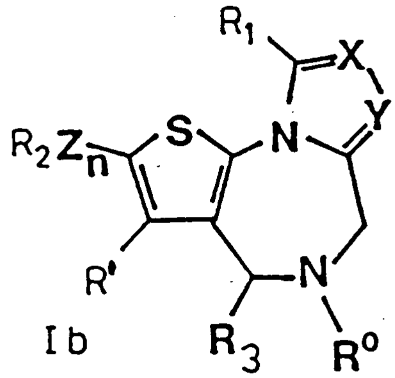

Ib

wherein

$R_1$ represents hydrogen, a branched or unbranched alkyl group with 1 to 4 carbon atoms which may optionally be substituted by hydroxy or halogen, a cyclopropyl group, a branched or unbranched alkoxy group with 1 to 4 carbon atoms, or a halogen, preferably chlorine or bromine;

when $n > 0$

$R_2$ represents halogen, hydroxy,

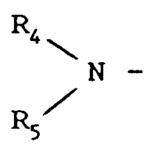

wherein $R_4$ and $R_5$, which may be identical or different, represent hydrogen, a branched or unbranched alkyl, alkenyl or alkynyl group with 1 to 10 carbon atoms which may optionally be substituted by halogen, hydroxy or a C-linked 5-, 6- or 7-membered heterocyclic group, whilst the carbon chain may be interrupted by nitrogen, oxygen or sulphur,

a branched or unbranched alkylcarbonyl group with 1 to 6 carbon atoms, optionally substituted by hydroxy or halogen, or substituted by an amino group which is optionally mono- or disubstituted by a branched or unbranched alkyl group with 1 to 6 carbon atoms, whilst the alkyl group may be substituted by halogen or hydroxy,

an optionally substituted arylcarbonyl group, an optionally substituted arylsulphonyl group, an alkylsulphonyl group with 1 to 4 carbon atoms, or

$R_4$ and $R_5$ together with the nitrogen atom form a saturated or unsaturated 5-, 6- or 7-membered ring optionally mono- or polysubstituted by branched or unbranched alkyl groups with 1 to 4 carbon atoms, this ring possibly containing nitrogen, oxygen or sulphur as further heteroatoms, whilst each additional nitrogen atom may optionally be substituted by a branched or unbranched alkyl group with 1 to 4 carbon atoms;

$R_2$ represents an arylsulphonyloxy group, optionally mono- or polysubstituted by branched or unbranched alkyl and/or alkoxy groups with 1 to 4 carbon atoms;

$R_2$ represents a branched or unbranched alkylsulphonyloxy group with 1 to 4 carbon atoms;

$R_2$ an arylcarbonyloxy group, optionally mono- or polysubstituted by branched or unbranched alkyl and/or alkoxy groups with 1 to 4 carbon atoms;

$R_2$ represents a branched or unbranched alkylcarbonyloxy group with 1 to 12 carbon atoms, whilst the alkyl chain may be interrupted by nitrogen, oxygen or sulphur;

$R_2$ represents

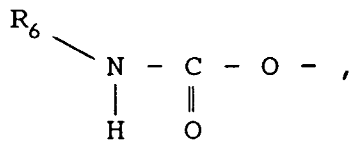

wherein $R_6$ represents a branched or unbranched alkyl, alkenyl or alkynyl group with 1 to 4 carbon atoms, optionally substituted by halogen, an aryl group optionally mono- or polysubstituted by branched or unbranched alkyl and/or alkoxy groups with 1 to 4 carbon atoms,

$R_2$ represents

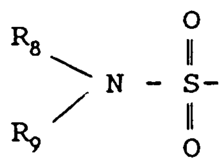

wherein $R_8$ and $R_9$, which may be identical or different, represent a branched or unbranched alkyl group with 1 to 4 carbon atoms,

or $R_8$ and $R_9$ together with the nitrogen atom represent a 5-, 6- or 7-membered ring optionally mono- or polysubstituted by branched or unbranched alkyl groups with 1 to 4 carbon atoms, this group optionally containing as further heteroatoms nitrogen, oxygen or sulphur, whilst each additional nitrogen atom is substituted by an alkyl group with 1 to 4 carbon atoms;

$R_2$ represents a branched or unbranched alkoxy group with 1 to 4 carbon atoms; an aryloxy group;

$R_2$ represents an imido group; dioxolan, substituted dioxolan;

when n is greater than or equal to 0

$R_2$ represents -CH=O; cyano;

$R_2$ represents an aryloxycarbonyl group,

$R_2$ represents a group of general formula

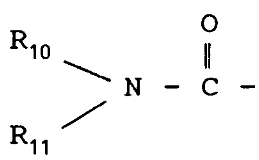

wherein $R_{10}$ and $R_{11}$, which may be identical or different, represent hydrogen, phenyl, substituted phenyl, a branched or unbranched alkyl, alkenyl or alkynyl group with 1 to 10 carbon atoms, which may optionally be substituted by halogen, hydroxy, nitro, amino, substituted amino or, if $R_{10}$ = hydrogen or alkyl and Y = C-$R_1$ or Y represents nitrogen and X represents C-alkyl, $R_{11}$ may be substituted by an ester function or an acid amide of general formula

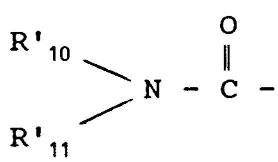

wherein $R'_{10}$ and $R'_{11}$ have the same meanings as $R_{10}$ and $R_{11}$, with the exception of an acid amide,

$R_{10}$ or $R_{11}$ represent a saturated or unsaturated 5-, 6- or 7-membered heterocyclic ring linked by a carbon atom, optionally mono- or polysubstituted by branched or unbranched alkyl with 1 to 4 carbon atoms,

$R_{10}$ and $R_{11}$ together with the nitrogen atom represent a saturated or unsaturated 5-, 6- or 7-membered ring optionally mono- or polysubstituted by branched or unbranched alkyl groups with 1 to 4 carbon atoms and optionally containing, as further heteroatoms, nitrogen, oxygen or sulphur, whilst each additional nitrogen atom may be substituted by a branched or unbranched alkyl group with 1 to 4 carbon atoms, preferably methyl,

$R_2$ represents a group of general formula

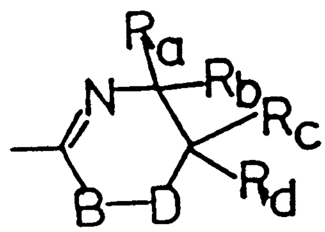

wherein

B represents oxygen, sulphur, NH or $NC_1$-$C_6$-alkyl

D represents the group (C Re Rf)n, wherein n may be from 0 to 3,

Ra represents hydrogen, alkyl with 1 to 6 carbon atoms optionally substituted by a hydroxy or amino group, $C_1$ to $C_4$ alkoxycarbonyl, dialkylaminocarbonyl,

Rb, Rc, Rd, Re, Rf represent hydrogen, alkyl with 1 to 6 carbon atoms optionally substituted by a hydroxy or amino group, or phenyl;

$R_3$ represents phenyl, wherein the phenyl ring, preferably in the 2 position, may be mono- or polysubstituted by methyl, halogen, nitro, alkoxy and/or trifluoromethyl, or $R_3$ may represent pyridyl;

$R°$ represents hydrogen, alkyl or an acyl group with 1 to 4 carbon atoms,

R' represents hydrogen, phenyl, substituted phenyl or a branched or unbranched alkyl group with 1 to 4 carbon atoms,

X,Y independently of each other represent C-$R_1$ or N but cannot both simultaneously represent C-$R_1$, or Y represents the group C-COOR*, wherein R* = alkyl or hydrogen and X = nitrogen;

Z represents a branched or unbranched alkyl or alkenyl group with n carbon atoms, wherein Z may optionally additionally be substituted by aryl or disubstituted by $R_2$, whilst $R_2$ may be identical or different;

and

n represents one of the numbers 0, 1, 2, 3, 4, 5, 6, 7, 8, 9 or 10,

in the form of their racemates, enantiomers, diastereomers and mixtures thereof, as free bases or as the physiologically acceptable acid addition salts thereof, whilst as substituents one or more atoms selected from halogen, methyl, methoxy, hydroxy and trifluoromethyl may be present, and aryl represents an aromatic group having up to 10 carbon atoms, which may optionally also be mono- or polysubstituted by halogen, methyl, methoxy, hydroxy and trifluoromethyl, with the proviso that in the case of a compound of general formula Ia, if X and Y both represent nitrogen, Z represents an unbranched alkyl chain, n = 0 or a number from 1 to 8, $R_1$ represents hydrogen, a branched or unbranched $C_{1-4}$ alkyl group (optionally substituted by halogen or hydroxy), a cyclopropyl group, a $C_{1-3}$ alkoxy group or a halogen atom and

$R_2$ represents a group of general formula

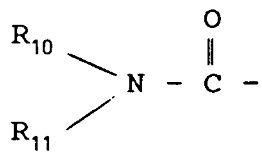

wherein $R_{10}$ and $R_{11}$, which may be identical or different, represent hydrogen, a branched or unbranched $C_{1-4}$ alkyl or hydroxyalkyl group or $R_{10}$ and $R_{11}$ together with the nitrogen atom form a 5-, 6- or 7-membered ring which may optionally contain nitrogen, oxygen or sulphur as additional heteroatoms, whilst the second nitrogen atom may optionally be substituted by a $C_{1-4}$ alkyl group,

$R_3$ does not represent phenyl or a phenyl group, which may be mono- or polysubstituted by methyl, halogen, nitro or trifluoromethyl or may represent α-pyridyl,

whilst additionally the following compounds are excluded:
2-(2-chloroethyl)-4-(2-chlorophenyl)-9-methyl-6H-thieno[3,2-f][1,2,4]triazole[4,3-a][1,4]-diazepine,
2-(methylsulphonylmethyl)-4-(2-chlorophenyl)-9-methyl-6H-thieno[3,2-d][1,2,4]triazolo[4,3-a]-[1,4]-diazepine,
2-cyano-4-(2-chlorophenyl)-9-methyl-thieno[3,2-f]-[1,2,4]triazolo[4,3-a][1,4]diazepine,
2-(1-hydroxyethyl)-4-(2-chlorophenyl)-9-methyl-6H-thieno[3,2-f][1,2,4]triazolo[4,3-a][1,4]-diazepine.

**2.** Compounds of general formula Ia or Ib as claimed in claim 1, wherein

$R_1$ represents methyl, ethyl, methoxy, ethoxy, chlorine or bromine;

$R_2$ represents chlorine, bromine, iodine or hydroxy,

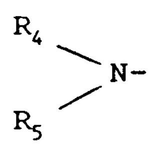

wherein $R_4$ and $R_5$, which may be identical or different, represent hydrogen, a branched or unbranched alkyl group with 1 to 6, more particularly 1 to 4, carbon atoms, whilst the carbon chain may be interrupted by nitrogen, a branched or unbranched alkylcarbonyl group with 1 to 4 carbon atoms, optionally substituted by a dimethylamino group, a phenylcarbonyl group, when $R_5$ = hydrogen, $R_2$ may also represent a phenylsulphonyl group optionally substituted by acylamino, particularly acetylamino, amino, alkylamino or dialkylamino,

or

$R_4$ and $R_5$ together with the nitrogen atom form a piperidine, pyrrolidine, N'-methylpiperazine, an optionally dimethyl-substituted morpholine ring, a pyrrole, pyrazole, imidazole or triazole ring;

$R_2$ represents -CH=O; -COOH;

a $\Delta^2$-imidazoline, -oxazoline, -thiazoline optionally mono- or polysubstituted by methyl; a tolylsulphonyloxy group; a methylsulphonyloxy group; a phenylcarbonyloxy group; a branched or unbranched alkylcarbonyloxy group with 1 to 5 carbon atoms; a methoxy- or ethoxycarbonyl group;

$R_2$ represents

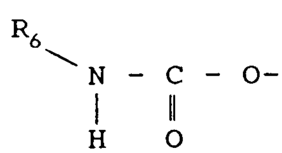
,

wherein $R_6$ represents a branched or unbranched alkyl group with 1 to 4 carbon atoms;

$R_2$ represents

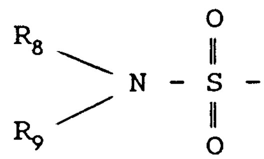

wherein $R_8$ and $R_9$, which may be identical or different, represent a methyl, ethyl, propyl or isopropyl group or $R_8$ and $R_9$ together with the nitrogen atom represent an N'-methyl-piperazine or morpholine ring;

$R_2$ represents

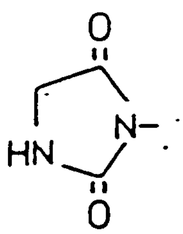

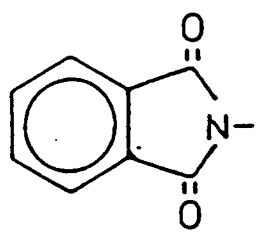

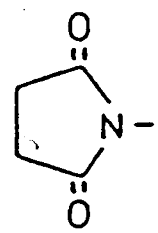

$R_3$ represents phenyl, whilst the phenyl ring may be substituted by chlorine in the 2 position;

R° represents hydrogen, methyl or acetyl;

R' represents hydrogen;

X,Y independently of each other represent C-$R_1$ or N but cannot both simultaneously represent C-$R_1$, ($R_1$ preferably represents hydrogen),
or Y represents the group C-COOR*, wherein R* = alkyl or hydrogen and X = nitrogen;

Z is defined as hereinbefore and preferably represents $-(CH_2)_n-$, optionally substituted by phenyl or disubstituted by $R_2$, whilst $R_2$ may also be different, or $-CH_2-CHR_2-CH_2-R_2$, $-CH_2-CHR_2R_2$, $-CH_2CHR_2-CH_2-C_6H_5$;
and

n represents one of the numbers 1, 2, 3, 4, 5, 6, 7, 8, 9 or 10,
and optionally the physiologically acceptable acid addition salts thereof.

**3.** Compounds of general formula Ia as claimed in claim 1 or 2, wherein $R_2$ is as hereinbefore defined and $R_1$ represents methyl or methoxy, $R_3$ represents o-chlorophenyl, R' represents hydrogen, X and Y both represent nitrogen or X represents C-H and Y represents nitrogen, Z represents $-(CH_2)_n-$, and n represents the number 2, 3 or 7.

4. Process for preparing compounds of general formula Ia as claimed in claim 1, 2 or 3, wherein $R_1$, $R_3$, R', Z, X, Y and n are as hereinbefore defined, $R_2$ represents -COOR*, where R* = alkyl, or $R_2$ represents hydroxy, alkylcarbonyl, alkoxycarbonyl, aryloxycarbonyl, dioxolan, substituted dioxolan or $R_8R_9NSO_2$, characterised in that a corresponding compound of general formula

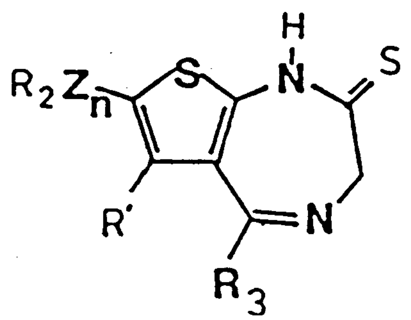

II

A) if X and Y represent nitrogen

a) is reacted with an acid hydrazide of general formula

$R_1$-CONHNH$_2$,

b) or is converted with hydrazine into a compound of general formula

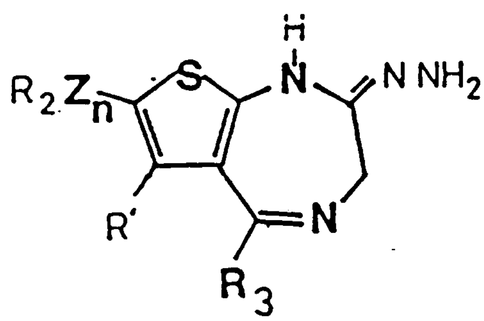

and is then reacted with an acid halide of general formula

$R_1$-CO-Hal

or with an orthoester of general formula

$R_1$ - C (OR')$_3$

wherein R' represents a lower alkyl group, or

B) if X represents C-H and Y represents nitrogen

a) is reacted with an aminoalkyne of general formula

$R_{11}$ - C = C - $CH_2$-$NH_2$

wherein $R_{11}$ represents hydrogen or a lower alkyl group, or

b) is reacted with an $\alpha$-aminoaldehyde-alkylacetal or $\alpha$-aminoketone-alkylketal of general formula

$H_2NCH_2$-$CR_1(OR')_2$

wherein $R_1$ represents a lower alkyl group or cyclopropyl group, or

C) if X represents nitrogen and Y represents C-H, a compound of general formula

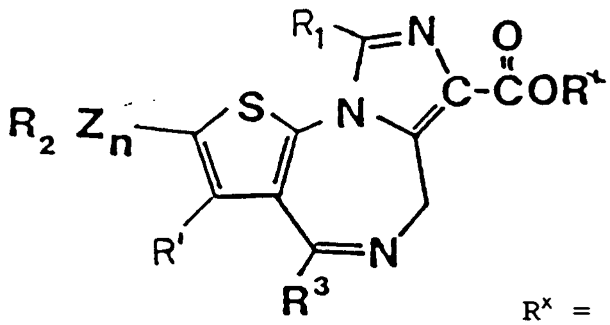

$R^x$ = lower alkyl

is decarboxylated, and optionally converted into the harmless acid addition salts thereof.

**5.** Process for preparing compounds of general formula

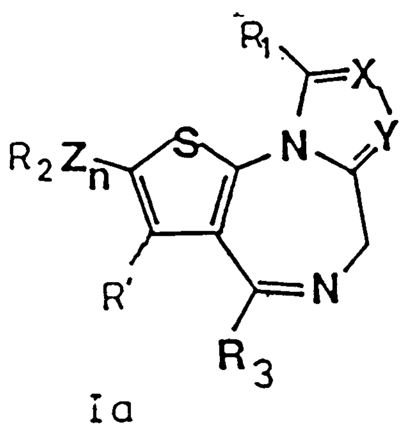

Ia

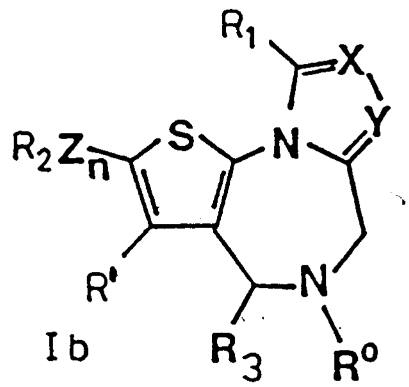

Ib

according to one of claims 1, 2 or 3, characterised in that, as starting compound, a compound of general formula I

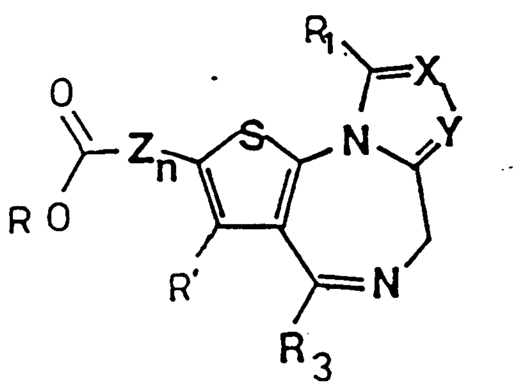

wherein $R_1$ = hydrogen, alkyl, cycloalkyl; Z, X, Y, R', n and $R_3$ are defined as hereinbefore and R represents a lower alkyl group, is reacted as follows:

(1) compounds of general formula Ia wherein

$R_2$ = COOH

are obtained by saponification of compounds of formula I;

(2) compounds of general formula Ia wherein

$R_2 = R_{10}R_{11}$ NCO-

are obtained by reacting Ia wherein

$R_2 = COOH$

with an amine of formula

$HNR_{10}R_{11}$

for example in the presence of sulphonyldiimidazole or carbonyldiimidazole;
(3) compounds of general formula Ia wherein

$R_2 = OH$

are obtained by selective reduction of I, e.g. with lithium alanate or sodium borohydride; or by hydrolysis of a compound of general formula Ia wherein $R_2$ represents an alkylcarbonyloxy group;
(4) compounds of general formula Ia wherein

$R_2 = R_6$NH-COO-

are obtained by reacting the alcohol, e.g. prepared according to 3), with an isocyanate of general formula

$R_6N=C=O$;

(5) compounds of general formula Ia wherein
$R_2$ = alkyl or arylcarbonyloxy
are obtained by reacting the alcohol, e.g. prepared according to 3), with an acid equivalent of a carboxylic acid of formula

R-COOH,

wherein R represents an aryl group or a branched or unbranched alkyl with 1 to 8 carbon atoms, whilst the carbon chain may be interrupted by nitrogen, oxygen or sulphur;
(6) compounds of general formula Ia wherein
$R_2$ = alkylsulphonyloxy or arylsulphonyloxy are obtained by reacting the alcohol prepared, for example, according to 3), with an alkyl- or arylsulphonyl halide, with the addition of an acid binding agent;
(7) compounds of general formula Ia wherein

$R_2 = NR_4R_5$

or an imido group,
are obtained by reacting the mesylate, e.g. prepared according to (6), with an amine of formula

$HNR_4R_5$

or an imide;
(8) compounds of general formula Ia wherein

$R_2 = NH_2$

are obtained, for example, by cleaving the phthalimide, or from the corresponding isocyanates, wherein $R_2$ is $N=C=O$, by hydrolysis;

(9) compounds of general formula Ia wherein

$R_2 = NR_4R_5$

where $R_4$ or $R_5$ represents alkyl- or arylcarbonyl are obtained by reacting a primary or secondary amine, e.g. prepared according to 7) or 8), with a carboxylic acid equivalent derived from a carboxylic acid of formula

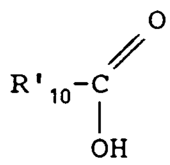

(10) compounds of general formula Ia wherein

$R_2$ = formyl

are obtained by oxidation of the alcohol, e.g. prepared according to 3), with shortening of the chain from n to n-1;
(11) compounds of general formula Ia wherein
$R_2$ represents a group of general formula

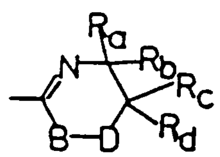

are obtained by reacting a compound of general formula Ia wherein

$R_2$ = COOH

with an amine of general formula

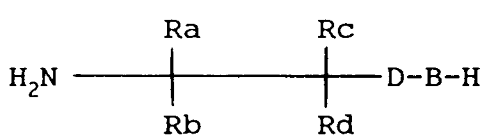

wherein $R_a$, $R_b$, $R_c$, $R_d$, D and B have the meanings given hereinbefore, in the presence of triphenylphosphine, $CCl_4$ and a base;

(11a) Compounds of general formula Ia wherein $R_2$ is an optionally substituted thiazoline group are obtained from the corresponding oxazolines, e.g. prepared according to (11) by sulphurisation, e.g. with phosphorus pentasulphide or Lawesson's reagent®;

(12) compounds of general formula Ia wherein

$R_2$ = CN

are obtained by reacting compounds of general formula Ia wherein

$R_2 = CONH_2$

with phosphorus oxychloride;

(13) compounds of general formula Ia
wherein $R_2$ is a 2-imidazoline optionally substituted by branched or unbranched alkyl groups,
are obtained by reacting compounds of general formula Ia wherein

$R_2$ = CN

a) with ethanolic HCl,
b) reacting the resulting imidoethyl ester with an ethylenediamine optionally substituted by branched or unbranched alkyl groups,
c) and optionally the free N-H function is alkylated with known alkylating agents;

(14) compounds of general formula Ia wherein $R_2$ is an aryloxy or alkyloxy group are obtained, for example, by reacting the mesylate, prepared according to 6), for example, with the corresponding alkoxides;
(15) compounds of general formula Ia,
wherein $R_2$ = alkyl- or aryloxycarbonyl, are obtained by reacting an acid equivalent of I ($R_2$ = COOH) with the corresponding alcohols;
(16) compounds of general formula Ia
wherein $R_2$ = halogen,
are obtained by reacting the tosylate, e.g. prepared according to 6), with a halogenating agent;

and subsequently, if desired, the resulting compounds Ia wherein $R_1$ = hydrogen are reacted in the presence of a base with a halogenating agent such as chlorine or bromine to form a compound of general formula Ia wherein $R_1$ = halogen, e.g. chlorine or bromine;
and subsequently, if desired, the halogen compound is converted into a compound of general formula Ia wherein $R_1$ = alkoxy with 1 to 4 carbon atoms by reacting with the corresponding alkoxide;
and subsequently, if desired, a compound of general formula Ia is selectively reduced to give a compound of general formula Ib wherein R° = hydrogen, and
subsequently, if desired, it is alkylated or acylated;
and optionally the resulting compounds Ia or Ib are converted into the harmless acid addition salts thereof.

**6.** Process for preparing compounds of general formula Ia as claimed in claim 4, wherein $R_1$ is halogen or alkoxy, characterised in that a compound of general formula Ia wherein $R_1$ is hydrogen
a) is halogenated with a halogenating agent such as chlorine or bromine
and subsequently, if desired,
b) the halogen compound is reacted with the corresponding alkoxide
and optionally converted into a harmless acid addition salt thereof.

**7.** Process for preparing compounds of general formula Ib as claimed in claim 1, 2 or 3, characterised in that a compound of general formula Ia is selectively reduced and, if desired, the resulting compound Ib wherein R° represents hydrogen is alkylated or acylated and optionally converted into a pharmacologically acceptable acid addition salt thereof.

**8.** Pharmaceutical preparations containing as active substances compounds of general formula Ia or Ib in conjunction with conventional excipients and/or carriers.

**9.** Process for preparing pharmaceutical preparations as claimed in claim 8, characterised in that compounds of general formula Ia or Ib are processed with conventional galenic excipients and/or carriers to form conventional pharmaceutical forms for administration.

**10.** Compounds of general formula Ia or Ib as claimed in claim 1, 2 or 3 for use in the manufacture of pharmaceuticals with a PAF-antagonistic activity.

**11.** Process for producing pharmaceutical compositions, containing compounds of general formula Ia or Ib as claimed in one of claims 1, 2 or 3, for the treatment of diseases in which PAF is implicated.

**12.** As new intermediates, compounds of general formula

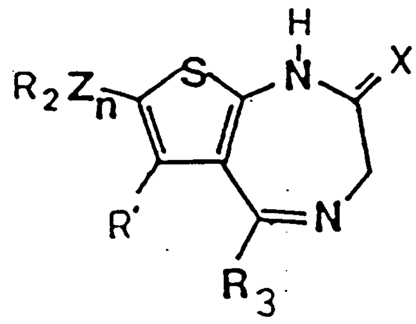

X= O, S

wherein

- R' represents hydrogen, phenyl, substituted phenyl or a branched or unbranched $C_{1-4}$ alkyl group,
- $R_3$ represents phenyl, whilst the phenyl ring may be mono- or polysubstituted, preferably in the 2 position, by methyl, preferably halogen, more especially chlorine or bromine, nitro, alkoxy, preferably methoxy and/or trifluoromethyl, or pyridyl;
- Z represents a branched or unbranched alkyl or alkenyl group having n carbon atoms, whilst Z may optionally also be substituted by aryl or disubstituted by $R_2$, and the $R_2$ substituents may be identical or different;
- n represents one of the numbers 0, 1, 2, 3, 4, 5, 6, 7, 8, 9 or 10;
- $R_2$ represents hydroxy, alkylcarbonyloxy, dioxolan, substituted dioxolan or

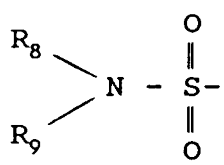

wherein $R_8$ and $R_9$, which may be identical or different, represent a straight-chained or branched $C_{1-4}$ alkyl group,
or $R_8$ and $R_9$ together with the nitrogen atom represent a 5-, 6- or 7-membered ring, optionally mono- or polysubstituted by a branched or unbranched $C_{1-4}$ alkyl, and optionally containing nitrogen, oxygen or sulphur as further heteroatoms, each additional nitrogen atom being substituted by a $C_{1-4}$ alkyl group, preferably methyl;
or
if R' does not represent hydrogen
$R_2$ may also represent alkoxycarbonyl.

## Revendications

1. Thiéno-1,4-diazépines de formule générale

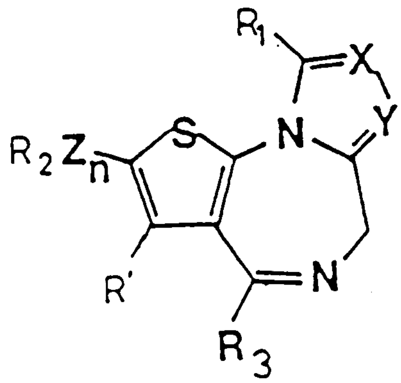

Ia

Ib

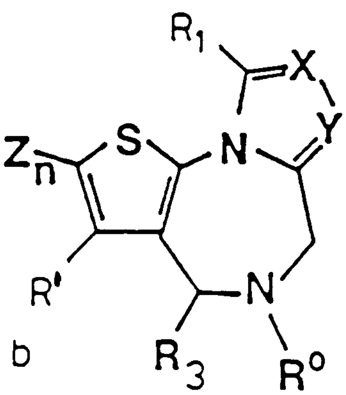

dans laquelle
$R_1$ représente un atome d'hydrogène, un groupe alkyle ramifié ou non ramifié contenant 1 à 4 atomes de carbone, oui peut être éventuellement substitué par hydroxy ou halogène, un groupe cyclopropyle, un groupe alcoxy ramifié ou non ramifié contenant 1 à 4 atomes de carbone, un atome d'halogène, de préférence de chlore ou de brome;
pour $n > 0$
$R_2$ représente un atome d'halogène, un groupe hydroxy,

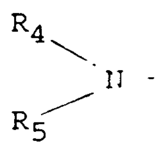

dans lequel $R_4$ et $R_5$, qui peuvent être identiques ou différents, représentent un atome d'hydrogène, un groupe alkyle, alcényle ou alcynyle ramifié ou non ramifié contenant 1 à 10 atomes de carbone, qui peut éventuellement être substitués par halogène, hydroxy ou par un hétérocycle à 5, 6 ou 7 chaînons lié par C, la chaîne carbonée pouvant être interrompue par de l'azote, de l'oxygène ou du soufre, un groupe alkylcarbonyle ramifié ou non ramifié contenant 1 à 6 atomes de carbone, éventuellement substitué par hydroxy ou halogène ou par un groupe amino éventuellement substitué une ou deux fois par un groupe alkyle ramifié ou non ramifié contenant 1 à 6 atomes de carbone, le groupe alkyle pouvant être substitué par halogène ou par hydroxy,
un groupe arylcarbonyle éventuellement substitué, un groupe arylsulfonyle éventuellement substitué, un groupe alkylsulfonyle contenant 1 à 4 atomes de carbone, ou
$R_4$ et $R_5$ forment avec l'atome d'azote un cycle à 5, 6 ou 7 chaînons saturé ou insaturé, éventuellement substitué une ou plusieurs fois par des groupes alkyle ramifiés ou non ramifiés contenant 1 à 4 atomes de carbone, cycle qui peut contenir comme hétéroatomes supplémentaires des atomes d'azote, d'oxygène ou de soufre, chacun des hétéroatomes supplémentaires pouvant éventuellement être substitué par un groupe alkyle ramifié ou non ramifié contenant 1 à 4 atomes de carbone;
$R_2$ représente un groupe arylsulfonyloxy, éventuellement substitué une ou plusieurs fois par des groupes alkyle et/ou alcoxy ramifiés ou non ramifiés contenant 1 à 4 atomes de carbone;
$R_2$ représente un groupe alkylsulfonyloxy ramifié ou non ramifié contenant 1 à 4 atomes de carbone;
$R_2$ représente un groupe arylcarbonyloxy éventuellement substitué une ou plusieurs fois par des groupes alkyle et/ou alcoxy ramifiés ou non ramifiés contenant 1 à 4 atomes de carbone;
$R_2$ représente un groupe alkylcarbonyloxy ramifié ou non ramifié contenant 1 à 12 atomes de carbone, la chaîne alkyle pouvant être interrompue par de l'azote, de l'oxygène ou du soufre;

$R_2$ représente le groupe

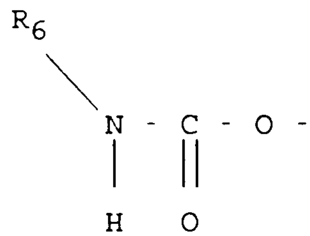

dans lequel $R_6$ représente un groupe alkyle, alcényle ou alcynyle ramifié ou non ramifié contenant 1 à 4 atomes de carbone, éventuellement substitué par halogène, un groupe aryle éventuellement substitué une ou plusieurs fois par des groupes alkyle et/ou alcoxy ramifiés ou non ramifiés contenant 1 à 4 atomes de carbone,
$R_2$ représente le groupe

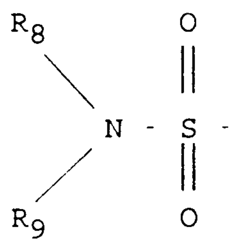

dans lequel $R_8$ et $R_9$, qui peuvent être identiques ou différents, représentent un groupe alkyle ramifié ou non ramifié contenant 1 à 4 atomes de carbone,
ou bien $R_8$ et $R_9$ forment avec l'atome d'azote un cycle à 5, 6 ou 7 chaînons éventuellement substitué une ou plusieurs fois par des groupes alkyle ramifiés ou non ramifiés contenant 1 à 4 atomes de carbone, cycle qui peut contenir comme hétéroatomes supplémentaires des atomes d'azote, d'oxygène ou de soufre, chaque atome d'azote supplémentaire étant substitué par un groupe alkyle contenant 1 à 4 atomes de carbone;
$R_2$ représente un groupe alkoxy ramifié ou non ramifié contenant 1 à 4 atomes de carbone; un groupe aryloxy;
$R_2$ représente un reste imido; dioxolane, dioxolane substitué;
pour n supérieur ou égal à 0
$R_2$ représente - CH = O; cyano;
$R_2$ représente un groupe aryloxycarbonyle,
$R_2$ représente un reste de formule générale

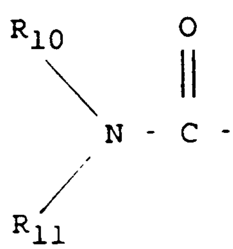

dans laquelle $R_{10}$ et $R_{11}$, qui peuvent être identiques ou différents, représentent un atome d'hydrogène, un groupe phényle, un groupe phényle substitué, un groupe alkyle, alcényle ou alcynyle ramifié ou non ramifié contenant 1 à 10 atomes de carbone, oui peut éventuellement être substitué par un atome

d'halogène, un groupe hydroxy, nitro, amino, amino substitué, ou lorsque $R_{10}$ = hydrogène ou alkyle et Y = C-$R_1$ ou Y est un atome d'azote et X un C-alkyle, $R_{11}$ peut être substitué par une fonction ester ou par un amide d'acide de formule générale

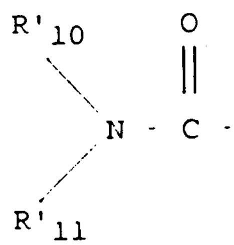

dans laquelle $R'_{10}$ et $R'_{11}$ ont la même signification que $R_{10}$ et $R_{11}$, à l'exception toutefois d'un amide d'acide,

$R_{10}$ ou $R_{11}$ représente un hétérocycle à 5, 6 ou 7 chaînons, saturé ou insaturé, lié par l'intermédiaire d'un atome de carbone, éventuellement substitué une ou plusieurs fois par un groupe alkyle ramifié ou non ramifié contenant 1 à 4 atomes de carbone,

$R_{10}$ et $R_{11}$ forment avec l'atome d'azote un cycle à 5, 6 ou 7 chaînons saturé ou insaturé, éventuellement substitué une ou plusieurs fois par des groupes alkyle ramifiés ou non ramifiés contenant 1 à 4 atomes de carbone, qui peut contenir comme hétéroatomes supplémentaires des atomes d'azote, d'oxygène ou de soufre, chaque atome d'azote supplémentaire pouvant être substitué par un groupe alkyle ramifié ou non ramifié contenant 1 à 4 atomes de carbone, de préférence un groupe méthyle,

$R_2$ représente un reste de formule générale

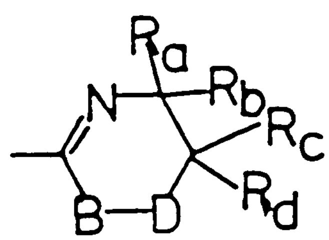

dans laquelle

B représente un atome d'oxygène, de soufre, un groupe NH ou N-alkyle en $C_1$-$C_6$

D représente le reste (C $R_e$ $R_f$)n dans lequel n peut être compris entre 0 et 3,

Ra représente un atome d'hydrogène, un groupe alkyle, éventuellement substitué par un groupe hydroxy ou amino, contenant 1 à 6 atomes de carbone, un groupe alcoxycarbonyle en $C_1$ à $C_4$, un groupe dialkylaminocarbonyle,

$R_b$, $R_c$, $R_d$, $R_e$, $R_f$ représentent un atome d'hydrogène, un groupe alkyle contenant 1 a 6 atomes de carbone, éventuellement substitué par un groupe hydroxy ou amino, ou représentent un groupe phényle;

$R_3$ représente un groupe phényle, le cycle phényle pouvant être substitué une ou plusieurs fois, de préférence en position 2, par un groupe méthyle, un atome d'halogène, un groupe nitro, alcoxy et/ou un groupe trifluorométhyle, ou représente un groupe pyridyle;

R° représente un atome d'hydrogène, un groupe alkyle ou acyle contenant 1 à 4 atomes de carbone,

R' représente un atome d'hydrogène, un groupe phényle, un groupe phényle substitué ou un groupe alkyle ramifié ou non ramifié Contenant 1 à 4 atomes de carbone;

X, Y représentent indépendamment l'un de l'autre C-$R_1$ ou N, mais pas tous les deux en même temps C-$R_1$,

ou bien Y représente le groupe C-COOR*, avec R* = alkyle ou hydrogène et X = azote;

Z représente un groupe alkyle ou alcényle ramifié ou non ramifié contenant n atomes de carbone, Z

pouvant en outre être éventuellement substitué par un groupe aryle ou deux fois par $R_2$, les groupes $R_2$ pouvant être identiques ou différents;
et
n représente l'un des nombres 0, 1, 2, 3, 4, 5, 6, 7, 8, 9 ou 10,
sous forme de leurs racémates, de leurs énantiomères, de leurs diastéréoisomères et de leurs mélanges, à chaque fois sous forme de bases libres ou de leurs sels d'addition d'acide irréprochables du point de vue physiologique, des substituants pouvant être présents sous forme d'un ou plusieurs atomes dans le groupe suivant: halogène, méthyle, méthoxy, hydroxy et trifluorométhyle, et aryle signifie un reste aromatique contenant jusqu'à 10 atomes de carbone, qui peut éventuellement être substitué aussi une ou plusieurs fois par un atome d'halogène ou un groupe méthyle, méthoxy, hydroxy et trifluorométhyle,
avec la condition que dans le cas d'un composé de formule générale Ia, lorsque X et Y représentent tous les deux un atome d'azote, Z représente une chaîne alkyle non ramifiée,
n = 0 ou un nombre de 1 à 8, $R_1$ représente un atome d'hydrogène, un groupe alkyle en $C_1$-$C_4$ ramifié ou non ramifié, éventuellement substitué par halogène ou hydroxy, un groupe cyclopropyle, un groupe alcoxy en $C_1$-$C_3$ ou un atome d'halogène et
$R_2$ représente un reste de formule générale

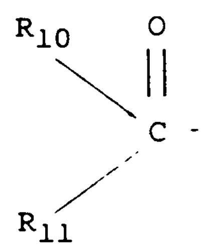

dans laquelle $R_{10}$ et $R_{11}$, qui peuvent être identiques ou différents, représentent un atome d'hydrogène, un groupe alkyle ou hydroxyalkyle en $C_1$-$C_4$ ramifié ou non ramifié, ou bien $R_{10}$ et $R_{11}$ forment avec l'atome d'azote un cycle à 5, 6 ou 7 chaînons qui peut contenir éventuellement de l'azote, de l'oxygène ou du soufre comme hétéroatomes supplémentaires, le second atome d'azote pouvant éventuellement être substitué par un groupe alkyle en $C_1$-$C_4$,
$R_3$ ne peut pas représenter un groupe phényle ou bien un groupe phényle qui peut être substitué une ou plusieurs fois par méthyle, halogène, nitro ou trifluorométhyle, ou un groupe α-pyridyle,
les composés suivants étant également exclus:
2-(2-chloroéthyl)-4-(2-chlorophényl)-9-méthyl-6H-thiéno-[3,2-f][1,2,4]triazolo[4,3-a][1,4]diazépine,
2-(méthylsulfonylméthyl)-4-(2-chlorophényl)-9-méthyl-6H-thiéno[3,2-f][1,2,4]triazolo[4,3-a][1,4]diazépine,
2-cyano-4-(2-chlorophényl)-9-méthyl-thiéno[3,2-f][1,2,4]-triazolo[4,3-a][1,4]diazépine,
2-(1-hydroxyéthyl)-4-(2-chlorophényl)-9-méthyl-6H-thiéno-[3,2-f][1,2,4]triazolo[4,3-a][1,4]diazépine.

**2.** Composés de formule générale Ia ou Ib selon la revendication 1, dans lesquels
$R_1$ représente un groupe méthyle, éthyle, méthoxy, éthoxy ou un atome de chlore ou de brome;
$R_2$ représente un atome de chlore, de brome, d'iode, un groupe hydroxy,

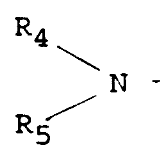

dans lequel $R_4$ et $R_5$, qui peuvent être identiques ou différents, représentent un atome d'hydrogène, un groupe alkyle ramifié ou non ramifié contenant 1 à 6, de préférence 1 à 4, atomes de carbone, la chaîne carbonée pouvant être interrompue par de l'azote, un groupe alkylcarbonyle ramifié ou non ramifié contenant 1 à 4 atomes de carbone, éventuellement substitué par un groupe diméthylamino, un groupe phénylcarbonyle,
dans le cas où $R_5$ = hydrogène un groupe phénylsulfonyle éventuellement substitué par un groupe

acylamino, de préférence acétylamino, amino, alkylamino ou dialkylamino,
ou
$R_4$ et $R_5$ forment avec l'atome d'azote un cycle pipéridine, pyrrolidine, N'-méthylpipérazine, un cycle morpholine éventuellement diméthylsubstitué, un cycle pyrrole, pyrazole, imidazole ou triazole;
$R_2$ représente -CH = O, COOH;
une $\Delta^2$-imidazoline, -oxazoline, -thiazoline éventuellement substituée une ou plusieurs fois par un groupe méthyle; un groupe tolylsulfonyloxy; un groupe méthylsulfonyloxy; un groupe phénylcarbonyloxy; un groupe alkylcarbonyloxy ramifié ou non ramifié contenant 1 à 5 atomes de carbone; un groupe méthoxy- ou éthoxycarbonyle;
$R_2$ représente

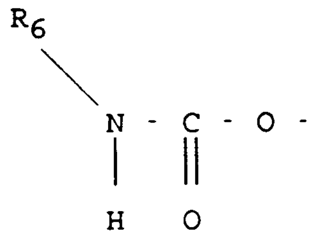

dans lequel $R_6$ représente un groupe alkyle ramifié ou non ramifié contenant 1 à 4 atomes de carbone;
$R_2$ représente

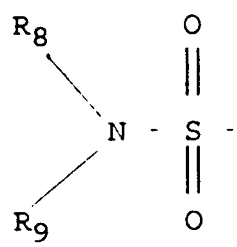

dans lequel $R_8$ et $R_9$, qui peuvent être identiques ou différents, représentent un groupe méthyle, éthyle, propyle ou isopropyle ou bien $R_8$ et $R_9$ forment avec l'atome d'azote un cycle N'-méthylpipérazine ou morpholine;
$R_2$ représente

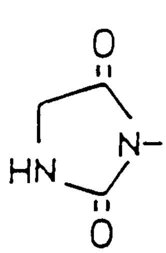 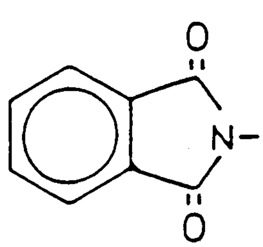 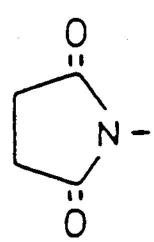

$R_3$ représente un groupe phényle, le cycle phényle pouvant être substitué en position 2 par un atome de chlore;
R° représente un atome d'hydrogène, ou un groupe méthyle, acétyle;
R' représente un atome d'hydrogène;
X, Y représentent indépendamment l'un de l'autre C-$R_1$ ou N, mais pas tous les deux en même temps C-$R_1$, ($R_1$ représente de préférence un atome d'hydrogène),
ou bien Y représente le groupe C-COOR*, avec R* = alkyle ou hydrogène et X = azote;
Z est défini comme précédemment, et représente de préférence $-(CH_2)_n-$, éventuellement substitué par

un groupe phényle ou deux fois par $R_2$, les groupes $R_2$ pouvant aussi être différents ou bien $-CH_2-CHR_2-CH_2-R_2$, $CH_2-CHR_2R_2$, $CH_2CHR_2-CH_2-C_6H_5$;
et
n représente l'un des nombres 1, 2, 3, 4, 5, 6, 7, 8, 9 ou 10 ainsi qu'éventuellement leurs sels d'addition d'acide irréprochables du point de vue physiologique.

**3.** Composés de formule générale Ia selon la revendication 1 ou 2, dans lesquels $R_2$ est défini comme précédemment et $R_1$ représente un groupe méthyle ou méthoxy, $R_3$ représente un groupe o-chlorophényle, R' représente un atome d'hydrogène, X, Y représentent l'un et l'autre un atome d'azote ou X représente C-H et Y représente un atome d'azote, $Z-(CH_2)_n-$, n représente les nombres 2, 3 ou 7.

**4.** Procédé de préparation de composés de formule générale Ia selon la revendication 1, 2 ou 3, dans lesquels $R_1$, $R_3$, R', Z, X, Y et n sont tels que définis précédemment, $R_2$ représente $-COOR^*$, avec $R^*$ = alkyle, ou bien $R_2$ peut représenter un groupe hydroxy, alkylcarbonyloxy, alcoxycarbonyle, aryloxycarbonyle, dioxolanne, dioxolanne substitué ou $R_8R_9NSO_2-$,
caractérisé en ce que l'on fait réagir un composé correspondant de formule générale

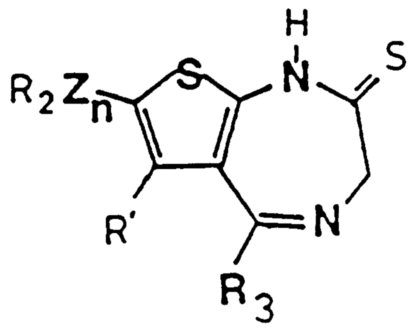

II

A) lorsque X et Y représentent un atome d'azote
a) avec un hydrazide d'acide de formule générale

$R_1-CONHNH_2$,

b) ou avec l'hydrazine pour le convertir en un composé de formule générale

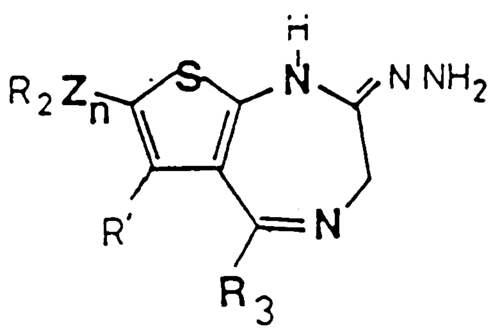

puis avec un halogénure d'acide de formule générale

$R_1-CO-Hal$

ou avec un orthoester de formule générale

$R_1-C(OR')_3$

dans laquelle R' représente un groupe alkyle inférieur,
ou bien

B) lorsque X représente C-H et Y un atome d'azote

a) avec un aminoalcyne de formule générale

$R_{11}$-C≡C-$CN_2$-$NH_2$

dans laquelle $R_{11}$ représente un atome d'hydrogène ou un groupe alkyle inférieur
ou bien

b) avec un alkylacétal d'α-aminoaldéhyde ou un alkylacétal d'α-aminocétone de formule générale

$H_2NCN_2$-$CR_1(OR')_2$

dans laquelle $R_1$ représente un groupe alkyle inférieur ou un groupe cyclopropyle;

C) lorsque X représente un atome d'azote et Y représente C-H on décarboxyle un composé de formule générale

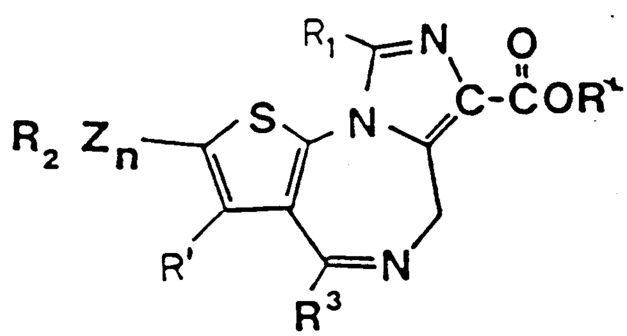

dans laquelle R* = alkyle inférieur,
et on le convertit éventuellement en ses sels d'addition d'acide irréprochables.

**5.** Procédé de préparation de composés de formule

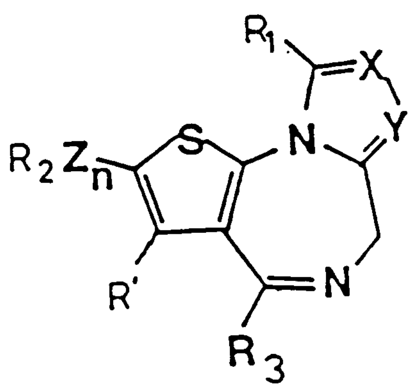

Ia

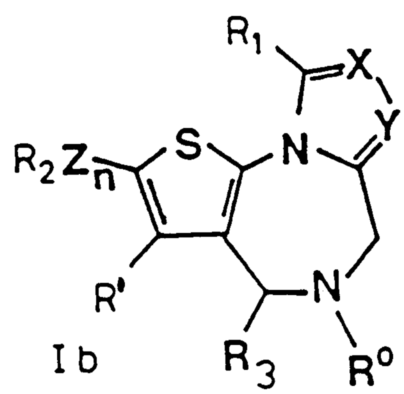

Ib

selon l'une des revendications 1, 2 ou 3
caractérisé en ce que l'on fait réagir comme composé de départ un composé de formule générale I

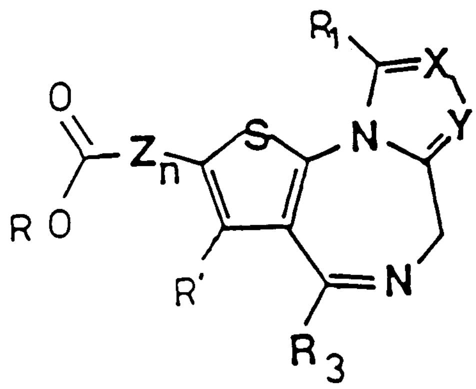

dans laquelle $R_1$ = hydrogène, alkyle, cycloalkyle; Z, X, Y, R', n et $R_3$ sont définis comme précédemment et R représente un groupe alkyle inférieur, de la manière suivante:

(1) on obtient des composés de formule générale Ia avec

$R_2$ = COOH

par saponification de composés de formule I;

(2) on obtient des composés de formule générale Ia avec

$R_2$ = $R_{10}R_{11}NCO-$

par réaction de Ia avec

$R_2$ = COOH

avec une amine de formule

$HNR_{10}R_{11}$

par exemple en présence de sulfonyldiimidazole ou de carbonyldiimidazole;

(3) on obtient des composés de formule générale Ia avec

$R_2$ = OH

par réduction sélective de I, par exemple avec le malonate de lithium ou le borohydrure de sodium; ou par hydrolyse d'un composé de formule générale Ia dans laquelle $R_2$ représente un reste alkylcarbonyloxy;

(4) on obtient des composés de formule générale Ia avec

$R_2$ = $R_6NH-COO-$

par réaction de l'alcool, préparé par exemple selon 3) avec un isocyanate de formule générale

$R_6-N=C=O$;

(5) on obtient des composés de formule générale Ia avec $R_2$ = alkyl- ou arylcarbonyloxy par réaction de l'alcool, préparé par exemple selon 3), avec un équivalent d'acide d'un acide carboxylique de formule

R-COOH

dans laquelle R représente un reste aryle ou un reste alkyle ramifié ou non ramifié contenant 1 à 8 atomes de carbone, la chaîne carbonée pouvant être interrompue par de l'azote, de l'oxygène ou du soufre;

(6) on obtient des composés de formule générale Ia avec $R_2$ = alkylsulfonyloxy ou arylsulfonyloxy par réaction de l'alcool préparé par exemple selon 3) avec un halogénure d'alkyl- ou arylsulfonyle avec addition d'un agent de fixation des acides;

(7) on obtient des composés de formule générale Ia avec

$R_2 = NR_4R_5$

ou un reste imidazole

par réaction du mésylate, préparé par exemple selon 6) avec une amine de formule

$HNR_4R_5$

ou un imide;

(8) on obtient des composés de formule générale Ia avec

$R_2 = NH_2$

par exemple par coupure du phtalimide ou à partir des isocyanates correspondants, $R_2 = N=C=O$ par hydrolyse;

(9) on obtient des composés de formule générale Ia avec

$R_2 = NR_4R_5$

$R_4$ ou $R_5$ représentant un groupe alkyl- ou arylcarbonyle, par réaction d'une amine primaire ou secondaire, préparée par exemple selon 7) ou 8), avec un équivalent d'acide carboxylique dérivé d'un acide carboxylique de formule

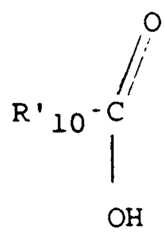

(10) on obtient des composés de formule générale Ia avec

$R_2$ = formyle

par oxydation de l'alcool, préparé par exemple selon 3) avec raccourcissement de la chaîne de n à n-1;

(11) on obtient des composés de formule générale Ia dans laquelle $R_2$ représente un reste de formule générale

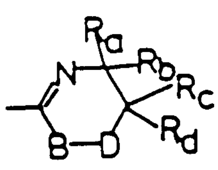

par réaction d'un composé de formule générale Ia avec

$R_2 = COOH$

avec une amine de formule générale

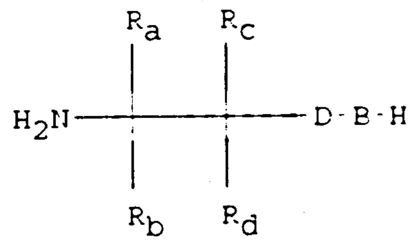

dans laquelle $R_a$, $R_b$, $R_c$, $R_d$, D et B ont la signification donnée ci-dessus,
en présence de triphénylphosphine de $CCl_4$ ou d'une base;

(11a) on obtient des composés de formule générale Ia, dans laquelle $R_2$ représente un reste thiazoline éventuellement substitué, à partir des oxazolines correspondantes, préparées par exemple selon 11) par sulfuration, par exemple avec le pentasulfure de phosphore ou le réactif de Lawesson®;

(12) on obtient des composés de formule générale Ia avec

$R_2$ = CN

par réaction de composés de formule générale Ia dans laquelle

$R_2$ = $CONH_2$

avec l'oxychlorure de phosphore;

(13) on obtient des composés de formule générale Ia, dans laquelle $R_2$ représente une 2-imidazoline substituée éventuellement par des groupes alkyle ramifiés ou non ramifiés, par réaction de composés de formule générale Ia avec

$R_2$ = CN

a) avec HCl éthanolique,
b) réaction de l'imidoéthylester formé avec une éthylènediamine éventuellement substituée par des groupes alkyle ramifiés ou non ramifiés,
c) l'alkylation de la fonction N-H libre a lieu éventuellement avec des agents d'alkylation connus;

(14) on obtient des composés de formule générale Ia dans laquelle $R_2$ représente un reste aryloxy ou alkyloxy, par exemple par réaction du mésylate, préparé par exemple selon 6), avec les alcoolates correspondants;

(15) on obtient des composés de formule générale Ia dans laquelle $R_2$ représente un groupe alkyl- ou aryloxycarbonyle, par exemple par réaction d'un équivalent d'acide de I, $R_2$ = COOH, avec les alcools correspondants;

(16) on obtient des composés de formule générale Ia avec
$R_2$ = halogène,
par réaction du tosylate, préparé par exemple selon 6), avec un agent d'halogénation;

puis, si on le souhaite, on fait réagir les composés Ia obtenus,
avec $R_1$ = hydrogène,
en présence d'une base, avec un agent d'halogénation, comme par exemple le chlore ou le brome, pour obtenir un composé de formule générale Ia dans laquelle $R_1$ représente un atome d'halogène, par exemple de chlore ou de brome;

puis, si on le souhaite, on convertit le composé halogéné en un composé de formule générale Ia, dans laquelle $R_1$ représente un groupe alcoxy contenant 1 à 4 atomes de carbone, par réaction avec l'alcoolate correspondant; puis, si on le souhaite, on réduit sélectivement un composé de formule générale Ia en un composé de formule générale Ib avec R° = hydrogène que l'on alkyle ou acyle si on le souhaite;

et on convertit éventuellement les composés Ia ou Ib ainsi obtenus en leurs sels d'addition d'acide irréprochables.

**6.** Procédé de préparation de composés de formule générale Ia selon la revendication 4, dans laquelle $R_1$ représente un atome d'halogène ou un groupe alcoxy, caractérisé en ce que
a) on halogène un composé de formule générale Ia, dans laquelle $R_1$ représente un atome d'hydrogène, avec un agent d'halogénation, comme par exemple le chlore ou le brome puis, si on le souhaite
b) on fait réagir le composé halogéné avec l'alcoolate correspondant
et éventuellement on le convertit en son sel d'addition d'acide irréprochable.

**7.** Procédé de préparation de composés de formule générale Ib selon la revendication 1, 2 ou 3, caractérisé en ce que l'on réduit sélectivement un composé de formule générale Ia et, si on le souhaite, on alkyle ou acyle le composé Ib obtenu dans lequel R° représente un atome d'hydrogène et on le convertit éventuellement en ses sels d'addition d'acide irréprochables du point de vue pharmacologique.

**8.** Préparations pharmaceutiques, contenant comme substances actives des composés de formule générale Ia ou Ib en combinaison avec des adjuvants et/ou véhicules habituels.

**9.** Procédé de préparation de préparations pharmaceutiques selon la revendication 8, caractérisé en ce que l'on traite des composés de formule générale Ia ou Ib avec des adjuvants et/ou véhicules galéniques habituels pour les mettre sous des formes d'utilisation pharmaceutiques habituelles.

**10.** Composés de formule générale Ia ou Ib selon la revendication 1, 2 ou 3 utilisables pour la préparation de médicaments à effet antagoniste du PAF.

**11.** Procédé de préparation de médicaments contenant des composés de formule générale Ia ou Ib selon l'une des revendications 1, 2 ou 3, pour le traitement de maladies auxquelles participe le PAF.

**12.** Nouveaux composés intermédiaires de formule générale

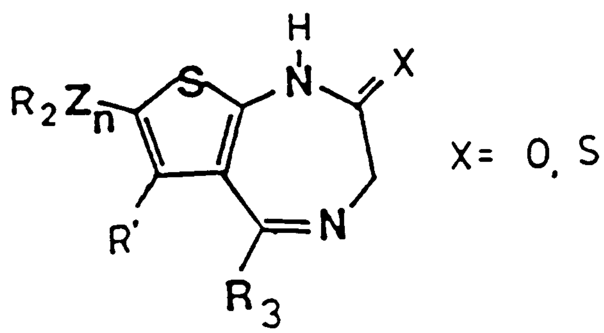

dans laquelle
R' représente un atome d'hydrogène, un groupe phényle, phényle substitué ou un groupe alkyle ramifié ou non ramifié contenant 1 à 4 atomes de carbone,
$R_3$ représente un groupe phényle, le cycle phényle pouvant être substitué une ou plusieurs fois, de préférence en position 2, par un groupe méthyle, de préférence par un atome d'halogène, de préférence encore un atome de chlore ou de brome, un groupe nitro, alcoxy, de préférence un groupe méthoxy et/ou un groupe trifluorométhyle, ou représente un groupe pyridyle;
Z représente un groupe alkyle ou alcényle ramifié ou non ramifié contenant n atomes de carbone, Z pouvant en outre être éventuellement substitué par un groupe aryle ou deux fois par $R_2$, les groupes $R_2$ pouvant être identiques ou différents;
n représente l'un des nombres 0, 1, 2, 3, 4, 5, 6, 7, 8, 9 ou 10
$R_2$ représente un groupe hydroxy, alkylcarbonyloxy, dioxolanne, dioxolanne substitué ou

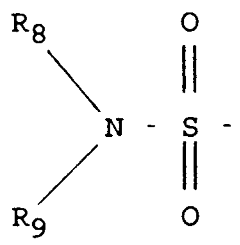

$R_8$ et $R_9$, qui peuvent être identiques ou différents, représentant un groupe alkyle ramifié ou non ramifié contenant 1 à 4 atomes de carbone,

ou bien $R_8$ et $R_9$ forment avec l'atome d'azote un cycle à 5, 6 ou 7 chaînons éventuellement substitué une ou plusieurs fois par un groupe alkyle ramifié ou non ramifié contenant 1 à 4 atomes de carbone, cycle qui peut contenir comme hétéroatomes supplémentaires de l'azote, de l'oxygène ou du soufre, chaque atome d'azote supplémentaire étant substitué par un groupe alkyle contenant 1 à 4 atomes de carbone, de préférence un groupe méthyle;

ou bien lorsque R' ≠ hydrogène

$R_2$ peut représenter aussi un groupe alcoxycarbonyle.